# EUROPÄISCHE PATENTSCHRIFT

(11) **EP 1 140 042 B1**
(45) Veröffentlichungstag und Bekanntmachung des Hinweises auf die Patenterteilung: **26.03.2008**
(21) Anmeldenummer: 99958170.5
(22) Anmeldetag: 07.12.1999
(51) Int. Cl.: A61K 31/00, A61K 31/353, A61K 31/18, A61K 31/4433, A61K 31/352, A01N 43/16, A61P 33/10, A61P 33/14

(54) **VERWENDUNG VON INHIBITOREN DES KQT1-KANALS ZUR HERSTELLUNG EINES MEDIKAMENTS ZUR BEHANDLUNG VON KRANKHEITEN, DIE DURCH HELMINTHEN UND EKTOPARASITEN HERVORGERUFEN WERDEN**
UTILIZATION OF INHIBITORS OF THE KQT1 CHANNEL IN ORDER TO PRODUCE A MEDICAMENT FOR TREATING DISEASES WHICH ARE CAUSED BY PARASITIC HELMINTHS AND ECTOPARASITES
UTILISATION D'INHIBITEURS DU CANAL KQT1 POUR LA PREPARATION D'UN MEDICAMENT DESTINE A TRAITER DES MALADIES PROVOQUEES PAR DES HELMINTHES ET DES ECTOPARASITES

(30) Priorität: 17.12.1998 DE 19858253
(43) Veröffentlichungstag der Anmeldung: 10.10.2001
(73) Patentinhaber: Sanofi-Aventis Deutschland GmbH, 65929 Frankfurt am Main (DE)
(72) Erfinder: GERLACH, Uwe, D-65795 Hattersheim (DE); HOFMANN, Joachim, D-65520 Bad Camberg (DE); LANG, Hans, Jochen, D-65719 Hofheim (DE); WEI, Aguan Washington Uni. School of Medicine, Saint Louis, MO 63110 (US)
(86) Internationale Anmeldenummer: PCT/EP1999/009547
(87) Internationale Veröffentlichungsnummer: WO 2000/035429

(56) Entgegenhaltungen:
- EP-A- 0 807 629
- EP-A- 0 860 440
- EP-A- 0 861 836
- EP-A- 0 905 131
- EP-A- 0 906 911
- EP-A- 0 913 396
- WO-A-96/13520
- WEI, AGUAN D. ET AL: "Functional properties and tissue distribution of C. elegans potassium channel homologs of human KvLQT1." BIOPHYSICAL JOURNAL, (FEB., 1998) VOL. 74, NO. 2 PART 2, PP. A206. MEETING INFO.: FORTY-SECOND ANNUAL MEETING OF THE BIOPHYSICAL SOCIETY KANSAS CITY, MISSOURI, USA FEBRUARY 22-26, 1998 , XP000905423
- LOUSSOUARN, GILDAS ET AL: "KvLQT1 potassium channel but not IsK is the molecular target for trans-6-cyano-4-(N-ethylsulfonyl-N-methyla mino)-3-hydroxy-2,2- dimethyl-chromane" MOL. PHARMACOL. (1997), 52(6), 1131-1136 , XP000905467
- BUSCH, A. E. ET AL: "The role of the IsK protein in the specific pharmacological properties of the IKs channel complex" BR. J. PHARMACOL. (1997), 122(2), 187-189 , XP000905465

## Beschreibung

Die Erfindung betrifft die Verwendung von Inhibitoren des KQT1-Kanals zur Herstellung eines Medikaments zur Behandlung von Krankheiten, die durch Helminthen und Ektoparasiten hervorgerufen werden

Parasitosen sind weitverbreitet und verursachen in Mensch und Tier ein weites Spektrum pathologischer Effekte, die von geringen physiologischen Störungen bis zu starken, sogar tödlichen Erkrankungen reichen. Heutzutage sind viele, intensiv erforschte Parasitenerkrankungen bekannt, die die Gesundheit und das Leben des Menschen und seiner Haus- und Nutztiere bedrohen.

Weltweit ist mit zunehmender Tendenz bei Millionen von Menschen eine Schwächung des Immunsystems festzustellen. Dieser Personenkreis wird von opportunistischen Parasiten so massiv befallen, daß jährlich Millionen von Todesopfern zu beklagen sind. Im Zeitalter der Fernreisen muß auch in Nicht- Drittewelt-Ländern, die normalerweise einen hohen Hygienestandard besitzen, mit exotischen Parasiten gerechnet werden. Dies hat seinen Grund darin, daß ein immer stärkerer Trend zu Trekkingreisen unter landesüblichen Hygienebedingungen besteht und daß - aus der vermeintlichen Sicherheit im eigenen Land - ein Verlust des Gefühls / Wissens um Hygienegefahren entstanden ist. Neben dem Schutz der menschlichen Gesundheit verlangt auch der Tierschutz, durch Parasitosen hervorgerufene Leiden und Schmerzen zu heilen und möglichst abzuwenden. Wirtschaftliche Gründe kommen vor allem in der Nutztierhaltung zum Tragen, wo durch ungünstige Bedingungen der Haltung und Fütterung von Tieren (z.B. bei bestimmten Formen der Massentierhaltung) parasitäre Erkrankungen bevorzugt auftreten, die zur Leistungsminderung quantitativer (Fleischmenge, Eizahl, Rennzeit) oder qualitativer Art (Fleisch- und Wollqualität) beitragen. Die großen Schäden, die durch Parasitosen in Mensch und Tier hervorgerufen werden, machen ihre Kontrolle wünschenswert wenn nicht unerläßlich im Interesse der Gesundheit und Wirtschaftlichkeit.

Der Einsatz chemischer, in ihrer Wirksamkeit gegen einzelne Parasiten oder größere Parasitengruppen bekannter und im Wirt (Mensch, Tier) toxikologisch unbedenklicher Substanzen, hat in der Parasitenbekämpfung noch eine überragende Bedeutung.

Nach ihrem Wirkungsspektrum unterscheidet man gegen Helminthen wirkende Anthelminthika, gegen Protozoen wirksame Antiprotozoika, gegen Insekten wirksame Insektizide, gegen Milben (Akaria) wirksame Akarizide; die letzten beiden Gruppen faßt man auch unter dem Begriff Ektoparasitizide zusammen.
Die Zunahme von Arzneimittelresistenzen, begünstigt durch langjährigen und intensiven Einsatz besonders in der modernen Massentierhaltung oder das Auftreten von zum Teil starken Nebenwirkungen insbesondere bei Dauermedikation von Menschen, die im Rahmen fortschreitender Globalisierung längere Zeit in den Tropen- und Subtropen arbeiten müssen sowie hohe Kosten bei der Prophylaxe/ Therapie mit bestimmten Chemotherapeutika macht die Suche nach preisgünstigen anderen Substanzklassen mit einem anderen Wirkmechanismus und besserer Verträglichkeit unumgänglich.

Es wurde im Rahmen der Erfindung erkannt, daß Inhibitoren des KQT1-Kanals ein neues letales Prinzip für Helminthen und Ektoparasiten darstellen. Die vorliegende Erfindung beschäftigt sich deshalb mit der Verwendung von Blockern des zellulären KQT1-Kanals, der in Helminthen und Ektoparasiten vorkommt, zum Herstellen eines Arzneimittels für die Behandlung von Wirbeltieren und Menschen, die von Helminthen oder Ektoparasiten befallen sind. Diese KQT1-Blocker werden als Arzneimittel allein oder in Kombination mit anderen Arzneimitteln und therapeutischen Maßnahmen angewendet.

Es wurde nämlich überraschenderweise gefunden, daß Inhibitoren des lks-Kanals den KQT1-Kanal in potenter Weise blockieren können. Somit sind Inhibitoren des Iks-Kanals in ihrer blockierenden Wirkung auf den KQT1-Kanal zur Herstellung eines Medikaments für die Behandlung von Krankheiten geeignet, die durch Helminthen und Ektoparasiten hervorgerufen werden.

Bei den Inhibitoren des Iks -Kanals handelt es sich um Wirkstoffe, die erst in den letzten 10 Jahren in den Blickpunkt des Interesses rückten. Zahlreiche Anwendungen wurden inzwischen für diese Wirkstoffklasse beschrieben, wie Ihre Verwendung als Arzneimittel zur Behandlung von Herzrhythmusstörungen, der Ulkustherapie durch Hemmung der Magensäuresekretion, der Therapie von Durchfallerkrankungen, der Behandlung von Menière Krankheit usw. Von besonderer Bedeutung für die therapeutische Verwendung von Iks-Blockern ist deren antiarrhythmische Wirkung.

Die antiarrhythmische Wirkung der IKs-Kanal-Blocker beruht auf der Verlängerung der Plateauphase von Herzzellen, besonders unter den Bedingungen der Sympathikusstimulation. Verzögerung der Repolarisation ist als antiarrhythmisches Prinzip der sogenannten Klasse III-Antiarrhythmika zur Beendigung von malignen Herzrhythmusstörungen anerkannt.

WO9613520 beschreibt die Klonierung und Expression von Kaliumkanälen und eine Methode zur Kontrolle von Nematoden- und Insektenplage durch die Blockade von Kaliumkanälen. In Wei et. al. (Biophysical Journal (Feb. 1998), Vol. 74, No. 2 part 2, A206) werden funktionelle Eigenschaften von KQT-1-Kanälen offenbart. Loussouarn et. al. (Mol. Phamacol. (1997), 52(6), 1131-1136) beschreibt, dass trans-6-cyano-4-(N-ethylsulfonyl-N-methylamino)-3-hydroxy-2,2-dimethyl-chroman (293 B) auf KvLQT1-Kanäle gerichtet ist, aber nicht auf den Iₛₖ-Kanal. Busch et al. (Br. J. Pharmacol. (1997), 122(2), 187-189) berichtet über die Affinitiät einiger lₖₛ-Blocker zu den Iₛₖ- und KvLQT1-Untereinheiten von lₖₛ-Kanälen.

Durch die überraschende Erkenntnis, daß lks-Inhibitoren in ihrer Wirkung als KQT1-Blocker die Lebensfähigkeit von Helminthen und Ektoparasiten beeinträchtigt, betrifft die Erfindung die Verwendung der in den Ansprüchen der nachfolgenden Patente umfaßten Iks-Kanalblocker als wertvolle Therapeutika zur Behandlung des Befalls von Menschen und Wirbeltieren durch Helminthen und Ektoparasiten und der hieraus resultierenden Erkrankungen.

Geeignete Verbindungen für die erfindungsgemäße Verwendung sind in den nachfolgend aufgezählten Patenschriften und Literaturstellen beschrieben.
1) EP 807 629 (HOE 96/F119)
   Chromane der Formel I 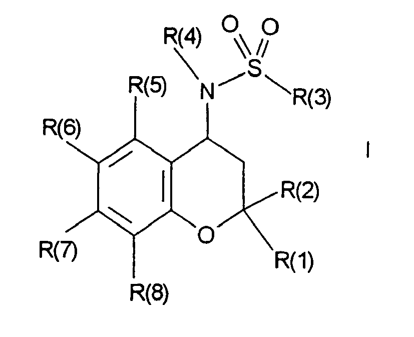 worin bedeuten:
   R(1) und R(2)
   unabhängig voneinander Wasserstoff, CₚF₂ₚ₊₁, Alkyl mit 1, 2, 3, 4, 5 oder 6 C-Atomen oder Phenyl,
   das unsubstituiert ist oder substituiert mit 1 oder 2 Substituenten ausgewählt aus der Gruppe bestehend aus F, Cl, Br, I, CF₃, Methyl,
   Methoxy, Sulfamoyl, Methylsulfonylamino und Methylsulfonyl;
   p 1, 2 oder 3;
   oder
   R(1) und R(2)
   gemeinsam eine Alkylenkette mit 2, 3, 4, 5, 6, 7, 8, 9 oder 10 C-Atomen;
   R(3) R(9)-CₙH₂ₙ[NR(11)]ₘ-;
   R(9) Wasserstoff oder Cycloalkyl mit 3, 4, 5, 6, 7 oder 8 C-Atomen;
   n Null, 1, 2, 3, 4, 5, 6, 7, 8, 9 oder 10;
   m Null oder 1;
   R(11) Wasserstoff oder Alkyl mit 1, 2, 3, 4, 5 oder 6 C-Atomen;
   oder
   R(11) gemeinsam mit R(9) eine Alkylengruppe mit 1, 2, 3, 4, 5, 6, 7 oder 8 C-Atomen;
   wobei eine CH₂-Gruppe der Gruppe CₙH₂ₙ durch -O-, -SO_{q} oder -NR(10) ersetzt sein kann;
   q Null, 1 oder 2;
   R(10) Wasserstoff, Methyl oder Ethyl;
   R(4) R(12)-CᵣH₂ᵣ;
   R(12)
   Wasserstoff, Cycloalkyl mit 3, 4, 5, 6, 7 oder 8 C-Atomen, Piperidyl, 1-Pyrrolidinyl, N-Morpholino, N-Methylpiperazino, CₚF₂ₚ₊₁, Pyridyl, Thienyl, Imidazolyl oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
   r 1, 2, 3, 4, 5, 6, 7, 8, 9, 10, 11, 12, 13, 14, 15, 16, 17, 18, 19 oder 20;
   wobei eine CH₂-Gruppe der Gruppe CᵣH₂ᵣ ersetzt sein kann durch -O-, -HC=CH-, -C=C-, -CO-, -CO-O-, -SO_{q}- oder -NR(10)-;
   q Null, 1 oder 2;
   R(10) Wasserstoff, Methyl oder Ethyl;
   R(5), R(6), R(7) und R(8)
   unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, -CN, -CF₃, -C₂F₅, -C₃F₇, -N₃, -NO₂, -CONR(13)R(14), -COOR(15), R(16)-CₛH₂ₛ-Y- oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   R(13) und R(14)
   unabhängig voneinander Wasserstoff oder Alkyl mit 1, 2 oder 3 C-Atomen;
   R(15) Wasserstoff, Methyl, Ethyl, Phenyl oder -CᵤH₂ᵤ-NR(13)R(14);
   u 2 oder 3;
   R(16) Wasserstoff, Cycloalkyl mit 3, 4, 5, 6, 7 oder 8 C-Atomen, -COOR(15), Thienyl, Imidazolyl, Pyridyl, Chinolyl, Isochinolyl, Piperidyl, 1-Pyrrolidinyl, N-Morpholino, N-Methylpiperazino, CₜF₂ₜ₊₁ oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
   s Null, 1, 2, 3, 4, 5 oder 6;
   t 1, 2 oder 3;
   Y SO_{q}, -CO-, -SO₂-NR(10)-, -O-, -NR(10)- oder -CO-NR(10);
   wobei jedoch R(6) nicht -OCF₃ oder -OC₂F₅ sein kann;
   und deren physiologisch verträgliche Salze;
   oder
2) EP 847 996 (HOE 96/F341)
   Verbindungen der Formel I, 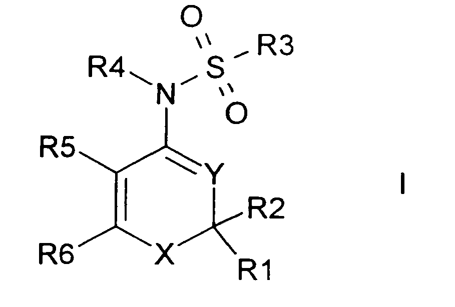 worin bedeuten:
   X -O-, -S-, -SO-, -SO₂-, -NR(7)-, -CR(8a)R(8b)- oder -CO-;
   R(7) Wasserstoff oder -(CₐH₂ₐ)-R(9),
   wobei eine CH₂-Gruppe der Gruppe CₐH₂ₐ ersetzt sein kann durch -O-, - CH=CH-, -C≡C-, -CO-, -CO-O-, -O-CO-, -S-, -SO-, -SO₂-, NR(10)- oder-CONR(10)-;
   R(10) Wasserstoff oder Alkyl mit 1, 2 oder 3 C-Atomen;
   a Null, 1, 2, 3, 4, 5, 6, 7 oder 8;
   R(9) Wasserstoff, CF₃, C₂F₅, C₃F₇, Cycloalkyl mit 3, 4, 5, 6, 7 oder 8 C-Atomen, Dimethylamino, Diethylamino, 1-Piperidyl, 1-Pyrrolidinyl, 4-Morpholinyl, 4-Methylpiperazin-1-yl, Pyridyl, Thienyl, Imidazolyl oder Phenyl,
   wobei Pyridyl, Thienyl, Imidazolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(7) und R(1)
   gemeinsam eine Bindung;
   R(8a) Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(8b) Wasserstoff, Alkyl mit 1, 2 oder 3 C-Atomen, -OR(10), -COOR(10), CO-
   R(10);
   R(10) Wasserstoff oder Alkyl mit 1, 2 oder 3 C-Atomen;
   oder
   einer der Reste R(8a) oder R(8b)
   gemeinsam mit R(1) eine Bindung, sofern Y die Bedeutung von N besitzt;
   Y N oder CR(11);
   R(11) Wasserstoff oder Alkyl mit 1, 2 oder 3 C-Atomen;
   R(1) und R(2)
   unabhängig voneinander Wasserstoff, CF₃, C₂F₅, C₃F₇, F, Cl, Methoxy, Alkyl
   mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(1) und R(2)
   gemeinsam eine Alkylenkette mit 2, 3, 4, 5, 6, 7, 8, 9 oder 10 C-Atomen;
   R(3) R(12)-CₙH₂ₙ-NR(13)-oderR(12)-CₙH₂ₙ-,
   wobei eine CH₂-Gruppe in den Gruppen CₙH₂ₙ ersetzt sein kann durch -O-, -CO-, -S-, -SO-, -SO₂- oder -NR(10a)-;
   R(10a)
   Wasserstoff, Methyl oder Ethyl;
   R(12)
   Wasserstoff, Methyl, Cycloalkyl mit 3, 4, 5, 6, 7 oder 8 C-Atomen, CF₃, C₂F₅ oder C₃F₇;
   n Null, 1, 2, 3, 4, 5, 6, 7, 8, 9 oder 10;
   R(13)
   Wasserstoff oder Alkyl mit 1, 2, 3, 4, 5 oder 6 C-Atomen;
   oder
   R(12) und R(13)
   gemeinsam eine Bindung, sofern n nicht kleiner als 3 ist;
   oder
   R(3) und R(4)
   gemeinsam eine Alkylenkette mit 3, 4, 5, 6, 7 oder 8 C-Atomen,
   wobei eine CH₂-Gruppe der Alkylenkette durch -O-, -CO-, -S-, -SO-, -SO₂- oder -NR(10a)- ersetzt sein kann;
   R(10a)
   Wasserstoff, Methyl oder Ethyl;
   R(4) R(14)-CᵣH₂ᵣ,
   wobei eine CH₂-Gruppe der Gruppe CᵣH₂ᵣ ersetzt sein kann durch -O-, - CH=CH-, -C≡C-, -CO-, -CO-O-, -O-CO-, -S-, -SO-, -SO₂-, -NR(10b)- oder -CONR(10b)-;
   R(10b)
   Wasserstoff oder Alkyl mit 1, 2 oder 3 C-Atomen;
   R(14)
   Methyl, CF₃, C₂F₅, C₃F₇, Cycloalkyl mit 3, 4, 5, 6, 7 oder 8 C-Atomen, -OH, -COOH, -NR(23)R(24), 1-Piperidyl, 1-Pyrrolidinyl, 4-Morpholinyl, 4-Methylpiperazin-1-yl, Pyridyl, Thienyl, Imidazolyl oder Phenyl,
   wobei Pyridyl, Thienyl, Imidazolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(23) und R(24)
   unabhängig voneinander Wasserstoff oder Alkyl mit 1, 2 oder 3 C-Atomen;
   oder
   R(23) und R(24)
   gemeinsam eine Kette von 4 oder 5 Methylengruppen, von denen eine CH₂-Gruppe durch -O-, -S-, -NH-, -N(CH₃)- oder
   -N(Benzyl)- ersetzt sein kann;
   r Null, 1, 2, 3, 4, 5, 6, 7, 8, 9, 10, 11, 12, 13, 14, 15, 16, 17, 18, 19 oder 20;
   R(5) und R(6)
   gemeinsam eine Gruppe
   -CR(15)=CR(16)-CR(17)=CR(18)-,
   -CR(15)=CR(16)-CR(17)=N-,
   -CR(15)=CR(16)-N=CR(18)-,
   -CR(15)=N-CR(17)=N-,
   -CR(15)=N-N=CR(18)-,
   -N=CR(16)-CR(17)=N- oder
   -S-CR(15)=CR(16)-,
   wobei jeweils beide Verknüpfungsrichtungen möglich sind;
   R(15), R(16), R(17) und R(18)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Z-CₛH₂ₛ-R(22), Thienyl oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Z -O-, -CO-, -CO-O-, -O-CO-, -S-, -SO-, -SO₂-, -SO₂-O-, -SO₂NR(10c), -NR(10c)- oder -CONR(10c)-;
   R(10c) Wasserstoff oder Alkyl mit 1, 2 oder 3 C-Atomen;
   s Null, 1, 2, 3, 4, 5 oder 6;
   R(22) Wasserstoff, CF₃, C₂F₅, C₃F₇, Cycloalkyl mit 3, 4, 5, 6, 7 oder 8 C-Atomen, -NR(19)R(20), -COOR(21), 1-Piperidyl, 1-Pyrrolidinyl, 4-Morpholinyl, 4-Methylpiperazin-1-yl, Pyridyl, Thienyl, Imidazolyl, Chinolyl, Isochinolyl oder Phenyl,
   wobei Pyridyl, Thienyl, Imidazolyl, Chinolyl, Isochinolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(19) und R(20)
   unabhängig voneinander Wasserstoff oder Alkyl mit 1, 2 oder 3 C-Atomen;
   oder
   R(19) und R(20)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(21) Wasserstoff oder Alkyl mit 1, 2 oder 3 C-Atomen;
   wobei jedoch nicht gleichzeitig Y gleich CR(11) und X gleich O sein kann; sowie ihre physiologisch verträglichen Salze;
   oder
3) EP 857 724 (HOE 97/F 024)
   Verbindungen der Formel I 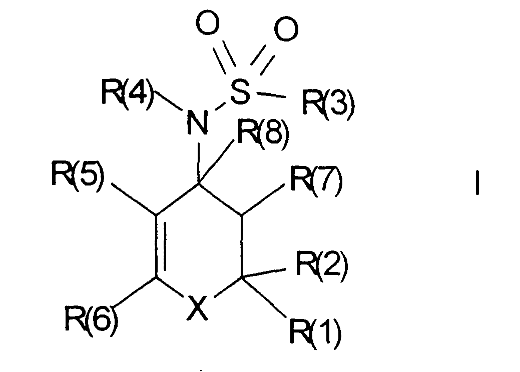 worin bedeuten:
   X -[S(O)_{Null, 1 oder 2}]-, -NR(9)-, -[CR(9)R(23)]- oder -CO-;
   R(9) Wasserstoff oder -(CₙH₂ₙ)-R(10);
   n Null, 1, 2, 3, 4, 5, 6, 7 oder 8;
   R(10) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
   wobei eine CH₂-Gruppe der Gruppe CₙH₂ₙ ersetzt sein kann durch -O-,
   -CH=CH-, -C=C-, -CO-, -CO-O-, -[SO_{Null, 1 oder 2}]- oder -NR(11)-;
   R(11) Wasserstoff, Methyl oder Ethyl;
   oder
   R(10) Pyridyl, Thienyl, Imidazolyl oder Phenyl,
   welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
   oder
   R(9) gemeinsam mit R(1) eine Bindung;
   R(23) Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
   R(24) Wasserstoff, Methyl oder Ethyl;
   R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   oder
   R(1) und R(2)
   gemeinsam eine Alkylenkette mit 2, 3, 4, 5, 6, 7, 8, 9 oder 10 C-Atomen;
   R(3) R(12)-CₐH₂ₐ[NR(13)]ₘ-;
   R(12) Wasserstoff oder Cycloalkyl mit 3, 4, 5, 6, 7 oder 8 C-Atomen, CF₃, C₂F₅ oder C₃F₇,
   a Null, 1, 2, 3, 4, 5, 6, 7, 8, 9 oder 10;
   m Null oder 1;
   R(13) Wasserstoff oder Alkyl mit 1, 2, 3, 4, 5 oder 6 C-Atomen;
   oder
   R(12) und R(13)
   gemeinsam eine Alkylengruppe mit 4, 5, 6, 7 oder 8 C-Atomen,
   wobei eine CH₂-Gruppe der Alkylengruppe durch -O-,
   -[SO_{Null, 1 oder 2}]-, -CO- oder -NR(11)- ersetzt sein kann;
   R(11) Wasserstoff, Methyl oder Ethyl;
   R(4) R(14)-CᵣH₂ᵣ;
   r 1, 2, 3, 4, 5, 6, 7, 8, 9, 10, 11, 12, 13, 14, 15, 16, 17, 18, 19 oder 20;
   R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
   wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
   oder
   R(3) und R(4)
   gemeinsam eine Alkylenkette mit 3, 4, 5, 6, 7 oder 8 C-Atomen,
   wobei eine CH₂-Gruppe der Alkylenkette durch -O-, -[SO_{Null, 1 oder 2}]-, - CO- oder -NR(11)- ersetzt sein kann;
   R(5) und R(6)
   gemeinsam -CR(15)=CR(16)-CR(17)=CR(18)-, -CR(15)=CR(16)-CR(17)=N-, - CR(15)=CR(16)-N=CR(18)-, -CR(15)=N-CR(17)=N-, -CR(15)=N-N=CR(18)-, -N=CR(16)-CR(17)=N- oder -S-CR(15)=CR(16)-;
   R(15), R(16), R(17) und R(18)
   unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   R(19) und R(20)
   unabhängig voneinander Wasserstoff oder Alkyl mit 1, 2 oder 3 C-Atomen;
   R(21) Wasserstoff, Methyl, Ethyl, Phenyl oder
   -CᵤH₂ᵤ-NR(19)R(20);
   u 2 oder 3;
   wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
   R(22) Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
   s Null, 1, 2, 3, 4, 5 oder 6;
   Z -[S(O)_{Null, 1 oder 2}]-, -CO-, -SO₂-NR(11)-, -SO₂-O-, -O-, -NR(11)- oder -[CO-NR(11)]-;
   R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
   R(8) Wasserstoff oder Alkyl mit 1, 2, 3, 4, 5 oder 6 C-Atomen;
   und deren physiologisch verträgliche Salze;
   oder
4) EP 860 440 (HOE 97/F033)
   Chroman-Derivate der Formel I 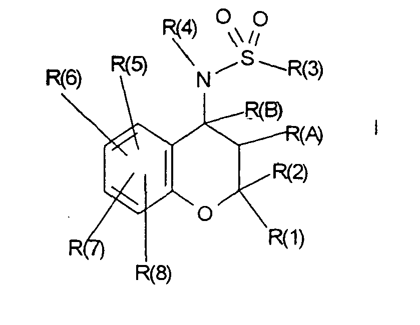 worin bedeuten:
   R(1)und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   oder
   R(1) und R(2)
   gemeinsam eine Alkylenkette mit 2, 3, 4, 5, 6, 7, 8, 9 oder 10 C-Atomen; R(A) Hydroxy, Alkanoyloxy mit 1, 2, 3, 4, 5 oder 6 C-Atomen oder Alkylsulfonyloxy
   mit 1, 2, 3, 4, 5 oder 6 C-Atomen;
   R(B) Wasserstoff;
   oder
   R(A) und R(B)
   zusammen eine Bindung;
   R(3) R(9)-CₙH₂ₙ[NR(11)]ₘ-;
   R(9) Wasserstoff oder Cycloalkyl mit 3, 4, 5, 6, 7 oder 8 C-Atomen;
   n Null, 1, 2, 3, 4, 5, 6, 7, 8, 9 oder 10;
   m Null oder 1;
   R(11) Wasserstoff oder Alkyl mit 1, 2, 3, 4, 5 oder 6 C-Atomen;
   oder
   R(11) und R(9)
   gemeinsam eine Alkylengruppe mit 1, 2, 3, 4, 5, 6, 7 oder 8 C-Atomen;
   wobei eine CH₂-Gruppe der Gruppe CₙH₂ₙ durch -O-, SO_{Null, 1 oder 2}- oder -NR(10) ersetzt sein kann;
   R(10) Wasserstoff, Methyl oder Ethyl;
   R(4) R(12)-CᵣH₂ᵣ;
   R(12) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
   wobei Pyridyl, Thienyl, Imidazolyl oder Phenyl unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
   r Null, 1, 2, 3, 4, 5, 6, 7, 8, 9, 10, 11, 12, 13, 14, 15, 16, 17, 18, 19 oder 20;
   wobei eine CH₂-Gruppe der Gruppe CᵣH₂ᵣ ersetzt sein kann durch -O-, >CH=CH<, -C≡C-, -CO-, -CO-O-, SO_{Null, 1 oder 2}- oder -NR(10)-;
   R(5), R(6), R(7) und R(8)
   unabhängig voneinander Wasserstoff, F, Cl, Br, I, Alkyl mit 1, 2, 3, 4, 5 oder 6 C-Atomen, Cycloalkyl mit 3, 4, 5, 6, 7 oder 8 C-Atomen, -CN, -CF₃, -C₂F₅, -C₃F₇, -N₃, -NO₂, -CONR(13)R(14), -COOR(15), R(16)-CₛH₂ₛ-Y- oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl-,
   R(13) und R(14)
   unabhängig voneinander Wasserstoff oder Alkyl mit 1, 2 oder 3 C-Atomen;
   R(15) Wasserstoff, Methyl, Ethyl, Phenyl oder -CᵤH₂ᵤ-NR(13)R(14);
   u 2 oder 3;
   R(16) Wasserstoff, Cycloalkyl mit 3, 4, 5, 6, 7 oder 8 C-Atomen, -COOR(15), Thienyl, lmidazolyl, Pyridyl, Chinolyl, lsochinolyl, Piperidyl, 1-Pyrrolidinyl, N-Morpholino, N-Methylpiperazino, CF₃, C₂F₅, C₃F₇ oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   s Null, 1, 2, 3, 4, 5 oder 6;
   Y -S-, -SO-, -SO₂₋, -CO-, -SO₂-NR(10)-, -O-, -NR(10)- oder -CO-NR(10);
   jedoch mit der Bedingung, daß zwei der Substituenten R(5), R(6), R(7) und R(8) nicht Wasserstoff sind;
   und deren physiologisch verträgliche Salze;
   oder
5) EP 861 836 (HOE 97/F038)
   Verbindungen der Formel I, 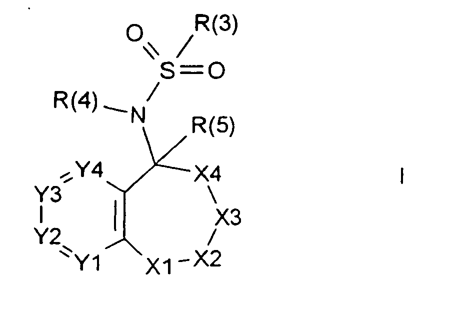 worin bedeuten:
   X1 -O-, -S-, -SO-, -SO₂-, -CR(1)R(2)-, -NR(6)-, -CO- oder -CR(1)R(7)-;
   R(1) und R(2)
   unabhängig voneinander Wasserstoff, CF₃, C₂F₅, C₃F₇, Alkyl
   mit 1, 2, 3, 4, 5 oder 6 C-Atomen oder Phenyl,
   das unsubstituiert ist oder substituiert ist mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(1) und R(2)
   gemeinsam eine Alkylenkette mit 2, 3, 4, 5, 6, 7, 8, 9 oder 10 C-Atomen;
   R(6) Wasserstoff oder -CₙH₂ₙ-R(8),
   wobei eine CH₂-Gruppe der Gruppe CₙH₂ₙ ersetzt sein kann durch -O-, -CH=CH-, -C≡C-, -CO-, -CO-O-, -O-CO-, -S-, -SO-, -SO₂-, -NR(9)- oder -CONR(9)-;
   R(9) Wasserstoff oder Alkyl mit 1, 2 oder 3 C-Atomen;
   n Null, 1, 2, 3, 4, 5, 6, 7 oder 8;
   R(8) Wasserstoff, CF₃, C₂F₅, C₃F₇, Cycloalkyl mit 3, 4, 5, 6, 7 oder 8 C-Atomen, Dimethylamino, Diethylamino, 1-Piperidyl, 1-Pyrrolidinyl, 4-Morpholinyl, 4-Methylpiperazin-1-yl, Pyridyl, Thienyl, Imidazolyl oder Phenyl,
   wobei Pyridyl, Thienyl, Imidazolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   X2 -CR(1)R(2)- oder -CR(2)R(10)-;
   oder
   X2 sofern X3 und X1 -CR(1)R(2)- bedeuten, auch -O-, -S-, -SO-, -SO₂- oder -NR(6)-,
   wobei die Reste R(1), R(2) und R(6)
   wie bei X1 angegeben definiert sind, aber unabhängig von den Bedeutungen der Reste in X1 sind;
   R(10) gemeinsam mit R(7) eine Bindung;
   X3 -CR(1)R(2)-;
   oder
   X3 sofern X2 und X4 -CR(1)R(2)- bedeuten, auch -O-, -S-, -SO-, -SO₂- oder -NR(6)-,
   wobei die Reste R(1), R(2) und R(6)
   wie bei X1 angegeben definiert sind, aber unabhängig von den Bedeutungen der Reste in X1 sind;
   X4 -CR(1)R(2)-, -NR(6)-, -NR(11)-, -CH(OR(30))- oder -CR(2)R(11)-,
   wobei die Reste R(1), R(2) und R(6)
   wie bei X1 angegeben definiert sind, aber unabhängig von den Bedeutungen der Reste in X1 sind;
   R(30) Wasserstoff, Alkyl mit 1, 2 oder 3 C-Atomen oder Acyl mit 1, 2, 3 oder 4 C-Atomen;
   R(11) gemeinsam mit R(5) eine Bindung;
   Y1, Y2, Y3 und Y4
   unabhängig voneinander -CR(12)- oder N,
   wobei maximal 2 der Gruppen Y1, Y2, Y3 und Y4 gleichzeitig N bedeuten können;
   die Reste R(12)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, C₂F₅, C₃F₇, N₃, NO₂, -Z-CₘH₂ₘ-R(13) oder Phenyl,
   das unsubstituiert ist oder substituiert ist mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Z -O-, -CO-, -CO-O-, -O-CO-, -S-, -SO-, -SO₂-, -SO₂NR(14)-,
   -NR(14)- oder -CONR(14)-;
   R(14) Wasserstoff oder Alkyl mit 1, 2 oder 3 C-Atomen;
   m Null, 1, 2, 3, 4, 5 oder 6;
   R(13) Wasserstoff, CF₃, C₂F₅, C₃F₇, Cycloalkyl mit 3, 4, 5, 6, 7 oder 8 C-Atomen, -NR(15)R(16), -CONR(15)R(16), -OR(30a), Phenyl, Thienyl oder einen N-haltigen Heterocyclus mit 1, 2, 3, 4, 5, 6, 7, 8 oder 9 C-Atomen,
   wobei Phenyl, Thienyl und der N-haltige Heterocyclus unsubstituiert sind oder substituiert sind mit 1 oder 2
   Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(15) und R(16)
   unabhängig voneinander Wasserstoff oder Alkyl mit 1, 2 oder 3 C-Atomen;
   oder
   R(15) und R(16)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(30a)
   Wasserstoff, Alkyl mit 1, 2 oder 3 C-Atomen oder Acyl mit 1, 2, 3 oder 4 C-Atomen;
   oder
   Y1 und Y2
   gemeinsam ein S-Atom und Y3 und Y4 jeweils -CR(12)-;
   die Reste R(12)
   unabhängig voneinander wie bei Y1, Y2, Y3, Y4 definiert;
   R(3) R(17)-CₓH₂ₓ-NR(18)- oder R(17)-CₓH₂ₓ-,
   wobei eine CH₂-Gruppe in den Gruppen CₓH₂ₓ ersetzt sein kann durch -O-, -CO-, -S-, -SO-, -SO₂- oder -NR(19)-;
   R(19) Wasserstoff, Methyl oder Ethyl;
   R(17) Wasserstoff, Methyl, Cycloalkyl mit 3, 4, 5, 6, 7 oder 8 C-Atomen, CF₃, C₂F₅ oder C₃F₇;
   x Null, 1, 2, 3, 4, 5, 6, 7, 8, 9 oder 10;
   R(18) Wasserstoff oder Alkyl mit 1, 2, 3, 4, 5, 6, 7 oder 8 C-Atomen;
   oder
   R(18) und R(17)
   gemeinsam eine Bindung, sofern x nicht kleiner als 3 ist;
   oder
   R(3) Phenyl,
   das unsubstituiert ist oder substituiert ist mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(3) gemeinsam mit R(4)
   eine Alkylenkette mit 3, 4, 5, 6, 7, oder 8 C-Atomen,
   wobei eine CH₂-Gruppe der Alkylenkette durch -O-, -CO-, -S-, -SO- oder SO₂- ersetzt sein kann;
   R(4) -CᵣH₂ᵣ-R(20),
   wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(21)- oder -CONR(21)-;
   R(21) Wasserstoff oder Alkyl mit 1, 2 oder 3 C-Atomen;
   r Null, 1, 2, 3, 4, 5, 6, 7, 8, 9, 10, 11, 12, 13, 14, 15, 16. 17, 18, 19 oder 20;
   R(20) Wasserstoff, Methyl, CF₃, C₂F₅, C₃F₇, Cycloalkyl mit 3, 4, 5, 6, 7 oder 8 C-Atomen, -NR(22)R(23), Phenyl, Thienyl oder einen N-haltigen Heterocyclus mit 1, 2, 3, 4, 5, 6, 7, 8 oder 9 C-Atomen,
   wobei Phenyl, Thienyl und der N-haltige Heterocyclus unsubstituiert sind oder substituiert sind mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(22) und R(23)
   unabhängig voneinander Wasserstoff oder Alkyl mit 1, 2 oder 3 C-Atomen;
   oder
   R(22) und R(23)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(5) Wasserstoff oder gemeinsam mit R(11) eine Bindung;
   in allen ihren stereoisomeren Formen und Gemische davon in beliebigen Verhältnissen, sowie ihre physiologisch verträglichen Salze;
   oder
6) HMR 97/L206 - EP 895 994
   Verbindungen der Formel I 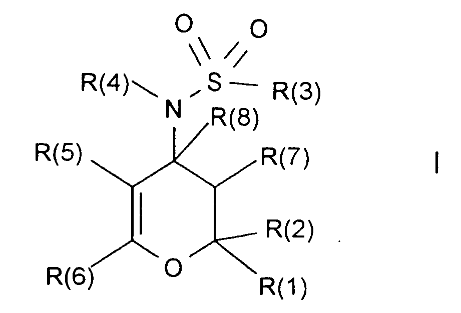 worin bedeuten:
   R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   oder
   R(1) und R(2)
   gemeinsam eine Alkylenkette mit 2, 3, 4, 5, 6, 7, 8, 9 oder 10 C-Atomen;
   wobei eine CH₂-Gruppe der Alkylenkette durch -O-, -CO-, -S-, -SO-,
   -SO₂- oder -NR(10)- ersetzt sein kann;
   R(10) Wasserstoff oder Alkyl mit 1, 2 oder 3 C-Atomen;
   R(3) R(12)-CₐH₂ₐ(NR(13)]ₘ-;
   R(12) Wasserstoff oder Cycloalkyl mit 3, 4, 5, 6, 7 oder 8 C-Atomen, CF₃, C₂F₅ oder C₃F₇;
   a Null, 1, 2, 3, 4, 5, 6, 7, 8, 9 oder 10;
   m Null oder 1;
   R(13) Wasserstoff oder Alkyl mit 1, 2, 3, 4, 5 oder 6 C-Atomen;
   oder
   R(12) und R(13)
   gemeinsam eine Alkylengruppe mit 4, 5, 6, 7 oder 8 C-Atomen,
   wobei eine CH₂-Gruppe der Alkylengruppe durch -O-, -[SO_{Null, 1 oder 2}]-, -CO- oder -NR(10)- ersetzt sein kann;
   R(10) Wasserstoff oder Alkyl mit 1, 2 oder 3 C-Atomen;
   R(4) R(14)-CᵣH₂ᵣ;
   r Null, 1, 2, 3, 4, 5, 6, 7, 8, 9, 10, 11, 12, 13, 14, 15, 16, 17, 18, 19 oder 20;
   R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
   wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
   R(11) Wasserstoff oder -(CₐH₂ₐ)-R(10);
   wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
   R(10) Wasserstoff oder Alkyl mit 1, 2 oder 3 C-Atomen;
   oder
   R(3) und R(4)
   gemeinsam eine Alkylenkette mit 3, 4, 5, 6, 7 oder 8 C-Atomen,
   wobei eine CH₂-Gruppe der Alkylenkette durch -O-, -[SO_{Null, 1 oder 2}]-, -CO- oder -NR(11)- ersetzt sein kann;
   R(11) Wasserstoff oder -(CₐH₂ₐ)-R(10),
   wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
   R(10) Wasserstoff oder Alkyl mit 1, 2 oder 3 C-Atomen;
   R(5) und R(6)
   -CR(15)=CR(16)-CR(17)=N-,
   -CR(15)=CR(16)-N=CR(17)-,
   -CR(15)=N-CR(17)=N-,
   -CR(15)=N-N=CR(17)-,
   -N=CR(16)-CR(17)=N- oder
   -S-CR(15)=CR(16)-;
   R(15), R(16) und R(17) -
   unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(21), -COOR(21), R(22)-CₛH₂ₛ-Z- oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   R(19) Wasserstoff oder Alkyl mit 1, 2 oder 3 C-Atomen;
   R(21) Wasserstoff, Methyl, Ethyl, Phenyl oder -CᵤH₂ᵤ-NR(19)R(20);
   wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl; R(20)
   Wasserstoff oder Alkyl mit 1, 2 oder 3 C-Atomen;
   u 2 oder 3;
   R(22) Wasserstoff, Cycloalkyl mit 3, 4, 5, 6, 7 oder 8 C-Atomen, -COOR(21), CONR(19)R(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅, C₃F₇ oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   s Null, 1, 2, 3, 4, 5 oder 6;
   Z -[S(O)_{Null, 1 oder 2]}-, -CO-, -SO_{(0,1 oder 2)}-NR(11)-, -SO₂-O-, -O-, -NR(11)- oder -[CO-NR(11)]-;
   R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
   R(8) Wasserstoff oder Alkyl mit 1, 2, 3, 4, 5 oder 6 C-Atomen;
   und deren physiologisch verträgliche Salze;
   oder
7) HMR 97/L223 EP-OS 905 131
   Verbindungen der Formel I, 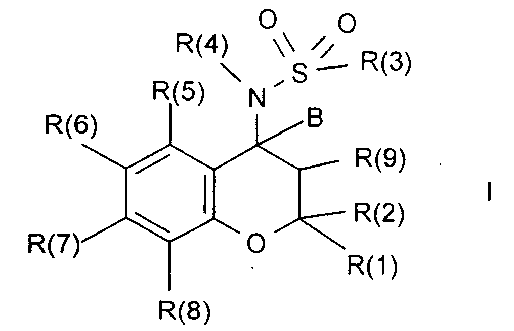 worin bedeuten:
   R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(1) und R(2)
   gemeinsam eine Alkylenkette mit 2, 3, 4, 5, 6, 7, 8, 9 oder 10 C-Atomen;
   R(3) R(10)-CₙH₂ₙ-NR(11)- oder R(10)-CₙH₂ₙ-,
   wobei eine CH₂-Gruppe in den Gruppen CₙH₂ₙ ersetzt sein kann durch -O-, -CO-, -S-, -SO-, -SO₂- oder -NR(12a)-;
   R(12a) Wasserstoff, Methyl oder Ethyl;
   R(10) Wasserstoff, Methyl, Cycloalkyl mit 3, 4, 5, 6, 7 oder 8 C-Atomen, CF₃, C₂F₅ oder C₃F₇;
   n Null, 1, 2, 3, 4, 5, 6, 7, 8, 9 oder 10;
   R(11) Wasserstoff oder Alkyl mit 1, 2, 3, 4, 5 oder 6 C-Atomen;
   oder
   R(10) und R(11)
   gemeinsam eine Bindung, sofern n nicht kleiner als 3 ist;
   R(4) R(13)-CᵣH₂ᵣ-Z-C_{q}H_{2q}-;
   q 0, 1, 2, 3, 4, 5, 6, 7 oder 8;
   r 0, 1, 2, 3, 4, 5, 6, 7 oder 8;
   Z -CO-NR(14)-,
   -OCO-NR(14)-,
   -O-CₓH₂ₓ-O-,
   -O-CₓH₂ₓ-NR(14)-,
   -O-CₓH₂ₓ-CO-O,
   -CO-O-CₓH₂ₓ-O- oder
   -CO-O-CₓH₂ₓ-NR(14)-,
   wobei jeweils beide Verknüpfungsrichtungen möglich sind;
   x 2, 3 oder 4;
   R(14) Wasserstoff, Alkyl mit 1, 2 oder 3 C-Atomen, -C_{y}H_{2y}-OR(12b), -C_{y}H_{2y}-NR(12b)₂;
   R(12b) Wasserstoff, Methyl oder Ethyl;
   y 2 oder 3;
   R(13) H, CF₃, C₂F₅, C₃F₇, Cycloalkyl mit 3, 4, 5, 6, 7 oder 8 C-Atomen,
   -NR(15)R(16), -CONR(15)R(16), -C(=NR(17))NR(15)R(16), -OR(17), - COOR(17), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, 1, CF₃, NO₂, CN, NH₂, OH, Methyl, Ethyl, Methoxy, Dimethylamino, Sulfamoyl, Methylsulfonyl, Aminosulfonyl und Methylsulfonylamino;
   R(15) und R(16)
   unabhängig voneinander Wasserstoff, Alkyl mit 1, 2, 3 oder 4 C-Atomen oder -C_{z}H_{2z}-phenyl,
   worin Phenyl unsubstituiert ist oder substituiert mit 1 oder 2 Substituenten ausgewählt aus der Gruppe bestehend aus F, Cl, Br, CF₃, NO₂, CN OH, Methyl, Ethyl, Methoxy, Dimethylamino, Sulfamoyl, Methylsulfonyl, Aminosulfonyl und Methylsulfonylamino;
   oder
   R(15) und R(16)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(17) Wasserstoff oder Alkyl mit 1, 2 oder 3 C-Atomen;
   R(5), R(6), R(7) und R(8)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18) oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Y -O-, -CO-, -CO-O-, -O-CO-, -S-, -SO-, -SO₂-, -SO₂-O-, -SO₂NR(10c), - NR(10c)- oder -CONR(10c)-;
   R(10c) Wasserstoff oder Alkyl mit 1, 2 oder 3 C-Atomen;
   s Null, 1, 2, 3, 4, 5 oder 6;
   R(18) Wasserstoff, CF₃, C₂F₅, C₃F₇, Cycloalkyl mit 3, 4, 5, 6, 7 oder 8 C-Atomen, -COOR(21), 1-Piperidyl, 1-Pyrrolidinyl, 4-Morpholinyl, 4-Methylpiperazin-1-yl, Pyridyl, Thienyl, Imidazolyl, Chinolyl, Isochinolyl oder Phenyl,
   wobei Pyridyl, Thienyl, Imidazolyl, Chinolyl, Isochinolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino ;
   R(21) Wasserstoff oder Alkyl mit 1, 2 oder 3 C-Atomen;
   R(9) Wasserstoff, OR(10d) oder OCOR(10d);
   R(10d) Wasserstoff oder Alkyl mit 1, 2 oder 3 C-Atomen;
   B Wasserstoff;
   oder
   R(9) und B
   gemeinsam eine Bindung;
   sowie ihre physiologisch verträglichen Salze;
   oder
8) HMR 97/L224 - EP-OS 906 911
   Verbindungen der Formel I, 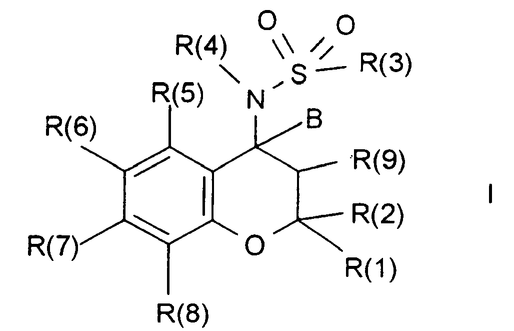 worin bedeuten:
   R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(1) und R(2) gemeinsam eine Alkylenkette mit 2, 3, 4, 5, 6, 7, 8, 9 oder 10 C-Atomen;
   R(3) R(10)-CₙH₂ₙ-NR(11)- oder R(10)-CₙH₂ₙ-,
   wobei eine CH₂-Gruppe in den Gruppen CₙH₂ₙ ersetzt sein kann durch -O-, -CO-, -S-, -SO-, -SO₂- oder -NR(12a)-;
   R(12a) Wasserstoff, Methyl oder Ethyl;
   R(10) Wasserstoff, Methyl, Cycloalkyl mit 3, 4, 5, 6, 7 oder 8 C-Atomen, CF₃, C₂Fs oder C₃F₇;
   n Null, 1, 2, 3, 4, 5, 6, 7, 8, 9 oder 10;
   R(11) Wasserstoff oder Alkyl mit 1, 2, 3, 4, 5 oder 6 C-Atomen;
   oder
   R(10) und R(11)
   gemeinsam eine Bindung, sofern n nicht kleiner als 3 ist;
   oder
   R(3) gemeinsam mit R(4)
   eine Alkylenkette mit 3, 4, 5, 6, 7 oder 8 C-Atomen,
   wobei eine CH₂-Gruppe der Alkylenkette durch -O-, -CO-, -S-, -SO-, -SO₂- oder -NR(12a)- ersetzt sein kann;
   R(12a) Wasserstoff, Methyl oder Ethyl;
   R(4) R(13)-CᵣH₂ᵣ.
   wobei eine CH₂-Gruppe der Gruppe CᵣH₂ᵣ ersetzt sein kann durch -O-, - CH=CH-, -C≡C-, -CO-, -CO-O-, -O-CO-, -S-, -SO-, -SO₂-, -NR(14)- oder -CONR(14)-;
   R(14) Wasserstoff, Alkyl mit 1, 2 oder 3 C-Atomen, -C_{y}H_{2y}-OR(12b), -CyH₂y-NR(12b)₂;
   R(12b) Wasserstoff, Methyl oder Ethyl;
   y 2 oder 3;
   R(13) H, CF₃, C₂F₅, C₃F₇, Cycloalkyl mit 3, 4, 5, 6, 7 oder 8 C-Atomen,
   -NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(15) und R(16)
   unabhängig voneinander Wasserstoff oder Alkyl mit 1, 2, 3 oder 4 C-Atomen;
   oder
   R(15) und R(16)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(17) Wasserstoff, Alkyl mit 1, 2 oder 3 C-Atomen, -CₓH₂ₓOR(12c);
   R(12c) Wasserstoff, Methyl oder Ethyl;
   x 2 oder 3;
   r Null, 1, 2, 3, 4, 5, 6, 7, 8, 9, 10, 11, 12, 13, 14, 15, 16, 17, 18, 19 oder 20;
   mindestens einer der Substituenten R(5), R(6), R(7) und R(8)
   -Y-CₛH₂ₛ-R(18), Thienyl, Furyl oder ein N-haltiger Heterocyclus mit 1, 2, 3, 4, 5, 6, 7, 8 oder 9 C-Atomen,
   wobei Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Methylamino, Dimethylamino, Ethylamino, Diethylamino, Sulfamoyl, Methylsulfonyl und Methylsulfonylamino;
   Y -O-, -CO-, -O-CO-, -S-, -SO-, -SO₂-, -SO₂-O-, -SO₂NR(12d)-, -NR(12d)- oder -CONR(12d)-,
   wobei die Anknüpfung an den Benzolkern jeweils über das links stehende Atom erfolgt;
   R(12d) Wasserstoff, Methyl oder Ethyl;
   s 1, 2, 3, 4, 5 oder 6;
   R(18) substituiertes Phenyl, das einen oder zwei Substituenten trägt ausgewählt aus der Gruppe bestehend aus NO₂, CN, NH₂, N(Methyl)₂, OH, Ethyl, -COOH, -COOMethyl, -COOEthyl, -CONH₂, -CON(Methyl)₂ ;
   oder
   R(18) ein substituierter N-haltiger Heterocyclus mit 1, 2, 3, 4, 5, 6, 7, 8 oder 9 C-Atomen, der einen oder 2 Substituenten trägt ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(18) -OR(19), -SO₂R(19), -NR(19)R(20), -CONR(19)R(20);
   R(19) und R(20)
   unabhängig voneinander CₜH₂ₜ-R(21);
   t Null, 1, 2, 3, 4, 5 oder 6;
   R(21) Wasserstoff, CF₃, C₂F₅, C₃F₇, Cycloalkyl mit 3, 4, 5, 6, 7 oder 8 C-Atomen, NR(22)R(23), -OR(24), Phenyl, Thienyl oder ein N-haltiger Heterocyclus mit 1, 2, 3, 4, 5, 6, 7, 8 oder 9 C-Atomen,
   wobei Phenyl, Thienyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(22) und R(23)
   unabhängig voneinander Wasserstoff, Alkyl
   mit 1, 2 oder 3 C-Atomen;
   oder
   R(22) und R(23)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(24) Wasserstoff, Alkyl mit 1, 2 oder 3 C-Atomen;
   und die jeweils übrigen Substituenten R(5), R(6), R(7) und R(8), die noch nicht durch die zuvor gegebene Definition vergeben sind,
   unabhängig voneinander Wasserstoff, F, Cl, Br, 1, Alkyl mit 1, 2, 3, 4 oder 5 C-Atomen, Cycloalkyl mit 3, 4, 5, 6, 7 oder 8 C-Atomen, CN, CF₃, NO₂, OR(12e) oder NR(12e)R(12f); R(12e) und R(12f)
   unabhängig voneinander Wasserstoff oder Alkyl mit 1, 2, 3 oder 4 C-Atomen;
   R(9) Wasserstoff, OR(12g) oder OCOR(12g);
   R(12g) Wasserstoff oder Alkyl mit 1, 2 oder 3 C-Atomen;
   B Wasserstoff;
   oder
   R(9) und B
   gemeinsam eine Bindung;
   sowie ihre physiologisch verträglichen Salze;
   oder
9) HMR 97/L232 - EP 913 396
   Verbindungen der Formel I, 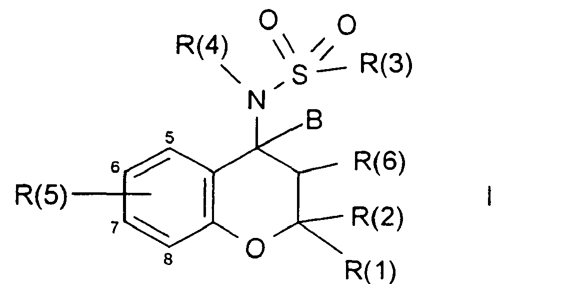 worin R(5) in einer der mit 5, 6, 7 und 8 gekennzeichneten Positionen gebunden ist und worin bedeuten:
   R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(1) und R(2)
   gemeinsam eine Alkylenkette mit 2, 3, 4, 5, 6, 7, 8, 9 oder 10 C-Atomen;
   R(3) R(10)-CₙH₂ₙ-NR(11)- oder R(10)-CₙH₂ₙ-,
   wobei eine CH₂-Gruppe in den Gruppen CₙH₂ₙ ersetzt sein kann durch -O-, -CO-, -S-, -SO-, -SO₂- oder -NR(12a)-;
   R(12a) Wasserstoff, Methyl oder Ethyl;
   R(10) Wasserstoff, Methyl, Cycloalkyl mit 3, 4, 5, 6, 7 oder 8 C-Atomen, CF₃, C₂F₅ oder C₃F₇;
   n Null, 1,2, 3, 4, 5, 6, 7, 8, 9 oder 10;
   R(11) Wasserstoff oder Alkyl mit 1, 2, 3, 4, 5 oder 6 C-Atomen;
   oder
   R(10) und R(11)
   gemeinsam eine Bindung, sofern n nicht kleiner als 3 ist;
   oder
   R(3) gemeinsam mit R(4)
   eine Alkylenkette mit 3, 4, 5, 6, 7 oder 8 C-Atomen,
   wobei eine CH₂-Gruppe der Alkylenkette durch -O-, -CO-, -S-, -SO-, -SO₂- oder -NR(12a)- ersetzt sein kann;
   R(12a) Wasserstoff, Methyl oder Ethyl;
   R(4) R(13)-CᵣH₂ᵣ,
   wobei eine CH₂-Gruppe der Gruppe CᵣH₂ᵣ ersetzt sein kann durch -O-, - CH=CH-, -C≡C-, -CO-, -CO-O-, -O-CO-, -S-, -SO-, -SO₂- oder -NR(14)-;
   R(14) Wasserstoff oder Alkyl mit 1, 2 oder 3 C-Atomen;
   R(13) CH₃, CF₃, C₂F₅, C₃F₇, Cycloalkyl mit 3, 4, 5, 6, 7 oder 8 C-Atomen, NR(15)R(16), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(15) und R(16)
   gemeinsam eine Kette von 4 oder 5 Methylengruppen, von denen eine CH₂-Gruppe durch -O-, -S-, -NH-, -N(Methyl)- oder-N(Benzyl)- ersetzt sein kann;
   r Null, 1, 2, 3, 4, 5, 6, 7, 8, 9, 10, 11, 12, 13, 14, 15, 16, 17, 18, 19 oder 20;
   R(5) -Y-CₛH₂ₛ-R(18) oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Y -O-, -S- oder-NR(10c)-;
   R(10c) Wasserstoff oder Alkyl mit 1, 2 oder 3 C-Atomen;
   s 1, 2, 3, 4, 5, 6, 7 oder 8;
   R(18) Wasserstoff, CF₃, C₂F₅, C₃F₇, Cycloalkyl mit 3, 4, 5, 6, 7 oder 8 C-Atomen, -COOR(21), NR(15a)R(16a), ein unsubstituierter N-haltiger Heterocyclus mit 1, 2, 3, 4, 5, 6, 7, 8 oder 9 C-Atomen, Phenyl oder Thienyl,
   wobei Phenyl und Thienyl unsubstituiert sind oder substituiert mit 1 oder 2 Substituenten, ausgewählt aus der Gruppe bestehend aus F, Cl, Br, I, CF₃, Methyl, Methoxy, Sulfamoyl, Methylsulfonyl und Methylsulfonylamino;
   R(15a) und R(16a)
   gemeinsam eine Kette von 4 oder 5 Methylengruppen, von denen eine CH₂-Gruppe durch -O-, -S-, -NH-, -N(Methyl)- oder - N(Benzyl)- ersetzt sein kann;
   R(21) Wasserstoff oder Alkyl mit 1, 2 oder 3 C-Atomen;
   R(6) OR(10d) oder OCOR(10d);
   R(10d) Wasserstoff oder Alkyl mit 1, 2 oder 3 C-Atomen;
   B Wasserstoff;
   oder
   R(6) und B
   gemeinsam eine Bindung;
   sowie ihre physiologisch verträglichen Salze;
   oder
10. HMR 97/L235 - EP 915 087
   Verbindungen der Formel I, 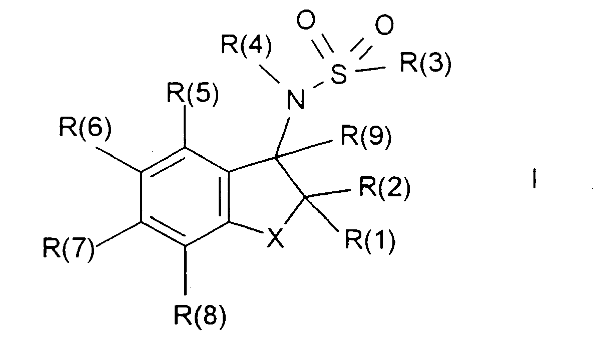 worin bedeuten:
   R(1) und R(2)
   unabhängig voneinander Wasserstoff, CF₃, Alkyl mit 1, 2, 3, 4, 5 oder 6 C-Atomen;
   oder
   R(2) und R(9)
   gemeinsam eine Bindung;
   oder R(2)
   -OR(10a), wenn X -CR(22)R(23)- bedeutet;
   R(10a) Wasserstoff, Acetyl oder Alkyl mit 1, 2 oder 3 C-Atomen;
   R(3) R(10b)-CₙH₂ₙ-NR(11)- oder R(10b)-CₙH₂ₙ-,
   wobei eine CH₂-Gruppe in den Gruppen CₙH₂ₙ ersetzt sein kann durch -O-, -CO-, -S-, -SO-, -SO₂- oder -NR(12a)-;
   R(12a) Wasserstoff, Methyl oder Ethyl;
   R(10b) Methyl, Cycloalkyl mit 3, 4, 5, 6, 7 oder 8 C-Atomen, CF₃, C₂F₅ oder C₃F₇;
   n Null, 1, 2, 3, 4, 5, 6, 7, 8, 9 oder 10;
   R(11) Wasserstoff oder Alkyl mit 1, 2, 3, 4, 5 oder 6 C-Atomen;
   oder
   R(10b) und R(11)
   gemeinsam eine Bindung, sofern n größer als 2 ist;
   oder
   R(3) gemeinsam mit R(4)
   eine Alkylenkette mit 3, 4, 5, 6, 7 oder 8 C-Atomen,
   wobei eine CH₂-Gruppe der Alkylenkette durch -O-, -CO-, -S-, -SO-, -SO₂- oder -NR(12a)- ersetzt sein kann;
   R(12a) Wasserstoff, Methyl oder Ethyl;
   R(4) R(13)-CᵣH₂ᵣ,
   wobei eine CH₂-Gruppe der Gruppe CᵣH₂ᵣ ersetzt sein kann durch -O-, - CH=CH-, -C≡C-, -CO-, -CO-O-, -O-CO-, -S-, -SO-, -SO₂-, -NR(14)- oder -CONR(14)-;
   R(14) Wasserstoff, Alkyl mit 1, 2 oder 3 C-Atomen, -C_{y}H_{2y} OR(12b),
   -C_{y}H_{2y}-NR(12b)₂;
   R(12b) Wasserstoff, Methyl oder Ethyl;
   y 2 oder 3;
   R(13) CH₃, CF₃, C₂F₅, C₃F₇, Cycloalkyl mit 3, 4, 5, 6, 7 oder 8 C-Atomen, -NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, l, CF₃, NO₂, CN, NH₂, OH, Methyl, Ethyl, Methoxy, Dimethylamino, Sulfamoyl, Methylsulfonyl und Methylsulfonylamino;
   R(15) und R(16)
   unabhängig voneinander Wasserstoff oder Alkyl mit 1, 2, 3 oder 4 C-Atomen;
   oder
   R(15) und R(16)
   gemeinsam eine Kette von 4 oder 5 Methylengruppen, von denen eine CH₂-Gruppe durch -O-, -S-, -NH-, -N(CH₃)- oder
   -N(Benzyl)- ersetzt sein kann;
   R(17) Wasserstoff, Alkyl mit 1, 2 oder 3 C-Atomen, -C_{z}H_{2z}OR(12c); R(12c) Wasserstoff, Methyl oder Ethyl;
   z 2 oder 3;
   r Null, 1, 2, 3, 4, 5, 6, 7, 8, 9, 10, 11, 12, 13, 14, 15, 16, 17, 18, 19 oder 20;
   R(5), R(6), R(7) und R(8)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18), Phenyl, Thienyl, Furyl oder ein N-haltiger Heterocyclus mit 1, 2, 3, 4, 5, 6, 7, 8 oder 9 C-Atomen,
   wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Y -O-, -CO-, -CO-O-, -O-CO-, -S-, -SO-, -SO₂-, -O-SO₂-, -SO₂NR(10c)- oder -CONR(10c)-, wobei die Verknüpfung mit dem Grundgerüst jeweils über das links stehende Atom erfolgt;
   R(10c) Wasserstoff oder Alkyl mit 1, 2 oder 3 C-Atomen;
   s Null, 1, 2, 3, 4, 5 oder 6;
   R(18) Wasserstoff, Methyl, CF₃, C₂F₅, C₃F₇, Cycloalkyl mit 3, 4, 5, 6, 7 oder 8 C-Atomen, -OR(21), -COOR(21), -NR(15a)R(16a), -CONR(15a)R(16a), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(15a) und R(16a)
   unabhängig voneinander Wasserstoff oder Alkyl mit 1, 2, 3 oder 4 C-Atomen;
   oder
   R(15a) und R(16a)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(21) Wasserstoff oder Alkyl mit 1, 2 oder 3 C-Atomen;
   R(9) Wasserstoff oder gemeinsam mit R(2) eine Bindung;
   X -CR(22)R(23)-, -O-, -NR(24)-, -S-, -SO-, -SO₂-;
   R(22) und R(23)
   unabhängig voneinander Wasserstoff, CF₃, Alkyl mit 1, 2, 3, 4, 5 oder 6 C-Atomen;
   R(24) Wasserstoff, Alkyl mit 1, 2, 3, 4, 5 oder 6 C-Atomen oder Phenyl, das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   sowie ihre physiologisch verträglichen Salze;
   oder
   WO96 05827, WO 96 40146, WO 96 05839, WO 95 14671, WO 97 49690, WO 95 14676, WO 95 14695, WO 95 14471, WO 98 00406, WO 97 48686, US 5 633 251, WO 96 40653
11. Verbindungen der Formel 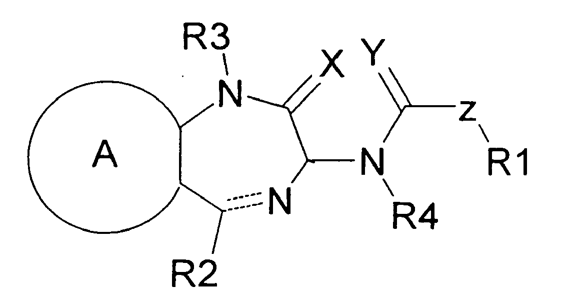 worin bedeuten:
   A Thienyl, Pyridyl, Phenyl, unsubstituiert oder substituiert;
   X O, S, =N-NH2, =N-OH, H2;
   Y O, NCN, H2;
   Z geradkettiges oder verzweigtes Alkyl, Alkenyl, unsubstituiert oder substituiert mit Phenyl, Cycloalkyl, wobei eine oder mehrere CH₂-Gruppen ersetzt sein können durch O, S, NH oder eine Bindung;
   R1 Phenyl, unsubstituiert oder substituiert, Alkyl oder Cycloalkyl, mono- oder bicyclischer Heterozyklus, Indanyl;
   R2 Phenyl, unsubstituiert oder substituiert, Alkyl, Cycloalkyl, 2- oder 3- Furyl, N-mono oder bis- Alkyl.
   R3 H, Alkyl, unsubstituiert oder substituiert mit N(CH₃)₂, OH; oder Fluoralkyl;
   R4 H, Alkyl welches durch ein oder zwei Sauerstoff-Atome unterbrochen sein kann;
   oder ihre pharmazeutisch verträglichen Salze.
   oder
   WO 95 14694, WO9514472
12. Verbindungen der Formel 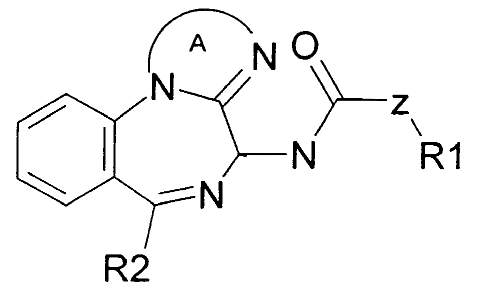 worin bedeuten:
   A eine 2- oder 3- gliedrige Kette, welche aus C-Atomen besteht, in der aber auch ein oder mehrere Glieder Stickstoff oder Sauerstoff sein können, welche Kette unsubstituiert oder mit Alkyl substituiert ist;
   Z geradkettiges oder verzweigtes Alkyl oder Alkenyl,
   welche unsubstituiert oder substituiert sind mit Phenyl, Cycloalkyl, wund worin eine oder mehrere CH₂-Gruppen durch O, S, NH oder eine Bindung oder N- Alkyl oder N- Phenyl ersetzt sein können;
   R1 Phenyl, unsubstituiert oder substituiert, Alkyl oder Cycloalkyl, mono- oder bicyclischer Heterozyklus;
   R2 Phenyl,
   unsubstituiert oder substituiert;
   oder
   R2 Alkyl, Cycloalkyl, 2- oder 3- Furyl, N-mono oder bis- Alkyl.;
   oder ihre pharmazeutisch verträglichen Salze;
   oder
13. WO 95 14473
   Verbindungen der Formel 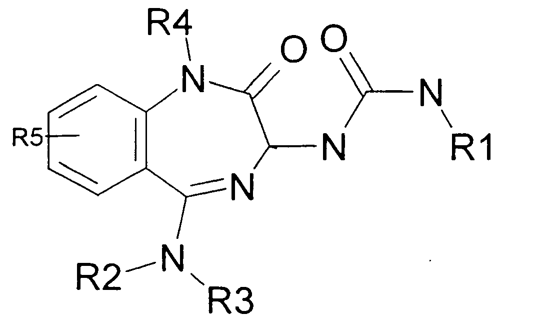 worin bedeuten:
   R1 Phenyl,
   unsubstituiert oder substituiert;
   oder
   R1 Alkyl oder Cycloalkyl, mono- oder bicyclischer Heterozyklus, Indanyl;
   R2 und R3
   unabhängig von einander Alkyl, das unsubstituiert ist oder substituiert mit Phenyl,
   oder
   R2 und R3 Cycloalkyl
   oder
   R2 und R3
   gemeinsam ein Azacyclus;
   R4 Alkyl, unsubstituiert oder substituiert mit Phenyl,
   oder
   R4 Phenyl oder Fluoralkyl;
   R5 H oder Alkyl;
   oder ihre pharmazeutisch verträglichen Salze;
   oder
   WO 9640655, WO 96 40656, WO 96 40653, WO 96 40654
14. Verbindungen der Formel 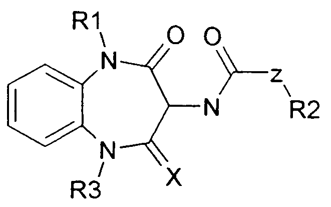 worin bedeuten:
   X O oder H₂;
   R1 Alkyl, Cycloalkyl, Fluoralkyl, oder Oxo- substituiertes Alkyl;
   Z Alkyl, unsubstituiert oder substituiert;
   oder
   Z Alkenyl, Cycloalkyl, Cycloalkenyl, oder eine Bindung;
   R2 Phenyl, unsubstituiert oder substituiert;
   oder
   R2 Cycloalkyl, unsubstituiert oder substituiert;
   R3 Alkyl, Cycloalkyl, Fluoralkyl oder oxo- substituiertes Alkyl;
   oder ihre pharmazeutisch verträglichen Salze;
   oder
   WO 93 04061
15. Verbindungen der Formel 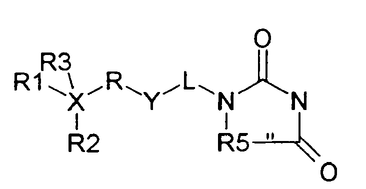 worin bedeuten:
   X ein gesättigter oder ungesättigter 5-, 6- oder 7-gliedriger Heterocyclus oder Carbocyclus;
   R eine Bindung, ein Heteroatom, Carbonyl, ein heterocyclischer Ring, ein carbocyclischer Ring, Alkyl, Alkenyl, Alkoxy, Alkylamino, Arylalkyl, Aryloxy, Acyl, Acyloxy oder Acylamino;
   Y ein substituierter oder unsubstituierter, gesättigter oder ungesättigter 5-, 6-, 7-gliedriger heterocyclischer oder carbocyclischer Ring, oder eine Bindung;
   R1, R2 und R3
   unabhängig von einander nicht vorhanden, Cl, F, Br, NH₂, CF₃, OH, SO₃H, CH₃SO₂NH, COOH, Alkoxy, Alkyl, Alkoxycarbonyl, Hydroxyalkyl, Carboxyalkyl, Aminoalkyl, Acylamino oder Acyloxy.
   L Alkylamino, Alkenylamino, Alkylimino, Alkenylimino oder Acylamino,
   worin der Stickstoff an den Stickstoff in Position 1 der 4-oxocyclischen Harnstoff-Einheit gebunden ist;
   R4 Alkyl, Alkenyl, Alkinyl, Alkylacyl oder Heteroalkyl;
   A substituiertes oder unsubstituiertes, gesättigtes oder ungesättigtes Alkyl oder Heteroalkyl oder eine substituierter oder unsubstituierter 5-, 6-, oder 7-gliedriger Heterocyclus;
   R5 Alkyl;
   oder ihre pharmazeutisch verträglichen Salze;
   oder
   WO9837068
16. Verbindungen der Formel 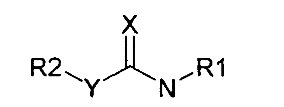 worin bedeuten:
   X O, S, NH, NR, C-CN, N-OR, N-NO₂;
   Y eine Bindung, -C=C- oder NH;
   R1 Alkyl, Alkenyl, Alkinyl, Aryl, Cycloalkyl, ein Heterocyclus oder (Heterocyclo)Alkyl;
   R2 Aryl oder Heterocyclus;
   oder ihre pharmazeutisch verträglichen Salze;
   oder
   WO9514695
17. Verbindungen der Formel 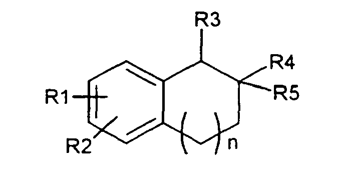 worin bedeuten:
   R1 Halogen, Alkyl, Alkenyl, Alkinyl, Cycloalkyl, Aryl, (Aryl)alkenyl, Alkoxy, 0-Alkenyl, O- Aryl, O-Alkyl(heterocyclo), COO-Alkyl, Alkanoyl, CO-Amino, COsubstituiertes Amino, Alkyl-CO-amino, Alkyl-substituiertes Amino, NHCO-Alkyl, NHCO-Aryl, NHCO- Alkyl(Heterocyclo), N(alkyl)CO- Alkyl, N(alkyl)CO-Aryl, N(Alkyl)CO-Heterocyclo, N- (Alkyl)CO- Alkyl(Heterocyclo);
   R2 Wasserstoff, Alkyl, Halogen, Aryl, Alkoxy, Amino, substituiertes Amino;
   R3 Oxo, Hydroxy, Alkoxy, O-CO- Alkyl, O-CO- Aryl, O-CO-Heterocyclo, NOH, NO- alkyl, N-Amino, N-substituiertes Amino, N-NHCONH- Alkyl, N-NHSO₂-alkyl, N-NHSO₂- Aryl, Amino, substituiertes Amino, NHCO- Alkyl, NHCO-Aryl, NHCO- Heterocyclo, Spiroheterocyclo,;
   R4 Wasserstoff, Alkyl, Alkyl (CO- Alkyl), Alkyl(COO- Alkyl);
   oder
   R3 und R4 zusammen mit dem Atom, an welches sie gebunden sind, ein 5- bis 7-gliedriger Ring, welcher bis zu drei Heteroatome O, N oder S enthalten kann;
   R5 Wasserstoff, Alkyl, Alkenyl, Alkyl (Heterocyclyl), Alkyl-NHCO(Alkyl), Alkyl-NHCO (Aryl), Alkyl-NHCO (alkylheterocyclyl);
   n 0, 1 oder 2;
   oder ihre pharmazeutisch verträglichen Salze.

Bevorzugt verwendet werden Verbindungen, wie sie in folgenden Publikationen beschrieben sind:
1) EP 807 629 (HOE 96/F119)
   Chromane der Formel I 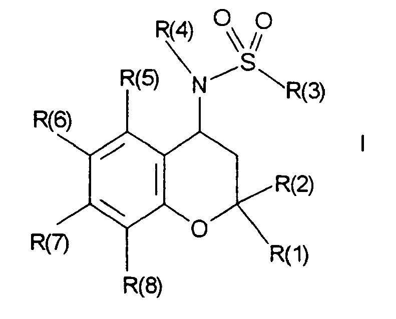 worin bedeuten:
   R(1) und R(2)
   unabhängig voneinander Wasserstoff, CₚF₂ₚ₊₁, Alkyl mit 1, 2, 3, 4, 5 oder 6 C-Atomen oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonylamino und Methylsulfonyl;
   p 1, 2 oder 3;
   oder
   R(1) und R(2)
   gemeinsam eine Alkylenkette mit 2, 3, 4, 5, 6, 7, 8, 9 oder 10 C-Atomen;
   R(3) R(9)-CₙH₂ₙ[NR(11)]ₘ-,
   R(9) Wasserstoff oder Cycloalkyl mit 3, 4, 5, 6, 7 oder 8 C-Atomen;
   n Null, 1, 2, 3, 4, 5, 6, 7, 8, 9 oder 10;
   m Null oder 1;
   R(11) Wasserstoff oder Alkyl mit 1, 2, 3, 4, 5 oder 6 C-Atomen;
   oder
   R(11) gemeinsam mit R(9) eine Alkylengruppe mit 1, 2, 3, 4, 5, 6, 7 oder 8 C-Atomen;
   wobei eine CH₂-Gruppe der Gruppe CₙH₂ₙ durch -O-, -SO_{q} oder -NR(10) ersetzt sein kann;
   q Null, 1 oder 2;
   R(10) Wasserstoff, Methyl oder Ethyl;
   R(4) R(12)-CᵣH₂ᵣ;
   R(12)
   Wasserstoff, Cycloalkyl mit 3, 4, 5, 6, 7 oder 8 C-Atomen, Piperidyl, 1-Pyrrolidinyl, N-Morpholino, N-Methylpiperazino, CₚF₂ₚ₊₁, Pyridyl, Thienyl, Imidazolyl oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
   r 1, 2, 3, 4, 5, 6, 7, 8, 9, 10, 11, 12, 13, 14, 15, 16, 17, 18, 19 oder 20;
   wobei eine CH₂-Gruppe der Gruppe CᵣH₂ᵣ ersetzt sein kann durch -O-,
   -HC=CH-, -C≡C-, -CO-, -CO-O-, -SO_{q}- oder -NR(10)-;
   q Null, 1 oder 2;
   R(10) Wasserstoff, Methyl oder Ethyl;
   R(5), R(6), R(7) und R(8)
   unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, -CN, -CF₃, -C₂F₅, -C₃F₇, -N₃, -NO₂, -CONR(13)R(14), -COOR(15), R(16)-CₛH₂ₛ-Y- oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   R(13) und R(14)
   unabhängig voneinander Wasserstoff oder Alkyl mit 1, 2 oder 3 C-Atomen;
   R(15) Wasserstoff, Methyl, Ethyl, Phenyl oder -CᵤH₂ᵤ-NR(13)R(14);
   u 2 oder 3;
   R(16) Wasserstoff, Cycloalkyl mit 3, 4, 5, 6, 7 oder 8 C-Atomen, -COOR(15), Thienyl, Imidazolyl, Pyridyl, Chinolyl, Isochinolyl, Piperidyl, 1-Pyrrolidinyl, N-Morpholino, N-Methylpiperazino, CₜF₂ₜ₊₁ oder Phenyl,
   das unsubstituiert ist oder substituiert mit 1 oder 2 Substituenten ausgewählt aus der Gruppe bestehend aus F, Cl, Br, 1, CF₃, Methyl, Methoxy, Sulfamoyl oder Methylsulfonyl;
   s Null, 1, 2, 3, 4, 5 oder 6;
   t 1, 2 oder 3;
   Y SO_{q}, -CO-, -SO₂-NR(10)-, -O-, -NR(10)- oder -CO-NR(10);
   wobei jedoch R(6) nicht -OCF₃ oder -OC₂F₅ sein kann;
   und deren physiologisch verträgliche Salze;
   oder
2) EP 847 996 (HOE 96/F341)
   Verbindungen der Formel I, 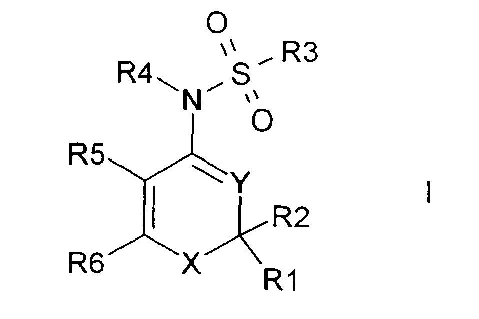 worin bedeuten:
   X -O-, -S-, -SO-, -SO₂-, -NR(7)-, -CR(8a)R(8b)- oder -CO-;
   R(7) Wasserstoff oder -(CₐH₂ₐ)-R(9),
   wobei eine CH₂-Gruppe der Gruppe CₐH₂ₐ ersetzt sein kann durch -O-, - CH=CH-, -C≡C-, -CO-, -CO-O-, -O-CO-, -S-, -SO-, -SO₂-, NR(10)- oder - CONR(10)-;
   R(10) Wasserstoff oder Alkyl mit 1, 2 oder 3 C-Atomen;
   a Null, 1, 2, 3, 4, 5, 6, 7 oder 8;
   R(9) Wasserstoff, CF₃, C₂F₅, C₃F₇, Cycloalkyl mit 3, 4, 5, 6, 7 oder 8 C-Atomen, Dimethylamino, Diethylamino, 1-Piperidyl, 1-Pyrrolidinyl, 4-Morpholinyl, 4-Methylpiperazin-1-yl, Pyridyl, Thienyl, Imidazolyl oder Phenyl,
   wobei Pyridyl, Thienyl, Imidazolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(7) und R(1)
   gemeinsam eine Bindung;
   R(8a) Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(8b) Wasserstoff, Alkyl mit 1, 2 oder 3 C-Atomen, -OR(10), -COOR(10), CO-R(10);
   R(10) Wasserstoff oder Alkyl mit 1, 2 oder 3 C-Atomen;
   oder
   einer der Reste R(8a) oder R(8b)
   gemeinsam mit R(1) eine Bindung, sofern Y die Bedeutung von N besitzt;
   Y N oder CR(11);
   R(11) Wasserstoff oder Alkyl mit 1, 2 oder 3 C-Atomen;
   R(1) und R(2)
   unabhängig voneinander Wasserstoff, CF₃, C₂F₅, C₃F₇, F, Cl, Methoxy, Alkyl mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(1) und R(2)
   gemeinsam eine Alkylenkette mit 2, 3, 4, 5, 6, 7, 8, 9 oder 10 C-Atomen;
   R(3) R(12)-CₙH₂ₙ-NR(13)- oder R(12)-CₙH₂ₙ-,
   wobei eine CH₂-Gruppe in den Gruppen CₙH₂ₙ ersetzt sein kann durch -O-, -CO-, -S-, -SO-, -SO₂- oder -NR(10a)-;
   R(10a)
   Wasserstoff, Methyl oder Ethyl;
   R(12)
   Wasserstoff, Methyl, Cycloalkyl mit 3, 4, 5, 6, 7 oder 8 C-Atomen, CF₃, C₂F₅ oder C₃F₇;
   n Null, 1, 2, 3, 4, 5, 6, 7, 8, 9 oder 10;
   R(13)
   Wasserstoff oder Alkyl mit 1, 2, 3, 4, 5 oder 6 C-Atomen;
   oder
   R(12) und R(13)
   gemeinsam eine Bindung, sofern n nicht kleiner als 3 ist;
   oder
   R(3) und R(4)
   gemeinsam eine Alkylenkette mit 3, 4, 5, 6, 7 oder 8 C-Atomen,
   wobei eine CH₂-Gruppe der Alkylenkette durch -O-, -CO-, -S-, -SO-, -SO₂- oder -NR(10a)- ersetzt sein kann;
   R(10a)
   Wasserstoff, Methyl oder Ethyl;
   R(4) R(14)-CᵣH₂ᵣ,
   wobei eine CH₂-Gruppe der Gruppe CᵣH₂ᵣ ersetzt sein kann durch -O-, - CH=CH-, -C≡C-, -CO-, -CO-O-, -O-CO-, -S-, -SO-, -SO₂-, -NR(10b)-oder -CONR(10b)-;
   R(10b)
   Wasserstoff oder Alkyl mit 1, 2 oder 3 C-Atomen;
   R(14)
   Methyl, CF₃, C₂F₅, C₃F₇, Cycloalkyl mit 3, 4, 5, 6, 7 oder 8 C-Atomen, -OH, -COOH, -NR(23)R(24), 1-Piperidyl, 1-Pyrrolidinyl, 4-Morpholinyl, 4-Methylpiperazin-1-yl, Pyridyl, Thienyl, Imidazolyl oder Phenyl,
   wobei Pyridyl, Thienyl, lmidazolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(23) und R(24)
   unabhängig voneinander Wasserstoff oder Alkyl mit 1, 2 oder 3 C-Atomen;
   oder
   R(23) und R(24)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   r Null, 1, 2, 3, 4, 5, 6, 7, 8, 9, 10, 11, 12, 13, 14, 15, 16, 17, 18, 19 oder 20;
   R(5) und R(6)
   gemeinsam eine Gruppe
   -CR(15)=CR(16)-CR(17)=CR(18)-,
   -CR(15)=CR(16)-CR(17)=N-,
   -CR(15)=CR(16)-N=CR(18)-,
   -CR(15)=N-CR(17)=N-,
   -CR(15)=N-N=CR(18)-,
   -N=CR(16)-CR(17)=N- oder
   -S-CR(15)=CR(16)-,
   wobei jeweils beide Verknüpfungsrichtungen möglich sind;
   R(15), R(16), R(17) und R(18)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Z-CₛH₂ₛ-R(22), Thienyl oder Phenyl, das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Z -O-, -CO-, -CO-O-, -O-CO-, -S-, -SO-, -SO₂-, -SO₂-O-, -SO₂NR(10c), -NR(10c)- oder -CONR(10c)-;
   R(10c) Wasserstoff oder Alkyl mit 1, 2 oder 3 C-Atomen;
   s Null, 1, 2, 3, 4, 5 oder 6;
   R(22) Wasserstoff, CF₃, C₂F₅, C₃F₇, Cycloalkyl mit 3, 4, 5, 6, 7 oder 8 C-Atomen, -NR(19)R(20), -COOR(21), 1-Piperidyl, 1-Pyrrolidinyl, 4-Morpholinyl, 4-Methylpiperazin-1-yl, Pyridyl, Thienyl, Imidazolyl, Chinolyl, Isochinolyl oder Phenyl,
   wobei Pyridyl, Thienyl, Imidazolyl, Chinolyl, Isochinolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(19) und R(20)
   unabhängig voneinander Wasserstoff oder Alkyl mit 1, 2 oder 3 C-Atomen;
   oder
   R(19) und R(20)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(21) Wasserstoff oder Alkyl mit 1, 2 oder 3 C-Atomen;
   wobei jedoch nicht gleichzeitig Y gleich CR(11) und X gleich 0 sein kann;
   sowie ihre physiologisch verträglichen Salze;
   oder
3) EP 857 724 (HOE 97/F 024)
   Verbindungen der Formel I 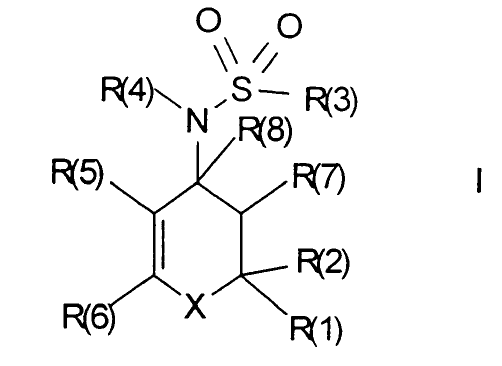 worin bedeuten:
   X -[S(O)_{Null, 1 oder 2}]-, -NR(9)-, -[CR(9)R(23)]- oder -CO-;
   R(9) Wasserstoff oder -(CₙH₂ₙ)-R(10);
   n Null, 1, 2, 3, 4, 5, 6, 7 oder 8;
   R(10) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
   wobei eine CH₂-Gruppe der Gruppe CₙH₂ₙ ersetzt sein kann durch -O-, -CH=CH-, -C≡C-, -CO-, -CO-O-, -[SO_{Null, 1 oder 2}]- oder -NR(11)-;
   R(11) Wasserstoff, Methyl oder Ethyl;
   oder
   R(10) Pyridyl, Thienyl, Imidazolyl oder Phenyl,
   welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
   oder
   R(9) gemeinsam mit R(1) eine Bindung;
   R(23) Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
   R(24) Wasserstoff, Methyl oder Ethyl;
   R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   oder
   R(1) und R(2)
   gemeinsam eine Alkylenkette mit 2, 3, 4, 5, 6, 7, 8, 9 oder 10 C-Atomen;
   R(3) R(12)-CₐH₂ₐ[NR(13)]ₘ-;
   R(12) Wasserstoff oder Cycloalkyl mit 3, 4, 5, 6, 7 oder 8 C-Atomen, CF₃, C₂F₅ oder C₃F₇,
   a Null, 1, 2, 3, 4, 5, 6, 7, 8, 9 oder 10;
   m Null oder 1;
   R(13) Wasserstoff oder Alkyl mit 1, 2, 3, 4, 5 oder 6 C-Atomen;
   oder
   R(12) undR(13)
   gemeinsam eine Alkylengruppe mit 4, 5, 6, 7 oder 8 C-Atomen,
   wobei eine CH₂-Gruppe der Alkylengruppe durch -O-, -[SO_{Null, 1 oder 2}]-, -CO- oder -NR(11)- ersetzt sein kann;
   R(11) Wasserstoff, Methyl oder Ethyl;
   R(4) R(14)-CᵣH₂ᵣ;
   r 1, 2, 3, 4, 5, 6, 7, 8, 9, 10, 11, 12, 13, 14, 15, 16, 17, 18, 19 oder 20;
   R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
   wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
   oder
   R(3) und R(4)
   gemeinsam eine Alkylenkette mit 3, 4, 5, 6, 7 oder 8 C-Atomen,
   wobei eine CH₂-Gruppe der Alkylenkette durch -O-, -[SO_{Null, 1 oder 2}]-, - CO- oder -NR(11)- ersetzt sein kann;
   R(5) und R(6)
   gemeinsam -CR(15)=CR(16)-CR(17)=CR(18)-, -CR(15)=CR(16)-CR(17)=N-, - CR(15)=CR(16)-N=CR(18)-, -CR(15)=N-CR(17)=N-, -CR(15)=N-N=CR(18)-, -N=CR(16)-CR(17)=N- oder -S-CR(15)=CR(16)-; R(15), R(16), R(17) und R(18)
   unabhängig voneinander Wasserstoff, F, Cl, Br, I, Alkyl mit 1, 2, 3 oder
   4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   R(19) und R(20)
   unabhängig voneinander Wasserstoff oder Alkyl mit 1, 2 oder 3 C-Atomen;
   R(21) Wasserstoff, Methyl, Ethyl, Phenyl oder -CᵤH₂ᵤ-NR(19)R(20);
   u 2 oder 3;
   wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
   R(22) Wasserstoff, Cycloalkyl mit 3, 4, 5, 6, 7 oder 8 C-Atomen,
   -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
   s Null, 1, 2, 3, 4, 5 oder 6;
   Z -[S(O)_{Null, 1 oder 2}]-, -CO-, -SO₂-NR(11)-, -SO₂-O-, -O-, -NR(11)- oder -[CO-NR(11)]-;
   R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
   R(8) Wasserstoff oder Alkyl mit 1, 2, 3, 4, 5 oder 6 C-Atomen;
   und deren physiologisch verträgliche Salze;
   oder
4) EP 860 440 (HOE 97/F033)
   Chroman-Derivate der Formel I 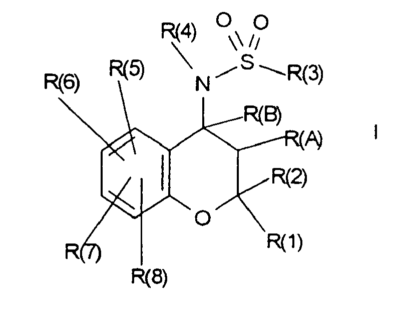 worin bedeuten:
   R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   oder
   R(1) und R(2)
   gemeinsam eine Alkylenkette mit 2, 3, 4, 5, 6, 7, 8, 9 oder 10 C-Atomen;
   R(A) Hydroxy, Alkanoyloxy mit 1, 2, 3, 4, 5 oder 6 C-Atomen oder Alkylsulfonyloxy
   mit 1, 2, 3, 4, 5 oder 6 C-Atomen;
   R(B) Wasserstoff;
   oder
   R(A) und R(B)
   zusammen eine Bindung;
   R(3) R(9)-CₙH₂ₙ[NR(11)]ₘ-;
   R(9) Wasserstoff oder Cycloalkyl mit 3, 4, 5, 6, 7 oder 8 C-Atomen;
   n Null, 1, 2, 3, 4, 5, 6, 7, 8, 9 oder 10;
   m Null oder 1;
   R(11) Wasserstoff oder Alkyl mit 1, 2, 3, 4, 5 oder 6 C-Atomen;
   oder
   R(11) und R(9)
   gemeinsam eine Alkylengruppe mit 1, 2, 3, 4, 5, 6, 7 oder 8 C-Atomen;
   wobei eine CH₂-Gruppe der Gruppe CₙH₂ₙ durch -O-, SO_{Null, 1 oder 2}- oder -NR(10) ersetzt sein kann;
   R(10) Wasserstoff, Methyl oder Ethyl;
   R(4) R(12)-CᵣH₂ᵣ;
   R(12) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
   wobei Pyridyl, Thienyl, Imidazolyl oder Phenyl unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
   r Null, 1, 2, 3, 4, 5, 6, 7, 8, 9, 10, 11, 12, 13, 14, 15, 16, 17, 18, 19 oder 20;
   wobei eine CH₂-Gruppe der Gruppe CᵣH₂ᵣ ersetzt sein kann durch -O-, >CH=CH<, -C=C-, -CO-, -CO-O-, SO_{Null, 1 oder 2}- oder -NR(10)-;
   R(5), R(6), R(7) und R(8)
   unabhängig voneinander Wasserstoff, F, Cl, Br, I, Alkyl mit 1, 2, 3, 4, 5 oder 6 C-Atomen, Cycloalkyl mit 3, 4, 5, 6, 7 oder 8 C-Atomen, -CN, -CF₃, -C₂F₅, -C₃F₇, -N₃, -NO₂, -CONR(13)R(14), -COOR(15), R(16)-CₛH₂ₛ-Y- oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   R(13) und R(14)
   unabhängig voneinander Wasserstoff oder Alkyl mit 1, 2 oder 3 C-Atomen;
   R(15) Wasserstoff, Methyl, Ethyl, Phenyl oder -CᵤH₂ᵤ-NR(13)R(14);
   u 2 oder 3;
   R(16) Wasserstoff, Cycloalkyl mit 3, 4, 5, 6, 7 oder 8 C-Atomen, -COOR(15), Thienyl, Imidazolyl, Pyridyl, Chinolyl, Isochinolyl, Piperidyl, 1-Pyrrolidinyl, N-Morpholino, N-Methylpiperazino, CF₃, C₂F₅, C₃F₇ oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   s Null, 1, 2, 3, 4, 5 oder 6;
   Y -S-, -SO-, -SO₂-, -CO-, -SO₂-NR(10)-, -O-, -NR(10)- oder -CO-NR(10);
   jedoch mit der Bedingung, daß zwei der Substituenten R(5), R(6), R(7) und R(8) nicht Wasserstoff sind;
   und deren physiologisch verträgliche Salze;
   oder
5) EP 861 836 (HOE 97/F038)
   Verbindungen der Formel I, 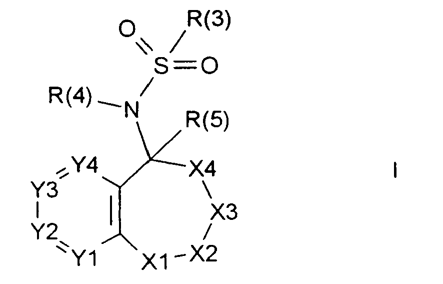 worin bedeuten:
   X1 -O-, -S-, -SO-, -SO₂-, -CR(1)R(2)-, -NR(6)-, -CO- oder -CR(1)R(7)-;
   R(1) und R(2)
   unabhängig voneinander Wasserstoff, CF₃, C₂F₅, C₃F₇, Alkyl
   mit 1, 2, 3, 4, 5 oder 6 C-Atomen oder Phenyl,
   das unsubstituiert ist oder substituiert ist mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(1) und R(2)
   gemeinsam eine Alkylenkette mit 2, 3, 4, 5, 6, 7, 8, 9 oder 10 C-Atomen;
   R(6) Wasserstoff oder -CₙH₂ₙ-R(8),
   wobei eine CH₂-Gruppe der Gruppe CₙH₂ₙ ersetzt sein kann durch -O-, -CH=CH-, -C≡C-, -CO-, -CO-O-, -O-CO-, -S-, -SO-, -SO₂-, -NR(9)- oder -CONR(9)-;
   R(9) Wasserstoff oder Alkyl mit 1, 2 oder 3 C-Atomen;
   n Null, 1, 2, 3, 4, 5, 6, 7 oder 8;
   R(8) Wasserstoff, CF₃, C₂F₅, C₃F₇, Cycloalkyl mit 3, 4, 5, 6, 7 oder 8 C-Atomen, Dimethylamino, Diethylamino, 1-Piperidyl, 1-Pyrrolidinyl, 4-Morpholinyl, 4-Methylpiperazin-1-yl, Pyridyl, Thienyl, Imidazolyl oder Phenyl,
   wobei Pyridyl, Thienyl, Imidazolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   X2 -CR(1)R(2)- oder -CR(2)R(10)-;
   oder
   X2 sofern X3 und X1 -CR(1)R(2)- bedeuten, auch -O-, -S-, -SO-, -SO₂- oder -NR(6)-,
   wobei die Reste R(1), R(2) und R(6)
   wie bei X1 angegeben definiert sind, aber unabhängig von den Bedeutungen der Reste in X1 sind;
   R(10) gemeinsam mit R(7) eine Bindung;
   X3 -CR(1)R(2)-;
   oder
   X3 sofern X2 und X4 -CR(1)R(2)- bedeuten, auch -O-, -S-, -SO-, -SO₂- oder -NR(6)-,
   wobei die Reste R(1), R(2) und R(6)
   wie bei X1 angegeben definiert sind, aber unabhängig von den Bedeutungen der Reste in X1 sind;
   X4 -CR(1)R(2)-, -NR(6)-, -NR(11)-, -CH(OR(30))- oder -CR(2)R(11)-,
   wobei die Reste R(1), R(2) und R(6)
   wie bei X1 angegeben definiert sind, aber unabhängig von den Bedeutungen der Reste in X1 sind;
   R(30) Wasserstoff, Alkyl mit 1, 2 oder 3 C-Atomen oder Acyl mit 1, 2, 3 oder 4 C-Atomen;
   R(11) gemeinsam mit R(5) eine Bindung;
   Y1, Y2, Y3 und Y4
   unabhängig voneinander -CR(12)- oder N,
   wobei maximal 2 der Gruppen Y1, Y2, Y3 und Y4 gleichzeitig N bedeuten können;
   die Reste R(12)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, C₂F₅, C₃F₇, N₃, NO₂, -Z-CₘH₂ₘ-R(13) oder Phenyl,
   das unsubstituiert ist oder substituiert ist mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Z -O-, -CO-, -CO-O-, -O-CO-, -S-, -SO-, -SO₂-, -SO₂NR(14)-, -NR(14)- oder -CONR(14)-;
   R(14) Wasserstoff oder Alkyl mit 1, 2 oder 3 C-Atomen;
   m Null, 1, 2, 3, 4, 5 oder 6;
   R(13) Wasserstoff, CF₃, C₂F₅, C₃F₇, Cycloalkyl mit 3, 4, 5, 6, 7 oder 8 C-Atomen, -NR(15)R(16), -CONR(15)R(16), -OR(30a), Phenyl, Thienyl oder einen N-haltigen Heterocyclus mit 1, 2, 3, 4, 5, 6, 7, 8 oder 9 C-Atomen,
   wobei Phenyl, Thienyl und der N-haltige Heterocyclus unsubstituiert sind oder substituiert sind mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino; R(15) und R(16)
   unabhängig voneinander Wasserstoff oder Alkyl mit 1, 2 oder 3 C-Atomen;
   oder
   R(15) und R(16)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(30a)
   Wasserstoff, Alkyl mit 1, 2 oder 3 C-Atomen oder Acyl mit 1, 2, 3 oder 4 C-Atomen;
   oder
   Y1 und Y2
   gemeinsam ein S-Atom und Y3 und Y4 jeweils -CR(12)-;
   die Reste R(12)
   unabhängig voneinander wie bei Y1, Y2, Y3, Y4 definiert;
   R(3) R(17)-CₓH₂ₓ-NR(18)- oder R(17)-CₓH₂ₓ-,
   wobei eine CH₂-Gruppe in den Gruppen CₓH₂ₓ ersetzt sein kann durch -O-, -CO-, -S-, -SO-, -SO₂- oder -NR(19)-;
   R(19) Wasserstoff, Methyl oder Ethyl;
   R(17) Wasserstoff, Methyl, Cycloalkyl mit 3, 4, 5, 6, 7 oder 8 C-Atomen, CF₃, C₂F₅ oder C₃F₇;
   x Null, 1, 2, 3, 4, 5, 6, 7, 8, 9 oder 10;
   R(18) Wasserstoff oder Alkyl mit 1, 2, 3, 4, 5, 6, 7 oder 8 C-Atomen;
   oder
   R(18) und R(17)
   gemeinsam eine Bindung, sofern x nicht kleiner als 3 ist;
   oder
   R(3) Phenyl,
   das unsubstituiert ist oder substituiert ist mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(3) gemeinsam mit R(4)
   eine Alkylenkette mit 3, 4, 5, 6, 7, oder 8 C-Atomen,
   wobei eine CH₂-Gruppe der Alkylenkette durch -O-, -CO-, -S-, -SO- oder SO₂- ersetzt sein kann;
   R(4) -CᵣH₂ᵣ-R(20),
   wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(21)- oder -CONR(21)-;
   R(21) Wasserstoff oder Alkyl mit 1, 2 oder 3 C-Atomen;
   r Null, 1, 2, 3, 4, 5, 6, 7, 8, 9, 10, 11, 12, 13, 14, 15, 16, 17, 18, 19 oder 20;
   R(20) Wasserstoff, Methyl, CF₃, C₂F₅, C₃F₇, Cycloalkyl mit 3, 4, 5, 6, 7 oder 8 C-Atomen, -NR(22)R(23), Phenyl, Thienyl oder einen N-haltigen Heterocyclus mit 1, 2, 3, 4, 5, 6, 7, 8 oder 9 C-Atomen,
   wobei Phenyl, Thienyl und der N-haltige Heterocyclus unsubstituiert sind oder substituiert sind mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(22) und R(23)
   unabhängig voneinander Wasserstoff oder Alkyl mit 1, 2 oder 3 C-Atomen;
   oder
   R(22) und R(23)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(5) Wasserstoff oder gemeinsam mit R(11) eine Bindung;
   in allen ihren stereoisomeren Formen und Gemische davon in beliebigen Verhältnissen, sowie ihre physiologisch verträglichen Salze;
   oder
6) HMR 97/L206 - EP 895 994
   Verbindungen der Formel I 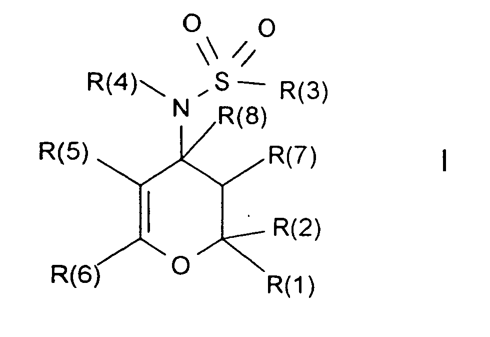 worin bedeuten:
   R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   oder
   R(1) und R(2)
   gemeinsam eine Alkylenkette mit 2, 3, 4, 5, 6, 7, 8, 9 oder 10 C-Atomen;
   wobei eine CH₂-Gruppe der Alkylenkette durch -O-, -CO-, -S-, -SO-, -SO₂- oder -NR(10)- ersetzt sein kann;
   R(10) Wasserstoff oder Alkyl mit 1, 2 oder 3 C-Atomen;
   R(3) R(12)-CₐH₂ₐ[NR(13)]ₘ-;
   R(12) Wasserstoff oder Cycloalkyl mit 3, 4, 5, 6, 7 oder 8 C-Atomen, CF₃, C₂F₅ oder C₃F₇;
   a Null, 1, 2, 3, 4, 5, 6, 7, 8, 9 oder 10;
   m Null oder 1;
   R(13) Wasserstoff oder Alkyl mit 1, 2, 3, 4, 5 oder 6 C-Atomen;
   oder
   R(12) und R(13)
   gemeinsam eine Alkylengruppe mit 4, 5, 6, 7 oder 8 C-Atomen,
   wobei eine CH₂-Gruppe der Alkylengruppe durch -O-, -[SO_{Null, 1 oder 2}]-, -CO- oder -NR(10)- ersetzt sein kann;
   R(10) Wasserstoff oder Alkyl mit 1, 2 oder 3 C-Atomen;
   R(4) R(14)-CᵣH₂ᵣ;
   r Null, 1, 2, 3, 4, 5, 6, 7, 8, 9, 10, 11, 12, 13, 14, 15, 16, 17, 18, 19 oder 20;
   R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
   wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
   R(11) Wasserstoff oder -(CₐH₂ₐ)-R(10);
   wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
   R(10) Wasserstoff oder Alkyl mit 1, 2 oder 3 C-Atomen;
   oder
   R(3) und R(4)
   gemeinsam eine Alkylenkette mit 3, 4, 5, 6, 7 oder 8 C-Atomen,
   wobei eine CH₂-Gruppe der Alkylenkette durch -O-, -[SO_{Null, 1 oder 2}]-, -CO- oder -NR(11)- ersetzt sein kann;
   R(11) Wasserstoff oder -(CₐH₂ₐ)-R(10),
   wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
   R(10) Wasserstoff oder Alkyl mit 1, 2 oder 3 C-Atomen;
   R(5) und R(6)
   -CR(15)=CR(16)-CR(17)=N-,
   -CR(15)=CR(16)-N=CR(17)-,
   -CR(15)=N-CR(17)=N-,
   -CR(15)=N-N=CR(17)-,
   -N=CR(16)-CR(17)=N- oder
   -S-CR(15)=CR(16)-;
   R(15), R(16) und R(17)
   unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(21), -COOR(21), R(22)-CₛH₂ₛ-Z-oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   R(19) Wasserstoff oder Alkyl mit 1, 2 oder 3 C-Atomen;
   R(21) Wasserstoff, Methyl, Ethyl, Phenyl oder -CᵤH₂ᵤ-NR(19)R(20);
   wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl; R(20)
   Wasserstoff oder Alkyl mit 1, 2 oder 3 C-Atomen;
   u 2 oder 3;
   R(22) Wasserstoff, Cycloalkyl mit 3, 4, 5, 6, 7 oder 8 C-Atomen, -COOR(21), CONR(19)R(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅, C₃F₇ oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   s Null, 1, 2, 3, 4, 5 oder 6;
   Z -[S(O)_{Null, 1 oder 2}]-, -CO-, -SO_{(0, 1 oder 2)}-NR(11)-, -SO₂-O-, -O-, -NR(11)- oder -[CO-NR(11)]-;
   R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
   R(8) Wasserstoff oder Alkyl mit 1, 2, 3, 4, 5 oder 6 C-Atomen;
   und deren physiologisch verträgliche Salze;
   oder
7) HMR 97/L223 EP-OS 905 131
   Verbindungen der Formel I, 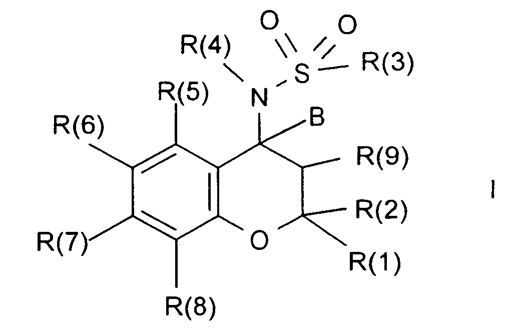 worin bedeuten:
   R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(1) und R(2)
   gemeinsam eine Alkylenkette mit 2, 3, 4, 5, 6, 7, 8, 9 oder 10 C-Atomen;
   R(3) R(10)-CₙH₂ₙ-NR(11)- oder R(10)-CₙH₂ₙ-,
   wobei eine CH₂-Gruppe in den Gruppen CₙH₂ₙ ersetzt sein kann durch -O-, -CO-, -S-, -SO-, -SO₂- oder -NR(12a)-;
   R(12a) Wasserstoff, Methyl oder Ethyl;
   R(10) Wasserstoff, Methyl, Cycloalkyl mit 3, 4, 5, 6, 7 oder 8 C-Atomen, CF₃, C₂F₅ oder C₃F₇;
   n Null, 1, 2, 3, 4, 5, 6, 7, 8, 9 oder 10;
   R(11) Wasserstoff oder Alkyl mit 1, 2, 3, 4, 5 oder 6 C-Atomen; oder
   R(10) und R(11)
   gemeinsam eine Bindung, sofern n nicht kleiner als 3 ist;
   R(4) R(13)-CᵣH₂ᵣ-Z-C_{q}H_{2q}-;
   q 0, 1, 2, 3, 4, 5, 6, 7 oder 8;
   r 0, 1, 2, 3, 4, 5, 6, 7 oder 8;
   Z -CO-NR(14)-,
   -OCO-NR(14)-,
   -O-CₓH₂ₓ-O-,
   -O-CₓH₂ₓ-NR(14)-,
   -O-CₓH₂ₓ-CO-O,
   -CO-O-CₓH₂ₓ-O- oder
   -CO-O-CₓH₂ₓ-NR(14)-,
   wobei jeweils beide Verknüpfungsrichtungen möglich sind;
   x 2, 3 oder 4;
   R(14) Wasserstoff, Alkyl mit 1, 2 oder 3 C-Atomen, -C_{y}H_{2y}-OR(12b),
   -C_{y}H_{2y}-NR(12b)₂;
   R(12b) Wasserstoff, Methyl oder Ethyl;
   y 2 oder 3;
   R(13) H, CF₃, C₂F₅, C₃F₇, Cycloalkyl mit 3, 4, 5, 6, 7 oder 8 C-Atomen, -NR(15)R(16), -CONR(15)R(16), -C(=NR(17))NR(15)R(16), -OR(17), - COOR(17), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl, Aminosulfonyl und Methylsulfonylamino;
   R(15) und R(16)
   unabhängig voneinander Wasserstoff, Alkyl mit 1, 2, 3 oder 4 C-Atomen oder -C_{z}H_{2z}-phenyl,
   worin Phenyl unsubstituiert ist oder substituiert mit 1 oder 2 Substituenten ausgewählt aus der Gruppe bestehend aus F, Cl, Br, CF₃, NO₂, CN OH, Methyl, Ethyl, Methoxy, Dimethylamino, Sulfamoyl, Methylsulfonyl, Aminosulfonyl und Methylsulfonylamino;
   oder
   R(15) und R(16)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(17) Wasserstoff oder Alkyl mit 1, 2 oder 3 C-Atomen;
   R(5), R(6), R(7) und R(8)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18) oder Phenyl,
   das unsubstituiert ist oder substituiert mit 1 oder 2 Substituenten, ausgewählt aus der Gruppe bestehend aus F, Cl, Br, 1, CF₃, NO₂, CN, NH₂, OH, Methyl, Ethyl, Methoxy, Dimethylamino, Sulfamoyl, Methylsulfonyl und Methylsulfonylamino;
   Y -O-, -CO-, -CO-O-, -O-CO-, -S-, -SO-, -SO₂-, -SO₂-O-, -SO₂NR(10c), - NR(10c)- oder -CONR(10c)-;
   R(10c) Wasserstoff oder Alkyl mit 1, 2 oder 3 C-Atomen;
   s Null, 1, 2, 3, 4, 5 oder 6;
   R(18) Wasserstoff, CF₃, C₂F₅, C₃F₇, Cycloalkyl mit 3, 4, 5, 6, 7 oder 8 C-Atomen, -COOR(21), 1-Piperidyl, 1-Pyrrolidinyl, 4-Morpholinyl, 4-Methylpiperazin-1-yl, Pyridyl, Thienyl, Imidazolyl, Chinolyl, Isochinolyl oder Phenyl,
   wobei Pyridyl, Thienyl, Imidazolyl, Chinolyl, Isochinolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(21) Wasserstoff oder Alkyl mit 1, 2 oder 3 C-Atomen;
   R(9) Wasserstoff, OR(10d) oder OCOR(10d);
   R(10d) Wasserstoff oder Alkyl mit 1, 2 oder 3 C-Atomen;
   B Wasserstoff;
   oder
   R(9) und B
   gemeinsam eine Bindung;
   sowie ihre physiologisch verträglichen Salze;
   oder
8) HMR 97/L224 - EP-OS 906 911
   Verbindungen der Formel I, 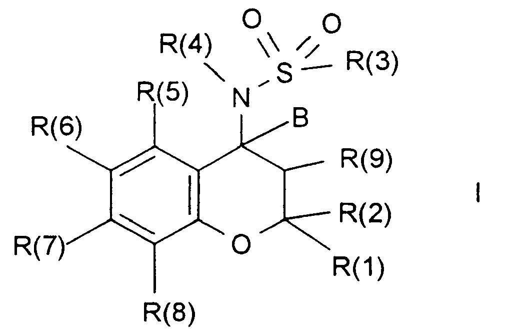 worin bedeuten:
   R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(1) und R(2)
   gemeinsam eine Alkylenkette mit 2, 3, 4, 5, 6, 7, 8, 9 oder 10 C-Atomen;
   R(3) R(10)-CₙH₂ₙ-NR(11)- oder R(10)-CₙH₂ₙ-,
   wobei eine CH₂-Gruppe in den Gruppen CₙH₂ₙ ersetzt sein kann durch -O-, -CO-, -S-, -SO-, -SO₂- oder -NR(12a)-;
   R(12a) Wasserstoff, Methyl oder Ethyl;
   R(10) Wasserstoff, Methyl, Cycloalkyl mit 3, 4, 5, 6, 7 oder 8 C-Atomen, CF₃, C₂F₅ oder C₃F₇;
   n Null, 1, 2, 3, 4, 5, 6, 7, 8, 9 oder 10;
   R(11) Wasserstoff oder Alkyl mit 1, 2, 3, 4, 5 oder 6 C-Atomen;
   oder
   R(10) und R(11)
   gemeinsam eine Bindung, sofern n nicht kleiner als 3 ist;
   oder
   R(3) gemeinsam mit R(4)
   eine Alkylenkette mit 3, 4, 5, 6, 7 oder 8 C-Atomen,
   wobei eine CH₂-Gruppe der Alkylenkette durch -O-, -CO-, -S-, -SO-, -SO₂- oder -NR(12a)- ersetzt sein kann;
   R(12a) Wasserstoff, Methyl oder Ethyl;
   R(4) R(13)-CᵣH₂ᵣ,
   wobei eine CH₂-Gruppe der Gruppe CᵣH₂ᵣ ersetzt sein kann durch -O-, - CH=CH-, -C≡C-, -CO-, -CO-O-, -O-CO-, -S-, -SO-, -SO₂-, -NR(14)- oder -CONR(14)-;
   R(14) Wasserstoff, Alkyl mit 1, 2 oder 3 C-Atomen, -C_{y}H_{2y}-OR(12b), -C_{y}H_{2y}-NR(12b)₂;
   R(12b) Wasserstoff, Methyl oder Ethyl;
   y 2 oder 3;
   R(13) H, CF₃, C₂F₅, C₃F₇, Cycloalkyl mit 3, 4, 5, 6, 7 oder 8 C-Atomen, -NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(15) und R(16)
   unabhängig voneinander Wasserstoff oder Alkyl mit 1, 2, 3 oder 4 C-Atomen;
   oder
   R(15) und R(16)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(17) Wasserstoff, Alkyl mit 1, 2 oder 3 C-Atomen, -CₓH₂ₓOR(12c); R(12c) Wasserstoff, Methyl oder Ethyl;
   x 2 oder 3;
   r Null, 1, 2, 3, 4, 5, 6, 7, 8, 9, 10, 11, 12, 13, 14, 15, 16, 17, 18, 19 oder 20;
   mindestens einer der Substituenten R(5), R(6), R(7) und R(8)
   -Y-CₛH₂ₛ-R(18), Thienyl, Furyl oder ein N-haltiger Heterocyclus mit 1, 2, 3, 4, 5, 6, 7, 8 oder 9 C-Atomen,
   wobei Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Methylamino, Dimethylamino, Ethylamino, Diethylamino, Sulfamoyl, Methylsulfonyl und Methylsulfonylamino;
   Y -O-, -CO-, -O-CO-, -S-, -SO-, -SO₂-, -SO₂-O-, -SO₂NR(12d)-, -NR(12d)- oder -CONR(12d)-,
   wobei die Anknüpfung an den Benzolkern jeweils über das links stehende Atom erfolgt;
   R(12d) Wasserstoff, Methyl oder Ethyl;
   s 1, 2, 3, 4, 5 oder 6;
   R(18) substituiertes Phenyl, das einen oder zwei Substituenten trägt ausgewählt aus der Gruppe bestehend aus NO₂, CN, NH₂, N(Methyl)₂, OH, Ethyl, -COOH, -COOMethyl, -COOEthyl, -CONH₂, -CON(Methyl)₂ ;
   oder
   R(18) ein substituierter N-haltiger Heterocyclus mit 1, 2, 3, 4, 5, 6, 7, 8 oder 9 C-Atomen, der einen oder 2 Substituenten trägt ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(18) -OR(19), -SO₂R(19), -NR(19)R(20), -CONR(19)R(20);
   R(19) und R(20)
   unabhängig voneinander CₜH₂ₜ-R(21);
   t Null, 1, 2, 3, 4, 5 oder 6;
   R(21) Wasserstoff, CF₃, C₂F₅, C₃F₇, Cycloalkyl mit 3, 4, 5, 6, 7
   oder 8 C-Atomen, NR(22)R(23), -OR(24), Phenyl, Thienyl oder ein N-haltiger Heterocyclus mit 1, 2, 3, 4, 5, 6, 7, 8 oder 9 C-Atomen,
   wobei Phenyl, Thienyl und der N-haltige Heterocyclus unsubstituiert sind oder substituiert mit 1 oder 2 Substituenten, ausgewählt aus der Gruppe bestehend aus F, Cl, Br, l, CF₃, NO₂, CN, NH₂, OH, Methyl, Ethyl, Methoxy, Dimethylamino, Sulfamoyl, Methylsulfonyl und Methylsulfonylamino;
   R(22) und R(23)
   unabhängig voneinander Wasserstoff, Alkyl
   mit 1, 2 oder 3 C-Atomen;
   oder
   R(22) und R(23)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(24) Wasserstoff, Alkyl mit 1, 2 oder 3 C-Atomen;
   und die jeweils übrigen Substituenten R(5), R(6), R(7) und R(8), die noch nicht durch die zuvor gegebene Definition vergeben sind,
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂, OR(12e) oder NR(12e)R(12f);
   R(12e) und R(12f)
   unabhängig voneinander Wasserstoff oder Alkyl mit 1, 2, 3 oder 4 C-Atomen;
   R(9) Wasserstoff, OR(12g) oder OCOR(12g);
   R(12g) Wasserstoff oder Alkyl mit 1, 2 oder 3 C-Atomen;
   B Wasserstoff;
   oder
   R(9) und B
   gemeinsam eine Bindung;
   sowie ihre physiologisch verträglichen Salze;
   oder
9) HMR 97/L232 - EP 913 396
   Verbindungen der Formel I, 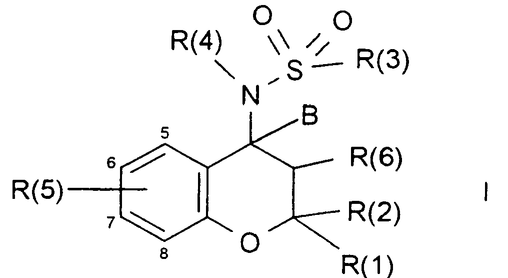 worin R(5) in einer der mit 5, 6, 7 und 8 gekennzeichneten Positionen gebunden ist und worin bedeuten:
   R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   oder
   R(1) und R(2)
   gemeinsam eine Alkylenkette mit 2, 3, 4, 5, 6, 7, 8, 9 oder 10 C-Atomen;
   R(3) R(10)-CₙH₂ₙ-NR(11)- oder R(10)-CₙH₂ₙ-,
   wobei eine CH₂-Gruppe in den Gruppen CₙH₂ₙ ersetzt sein kann durch -O-, -CO-, -S-, -SO-, -SO₂- oder -NR(12a)-;
   R(12a) Wasserstoff, Methyl oder Ethyl;
   R(10) Wasserstoff, Methyl, Cycloalkyl mit 3, 4, 5, 6, 7 oder 8 C-Atomen, CF₃, C₂F₅ oder C₃F₇;
   n Null, 1, 2, 3, 4, 5, 6, 7, 8, 9 oder 10;
   R(11) Wasserstoff oder Alkyl mit 1, 2, 3, 4, 5 oder 6 C-Atomen;
   oder
   R(10) und R(11)
   gemeinsam eine Bindung, sofern n nicht kleiner als 3 ist;
   oder
   R(3) gemeinsam mit R(4)
   eine Alkylenkette mit 3, 4, 5, 6, 7 oder 8 C-Atomen,
   wobei eine CH₂-Gruppe der Alkylenkette durch -O-, -CO-, -S-, -SO-, -SO₂- oder -NR(12a)- ersetzt sein kann;
   R(12a) Wasserstoff, Methyl oder Ethyl;
   R(4) R(13)-CᵣH₂ᵣ,
   wobei eine CH₂-Gruppe der Gruppe CᵣH₂ᵣ ersetzt sein kann durch -O-, - CH=CH-, -C≡C-, -CO-, -CO-O-, -O-CO-, -S-, -SO-, -SO₂- oder -NR(14)-
   R(14) Wasserstoff oder Alkyl mit 1, 2 oder 3 C-Atomen;
   R(13) CH₃, CF₃, C₂F₅, C₃F₇, Cycloalkyl mit 3, 4, 5, 6, 7 oder 8 C-Atomen,
   NR(15)R(16), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, 1, CF₃, NO₂, CN, NH₂, OH, Methyl, Ethyl, Methoxy, Dimethylamino, Sulfamoyl, Methylsulfonyl und Methylsulfonylamino;
   R(15) und R(16)
   gemeinsam eine Kette von 4 oder 5 Methylengruppen, von denen eine CH₂-Gruppe durch -O-, -S-, -NH-, -N(Methyl)- oder-N(Benzyl)- ersetzt sein kann;
   r Null, 1, 2, 3, 4, 5, 6, 7, 8, 9, 10, 11, 12, 13, 14, 15, 16, 17, 18, 19 oder 20;
   R(5) -Y-CₛH₂ₛ-R(18) oder Phenyl,
   das unsubstituiert ist oder substituiert mit 1 oder 2 Substituenten, ausgewählt aus der Gruppe bestehend aus F, Cl, Br, l, CF₃, NO₂, CN, NH₂, OH, Methyl, Ethyl, Methoxy, Dimethylamino, Sulfamoyl, Methylsulfonyl und Methylsulfonylamino;
   Y -O-, -S- oder-NR(10c)-;
   R(10c) Wasserstoff oder Alkyl mit 1, 2 oder 3 C-Atomen;
   s 1, 2, 3, 4, 5, 6, 7 oder 8;
   R(18) Wasserstoff, CF₃, C₂F₅, C₃F₇, Cycloalkyl mit 3, 4, 5, 6, 7 oder 8 C-Atomen, -COOR(21), NR(15a)R(16a), ein unsubstituierter N-haltiger Heterocyclus mit 1, 2, 3, 4, 5, 6, 7, 8 oder 9 C-Atomen, Phenyl oder Thienyl,
   wobei Phenyl und Thienyl unsubstituiert sind oder substituiert mit 1 oder 2 Substituenten, ausgewählt aus der Gruppe bestehend aus F, Cl, Br, I, CF₃, Methyl, Methoxy, Sulfamoyl, Methylsulfonyl und Methylsulfonylamino;
   R(15a) und R(16a)
   gemeinsam eine Kette von 4 oder 5 Methylengruppen, von denen eine CH₂-Gruppe durch -O-, -S-, -NH-, -N(Methyl)- oder - N(Benzyl)- ersetzt sein kann;
   R(21) Wasserstoff oder Alkyl mit 1, 2 oder 3 C-Atomen;
   R(6) OR(10d) oder OCOR(10d);
   R(10d) Wasserstoff oder Alkyl mit 1, 2 oder 3 C-Atomen;
   B Wasserstoff;
   oder
   R(6) und B
   gemeinsam eine Bindung;
   sowie ihre physiologisch verträglichen Salze;
   oder
10. HMR 97/L235 - EP 915 087
   Verbindungen der Formel I, 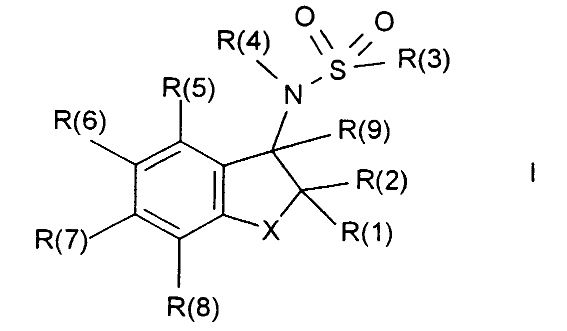 worin bedeuten:
   R(1) und R(2)
   unabhängig voneinander Wasserstoff, CF₃, Alkyl mit 1, 2, 3, 4, 5 oder 6 C-Atomen;
   oder
   R(2) und R(9)
   gemeinsam eine Bindung;
   oder R(2)
   -OR(10a), wenn X -CR(22)R(23)- bedeutet;
   R(10a) Wasserstoff, Acetyl oder Alkyl mit 1, 2 oder 3 C-Atomen;
   R(3) R(10b)-CₙH₂ₙ-NR(11)-oder R(10b)-CₙH₂ₙ-,
   wobei eine CH₂-Gruppe in den Gruppen CₙH₂ₙ ersetzt sein kann durch
   -O-, -CO-, -S-, -SO-, -SO₂- oder -NR(12a)-;
   R(12a) Wasserstoff, Methyl oder Ethyl;
   R(10b) Methyl, Cycloalkyl mit 3, 4, 5, 6, 7 oder 8 C-Atomen, CF₃, C₂F₅ oder C₃F₇;
   n Null, 1, 2, 3, 4, 5, 6, 7, 8, 9 oder 10;
   R(11) Wasserstoff oder Alkyl mit 1, 2, 3, 4, 5 oder 6 C-Atomen;
   oder
   R(10b) und R(11)
   gemeinsam eine Bindung, sofern n größer als 2 ist;
   oder
   R(3) gemeinsam mit R(4)
   eine Alkylenkette mit 3, 4, 5, 6, 7 oder 8 C-Atomen,
   wobei eine CH₂-Gruppe der Alkylenkette durch -O-, -CO-, -S-, -SO-, -SO₂- oder -NR(12a)- ersetzt sein kann;
   R(12a) Wasserstoff, Methyl oder Ethyl;
   R(4) R(13)-CᵣH₂ᵣ,
   wobei eine CH₂-Gruppe der Gruppe CᵣH₂ᵣ ersetzt sein kann durch -O-, - CH=CH-, -C≡C-, -CO-, -CO-O-, -O-CO-, -S-, -SO-, -SO₂-, -NR(14)- oder -CONR(14)-;
   R(14) Wasserstoff, Alkyl mit 1, 2 oder 3 C-Atomen, -C_{y}H_{2y}-OR(12b), -C_{y}H_{2y}-NR(12b)₂;
   R(12b) Wasserstoff, Methyl oder Ethyl;
   y 2 oder 3;
   R(13) CH₃, CF₃, C₂F₅, C₃F₇, Cycloalkyl mit 3, 4, 5, 6, 7 oder 8 C-Atomen, -NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(15) und R(16)
   unabhängig voneinander Wasserstoff oder Alkyl mit 1, 2, 3 oder 4 C-Atomen;
   oder
   R(15) und R(16)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
   R(17) Wasserstoff, Alkyl mit 1, 2 oder 3 C-Atomen, -C_{z}H_{2z}OR(12c); R(12c) Wasserstoff, Methyl oder Ethyl;
   z 2 oder 3;
   r Null, 1, 2, 3, 4, 5, 6, 7, 8, 9, 10, 11, 12, 13, 14, 15, 16, 17, 18, 19 oder 20;
   R(5), R(6), R(7) und R(8)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18), Phenyl, Thienyl, Furyl oder ein N-haltiger Heterocyclus mit 1, 2, 3, 4, 5, 6, 7, 8 oder 9 C-Atomen,
   wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Y -O-, -CO-, -CO-O-, -O-CO-, -S-, -SO-, -SO₂-, -O-SO₂-, -SO₂NR(10c) - oder -CONR(10c)-, wobei die Verknüpfung mit dem Grundgerüst jeweils über das links stehende Atom erfolgt;
   R(10c) Wasserstoff oder Alkyl mit 1, 2 oder 3 C-Atomen;
   s Null, 1, 2, 3, 4, 5 oder 6;
   R(18) Wasserstoff, Methyl, CF₃, C₂F₅, C₃F₇, Cycloalkyl mit 3, 4, 5, 6, 7 oder 8 C-Atomen, -OR(21), -COOR(21), -NR(15a)R(16a), -CONR(15a)R(16a), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, l, CF₃, NO₂, CN, NH₂, OH, Methyl, Ethyl, Methoxy, Dimethylamino, Sulfamoyl, Methylsulfonyl und Methylsulfonylamino;
   R(15a) und R(16a)
   unabhängig voneinander Wasserstoff oder Alkyl mit 1, 2, 3 oder 4 C-Atomen;
   oder
   R(15a) und R(16a)
   gemeinsam eine Kette von 4 oder 5 Methylengruppen, von denen eine CH₂-Gruppe durch -O-, -S-, -NH-, -N(CH₃)- oder
   -N(Benzyl)- ersetzt sein kann;
   R(21) Wasserstoff oder Alkyl mit 1, 2 oder 3 C-Atomen;
   R(9) Wasserstoff oder gemeinsam mit R(2) eine Bindung;
   X -CR(22)R(23)-, -O-, -NR(24)-, -S-, -SO-, -SO₂-;
   R(22) und R(23)
   unabhängig voneinander Wasserstoff, CF₃, Alkyl mit 1, 2, 3, 4, 5 oder 6 C-Atomen;
   R(24) Wasserstoff, Alkyl mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   sowie ihre physiologisch verträglichen Salze.

Sofern nicht anders definiert bedeuten: Alkyl und Alkenyl geradkettiges oder verzweigtes (C₁-C₄)- Alkyl oder - Alkenyl; Alkoxy (C₁-C₄)- Alkoxy; Cycloalkyl (C₃-C₈)-Cycloalkyl; Aryl (C₆-C₁₂)- Aryl; Heteroaryl Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, lsochinolyl, Phthalazinyl, Chinoxalinyl, Chinazolinyl und Cinnolinyl; Acyl (C₁-C₄)-CO oder (C₆-C₁₂)- Aryl-CO oder Heteroaryl-CO; Halo oder Halogen F, Cl, Br; Heterocyclo wie bei Heteroaryl definiert aber auch teilweise oder ganz hydriert.

Die vorliegende Erfindung beschreibt die Anwendung einer neuen Wirkklasse, der lKs-Kanal-Blocker als Chemotherapeutika gegen human- und tierpathogene Endo- und Ektoparasiten. Die Wirkung dieser Blocker basiert auf einem neuartigen anthelminthischem und ektoparasitizidem Prinzip; lKs-Kanal-Blocker blockieren den langsam aktivierten Kaliumkanal und führen damit zum Funktionsverlust des Pharyngalmuskels, der für die Nahrungsaufnahme essentiell ist; dieser Wirkmechanismus führt letztlich zum Hungertod der Parasiten.
IKs-Kanal-Blocker sind wirksam gegen tier- und humanpathogene Trematoden (Fasciola hepatica, Fasciolopsis buski, Fasciola gigantica, Fascioioides magna, Dicrocoelium dendriticum, Opisthorchis felineus, Clonorchis sinensis, Paragonimus westermanni, Paragonimus kellikotti, Schistosoma haematobium, Schistosoma japonicum, Schistosoma mansoni) und tier- und humanpathogene Nematoden, die zur Familie der Trichuridae, Trichinellidae, Strongyloididae, Ancylostomatidae, Strongylidae, Trichostrongylidae, Metastrongylidae, Oesophagostomatidae, Dictyocaulidae, Protostrongylidae, Angiostrongylidae, Oxyuridae, Ascaridae, Toxocaridae, Dracunculidae, Habronematidae und Filariidae zählen; darüber hinaus umfaßt das Wirkungsspektrum der lKs-Kanal-Blocker auch tier- und humanpathogene Ektoparasiten die zur Klasse der Arachnida (Familie: Argasidae, Ixodidae, Dermanyssidae, Demodicidae, Sarcoptidae, Psoroptidae, Varroidae) und zur Klasse der Insecta zählen, die die Ordnungen der Phthiraptera (Anoplura, Mallophaga), Diptera und Siphonaptera umfassen.

Die Erfindung betrifft auch Mittel, insbesondere insektizide und akarizide Mittel, die die Verbindungen der Formel (I) neben geeigneten Formulierungshilfsmitteln enthalten.

Die erfindungsgemäßen Mittel enthalten die Wirkstoffe der Formeln (I) im allgemeinen zu 1 bis 95 Gew.-%.

Sie können auf verschiedene Art formuliert werden, je nachdem wie es durch die biologischen und/oder chemisch-physikalischen Parameter vorgegeben ist.

Als Formulierungsmöglichkeiten kommen daher bevorzugt in Frage:

Spritzpulver (WP), emulgierbare Konzentrate (EC), wäßrige Lösungen (SL), Emulsionen, versprühbare Lösungen, Dispersionen auf Öl- oder Wasserbasis (SC), Suspoemulsionen (SE), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und beispielsweise beschrieben in: Watkins, "Handbook of Insecticide Dust Diluents and Garriers", 2nd Ed., Darland Books, Caldwell N.J.; H. v. Olphen, "Introduction to Clay Colloid Chemistry", 2nd Ed., J. Wiley & Sons, N.Y.; Marsden, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1967; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze wie Ca-dodecylbenzol-sulfonat oder nichtionische Emulgatoren wie Fettsäurepolyglykolester, Alkylarylpolyglykolether, Fettalkoholpolyglykolether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration im allgemeinen etwa 10 bis 90 Gew.-% der Rest zu 100 Gew.% besteht aus üblichen Formulierungsbestandteilen. Bei emulgierbaren Konzentraten kann die Wirkstoffkonzentration im allgemeinen etwa 5 bis 80 Gew.-% betragen. Staubförmige Formulierungen enthalten im allgemeinen 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstoff-Formulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen, durch Mikroorganismen hergestellte Stoffe u.a.. Bevorzugte Mischungspartner sind

### 1. aus der Gruppe der Phosphorverbindungen

Acephate, Azamethiphos, Azinphos-ethyl-, Azinphosmethyl, Bromophos, Bromophos-ethyl, Cadusafos (F-67825), Chlorethoxyphos, Chlorfenvinphos, Chlormephos, Chlorpyrifos, Chlorpyrifos-methyl, Demeton, Demeton-S-methyl, Demeton-S-methyl sulphone, Dialifos, Diazinon, Dichlorvos, Dicrotophos, Dimethoate, Disulfoton, EPN, Ethion, Ethoprophos, Etrimfos, Famphur, Fenamiphos, Fenithriothion, Fensulfothion, Fenthion, Fonophos, Formothion, Fosthiazate (ASC-66824), Heptenophos, Isozophos, Isothioate, Isoxathion, Malathion, Methacrifos, Methamidophos, Methidathion, Salithion, Mevinphos, Monocrotophos, Naled, Omethoate, Oxydemeton-methyl, Parathion, Parathionmethyl, Phenthoate, Phorate, Phosalone, Phosfolan, Phosphocarb (BAS-301), Phosmet, Phosphamidon, Phoxim, Pirimiphos, Primiphos-ethyl, Pirimiphosmethyl, Profenofos, Propaphos, Proetamphos, Prothiofos, Pyraclofos, Pyridapenthion, Quinalphos, Sulprofos, Temephos, Terbufos, Tebupirimfos, Tetrachlorvinphos, Thiometon, Triazophos, Trichlorphon, Vamidothion;

### 2. aus der Gruppe der Carbamate

Alanylcarb (OK-135), Aldicarb, 2-sec-Butylphenylmethylcarbamate (BPMC), Carbaryl, Carbofuran, Carbosulfan, Cloethocarb, Benfuracarb, Ethiofencarb, Furathiocarb, HCN-801, Isoprocarb, Methomyl, 5-Methyl-m-cumenylbutyryl(methyl)carbamate, Oxamyl, Pirimicarb, Propoxur, Thiodicarb, Thiofanox, 1-Methylthio(ethylideneaminol-N-methyl-N-(morpholinothio)carbamate (UC 51717), Triazamate;

### 3. aus der Gruppe der Carbonsäureester

Acrinathrin, Allethrin, Alphametrin, Beta-Cypermethrin, 5-Benzyl-3-furylmethyl-(E)-, (1R)-cis-2,2-di-methyl-3-(2-oxothiolan-3-ylidenemethyl)cyclopropanecarboxylate, Beta-Cyfluthrin, Beta-Cypermethrin, Bioallethrin, Bioallethrin(S)-cyclopentylisomer), Bioresmethrin, Biphenate, (RS)-1-Cyano-1-(6-phenoxy-2-pyridyl)methyl-(1RS)-trans-3-(4-tert.-butylphenyl)-2,2-dimethylcyclopropanecarboxylate (NCI 85193), Cycloprothrin, Cyfluthrin, Cyhalothrin, Cythithrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-Isomer), lmiprothrin (S-41311), Lambda-Cyhalothrin, Permethrin, Pheothrin ((R-Isomer), Prallethrin, Pyrethrine (natürliche Produkte), Resmethrin, Tefluthrin, Tetramethrin, Theta-Cypermethrin (TD-2344), Tralomethrin, Transfluthrin, Zeta-Cypermethrin (F-56701);

### 4. aus der Gruppe der Amidine

Amitraz, Chlordimeform;

### 5. aus der Gruppe der Zinnverbindungen

Cyhexatin, Fenbutatinoxide;

### 6. Sonstige

Abamectin, ABG-9008; Acetamipirid, Anagrapha falcitera, AKD-1022, AKD-3059, ANS-1 18, Bacillus thuringiensis, Beauveria bassianea, Bensultap, Bifenazate (D-2341), Binapacryl, BJL-932, Bromopropylate, BTG-504, BTG-505, Buprofezin, Camphechlor, Cartap, Chlorobenzilate, Chlorfenapyr, Chlorfluazuron, 2-(4-Chlorphenyl)-4,5-diphenylthiophen (UBI-T 930), Chlorfentezine, Chromafenozide (ANS-118), CG-216, CG-217, CG-234, A-184699, Cyclopropancarbonsäure-(2-naphthylmethyl)ester (Ro12-0470), Cyromazin, Diacloden (Thiamethoxam), Diafenthiuron, N-(3,5-Dichlor-4-(1,1,2,3,3,3-hexafluor-1-propyloxy)phenyl)carbamoyl)-2-chlorbenzcarboximidsäureethylester, DDT, Dicofol, Difluobenzuron, N-(2,3-Dihydro-3-methyl-1,3-thiazol-2-ylidene)-2,4-ylidene)-2,4-xylidene, Dinobuton, Dinocap, Diofenolan, DPX-062, Emamectin-Benzoate (MK-244), Endosulfan, Ethiprole (Sulfethiprole), Ethofenprox, Etoxazole (YI-5301), Fenazaquin, Fenoxycarb, Fipronil, Flumite (Flufenzine, SZI-121), 2-Fluoro-5-(4-(4-ethoxyphenyl)-4-methyl-1-pentyl)diphenylether (MTI 800), Granulose- und Kernpolyederviren, Fenpyroximate, Fenthiocarb, Flubenzimine, Flucycloxuron, Flufenoxuron, Flufenprox (ICI-A5683), Fluproxyfen, Gamma-HCH, Halofenozide (RH-0345), Halofenprox (MTI-732), Hexaflumuron (DE_473), Hexythiazox, HOI-9004, Hydramethylnon (AC 217300), Lufenuron, Imidacloprid, Indoxacarb (DPX-MP062), Kanemite (AKD-2023), M-020, MTI-446, Ivermectin, M-020, Methoxyfenozide (Intrepid, RH-2485), Milbemectin, NC-196, Neemgard, Nitenpyram (TI-304), 2-Nitromethyl-4,5-dihydro-6H-thiazin (DS 52618), 2-Nitromethyl-3,4-dihydrothiazol (SD 35651), 2-Nitromethylene-1,2-thiazinan-3-ylcarbamaldehyde (WL 108477), Pyriproxyfen (S-71639), NC-196, NC-111, NNI-9768, Novaluron (MCW-275), OK-9701, OK-9601, OK-9602, Propargite, Pymethrozine, Pyridaben, Pyrimidifen (SU-8801), RH-0345, RH-2485, RYI-210, S-1283, S-1833, SB7242, SI-8601, Silafluofen, Silamadine (CG-177), Spinosad, SU-9118, Tebufenozide, Tebufenpyrad (MK-239), Teflubenzuron, Tefuranitozine MTI-446), Tetradifon, Tetrasul, Thiacloprid, Thiocyclam, TI-435, Tolfenpyrad (OMI-88), Triazamate (RH-7988), Trifumuron, Verbutin, Vertalec, (Mykotal), YI-5301.

Der Wirkstoffgehalt der aus den handelsüblichen Fomulierungen bereiteten Anwendungsformen kann von 0,00000001 bis zu 95 Gew.-% Wirkstoff, vorzugsweise zwischen 0,00001 und 1 Gew.-%, liegen.

Die Anwendung geschieht in einer den Anwendungsformen angepaßten üblichen Weise.

Die erfindungsgemäßen Wirkstoffe der Formel (I) eignen sich auch zur Bekämpfung von Endo- und Ektoparasiten auf dem veterinärmedizinischen Gebiet bzw. auf dem Gebiet der Tierhaltung.

Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion.

Die erfindungsgemäß verwendeten Verbindungen können demgemäß auch besonders vorteilhaft in der Viehhaltung (z. B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die neuen Verbindungen können bei Rindern z.B. in Dosierungen von 0,01 bis 100 mg/kg Körpergewicht eingesetzt werden.

Die erfindungsgemäß verwendeten Verbindungen zeichnen sich auch durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt verschiedene wirtschaftlich bedeutende, phytopathogener Pilze, wie Plasmopara viticola, Phytophthora infestans, Erysiphe graminis, Piricularia oryzae, Pyrenophora teres, Leptosphaerea nodorum und Pellikularia sasakii und Puccinia recondita.

Die erfindungsgemäß verwendeten Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formel I kombiniert werden können, sind z.B. folgende Produkte zu nennen: Aldimorph, Andoprim, Anilazine, Azoxystrobin, Azaconazole, BAS 450F, Benalaxyl, Benodanil, Benomyl, Bethoxazin, Binapacryl, Bion (CGA-245704), Bitertanol, Bromuconazole, Buthiobate, Captafol, Captan, Carbendazim, Carboxin, Carpropamide, CGA 173506, Cymoxanil, Cyproconazole, Cyprodinil, Cyprofuram, Diflumetorim, Dichlorfluanid, Dichlormezin, Diclobutrazol, Diclocymet (S-2900), Diclomezine, Diethofencarb, Difenconazol (CGA 169374), Difluconazole, Dimethirimol, Dimethomorph, Diniconazole, Dinocap, Dithianon, Dodemorph, Dodine, Edifenfos, Epoxidconazole, Ethirimol, Etridiazol, Famoxadone, (DPX-JE874), Fenarimol, Fenbuconazole, Fenfuram, Fenhexamid, Fenpiclonil, Fenpropidin, Fenpromorph, Fentinacetate, Fentihydroxide, Ferimzone (TF 164), Fluazinam, Fluobenzimine, Fludioxonil, Flumetover (RPA-403397), Fluquinconazole, Fluorimide, Flusilazole, Flusulfamide, Flutolanil, Flutriafol, Folpet, Fosetylaluminium, Fuberidazole, Furalaxyl, Furconazol, Furametpyr (S-82658), Furmecyclox, Guazatine, Hexaconazole, Imazalil, Imibenconazole, Ipconazole, lprobenfos, lprodione, Isoprothiolane, KNF 317, Kresoxim-methyl (BAS-490F), Kupferverbindungen wie Cu-oxychlorid, Oxine-Cu, Cu-oxide, Mancozeb, Maneb, Mepapanipyrim (KIFD 3535), Mepronil, Metalaxyl, Metalaxyl-M (CGA-329351), Metconazole, Methasulfocarb, Methfuroxam, Metominofen (SSF-126), Metominostrobin (Fenominostrobin, SSF-126), MON 24000, MON-6550, MON-41100, Myclobutanil, Nabam, Nitrothalidopropyl, Nuarimol, Ofurace, OK-9601, OK-9603, Oxadixyl, Oxycarboxin, Penconazol, Pencycuron, PP 969, Polyoxins, Probenazole, Propineb, Prochloraz, Procymidon, Propamocarb, Propiconazol, Prothiocarb, Pyracarbolid, Pyrazophos, Pyrifenox, Pyrimethanil, Pyroquilon, Quinoxyfen (DE-795), Rabenzazole, RH-7592, RH-7281, Schwefel, Spiroxamine, SSF-109, Tebuconazole, Tetraconazole, TTF 167, Thiabendazole, Thicyofen, Thifluzamide (RH-130753), Thiofanatemethyl, Thiram, TM-402, Tolclofos-methyl, Tolylfluanid, Triadimefon, Triadimenol, Triazoxide, Trichoderma harzianum (DHF-471), Tricyclazole, Tridemorph, Triflumizol, Triforine, Triflumizole (UCC-A815), Triticonazole, Validamycin, Vinchlozolin, XRD 563, Zineb, Natriumdodecylsulfonate, Natrium-dodecyl-sulfat, Natrium-C13/C15-alkohol-ethersulfonat, Natrium-cetostearyl-phosphatester, Dioctyl-natrium-sulfosuccinat, Natrium-isopropyl-napthalenesulfonat, Natriummethylenebisnaphthalene-sulfonat, Cetyl-trimethyl-ammoniumchlorid, Salze von langkettigen primären, sekundären oder tertiären Aminen, Alkyl-propyleneamine, Lauryl-pyrimidiniumbromid, ethoxylierte quarternierte Fettamine, Alkyl-dimethylbenzyl-ammoniumchlorid und 1-Hydroxyethyl-2-alkyl-imidazolin.

Die oben genannten Kombinationspartner stellen bekannte Wirkstoffe dar, die zum großen Teil in The Pesticide Manual (Editor: Clive Tomlin), 11. Auflage (1997), Crop Protection Publications / ISBN 1-901396-11-8 795 beschrieben sind.

Der Wirkstoffgehalt der aus den handelsüblichen Formulierungen bereiteten Anwendungsformen kann in weiten Bereichen variieren, die Wirkstoffkonzentration der Anwendungsformen kann von 0,0001 bis zu 95 Gew.-% Wirkstoff, vorzugsweise zwischen 1 und 50 Gew.-% liegen. Die Anwendung geschieht in einer den Anwendungsformen angepaßten üblichen Weise.

### A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.
d) Ein emulgierbares Konzentrat läßt sich herstellen aus 15 Gew.-Teilen Wirkstoff, 75 Gew.-Teilen Cyclohexan als Lösungsmittel und 10 Gew.-Teilen oxethyliertem Nonylphenol (10 EO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

Zur Prüfung der Wirkung von I_{KS}-Kanalblockern auf den KQT1-Kanal wurden elektrophysiologische Studien an Xenopus- Oozyten durchgeführt, die den KQT1-Kanal (spezifischer I_{KS}- Kanal des Nematoden Caenorhabditis elegans) exprimierten. Ein adultes Froschweibchen (Xenopus laevis) wurde lege artis getötet und aus den Ovarialsäcken Oozyten aller Reifestadien entnommen. Oozyten des Reifestadiums V und VI wurden aussortiert; mit Hilfe eines Nanoliterinjektors, der in einem Mikromanipulator eingespannt war, wurde in jede Oozyte 10 ng der KQT1-spezifischen m- RNA injiziert. Nach 1 - 4 Tagen wurden ausreichend KQT1-Kanäle exprimiert, so daß die entsprechenden Ströme gemessen werden konnten. Zur Messung der langsam aktivierten Kaliumströme bei konstanten intrazellulären Bedingungen wurde die Zwei- Mikroelektroden- Spannungs- Klemmen- Methode (Voltage Clamp- Methode) verwendet. Sie basiert darauf, daß mit 2 intrazellulären Mikroelektroden gearbeitet wird; die erste Elektrode mißt das Membranpotenial gegen eine Referenzelektrode die zweite Elektrode dient dazu, Strom zu injizieren, der nötig ist, um ein gewünschtes Potential zu halten. Während der Messung wurde ein Haltepotential von -80 mV eingestellt, welches dem Ruhemembranpotential entspricht.

Der elektrophysiologische Meßstand umfaßte einen schwingungsgedämpften Experimentiertisch, auf dem eine speziell angefertigte Meßkammer, 2 Mikromanipulatoren, jeweils mit Vorverstärker und Einstechmikroelektrodenhalter sowie ein Stereomikroskop fixiert waren.

Der komplette Meßstand wurde von einem Faraday- Käfig umgeben, um elektrische Störungen während der Messung zu minimieren. Die Oozyte wurde während der Messung von einer ND96- Pufferlösung umspült. Zur Aktivierung des spannungsabhängigen I_{KS}- Kanals wurde die Oozytenmembran durch einen Spannungssprung vom Haltepotential (-80 mV) auf 0 mV depolarisiert und dieser Zustand für 10 Sekunden aufrechterhalten, ehe das Haltepotential wieder eingenommen wurde. Der zeitliche Ablauf des Meßprotokolls wiederholte sich alle 45s. Während der Depolarisationsphase wurde der langsam aktivierte Kaliumstrom gemessen. Der kanalblockierende Effekt verschiedener I_{KS}- Kanalblocker wurde in Konzentrationen von 100, 10, 1 und 0.1 µM getestet; die gemessene Stromamplitude ohne Präparateinfluß verglichen mit der Stromamplitude unter Präparateinfluß diente als Maß für die Aktivität eines I_{KS}- Kanalblockers und wurde in Prozent Hemmung bzw. IC₅₀ ausgedrückt. Abhängig von der Struktur der I_{KS}-Kanalblocker wurden bei 10 µM- Konzentrationen Hemmwerte von 90% bzw. IC50-Werte von <2 µM ermittelt.

Mit Hilfe der Genfunktionsanalytik, die durch zielgerichtete Mutation zur Inaktivierung des KQT1- Gens führte, konnte die physiologische Rolle des I_{KS}- Kanals während des pharyngalen Pumpmechanismus geklärt werden. Nach Herstellung KQT1-negativer Mutanten von C. elegans wurde beobachtet, daß diese nicht lebensfähig waren und verhungerten.

Zur Prüfung der Wirkung der Iₖₛ-Kanalblocker an Helminthen wurde ein in vitro-Test (Larvenentwicklungstest) mit Larven des Hühnerspulwurms Ascaridia galli durchgeführt. Embryonierte Eier von A. galli wurden durch Behandlung mit 5% iger Natriumhypochloritlösung oberflächensterilisiert und nach Entfernung dieser Lösung durch rotierende Glasperlen (5 mm) mechanisch geöffnet. Die geschlüpften L2-Larven wurden via Larvenanreicherungsverfahren angereichert, in einem speziellen Nährmedium (KW-2 Medium) aufgenommen und bei 41 °C und 10% CO₂ inkubiert. Die Larven wurden 48 Stunden nach dem Schlupf in Mikrotiterplatten (96 well) 5 Tage mit medikiertem Medium (200µl) in den Konzentrationen (200, 100, 50 ..... 0.1 µg/ml) inkubiert (41°C,10% CO₂). Während der 5-tägigen Inkubationszeit wurden Motilität, Morphologie und Vitalität täglich mikroskopisch kontrolliert und dokumentiert. Nach Medikation wurde die Larvenkultur 48 Stunden mit Neutralrot in einer Konzentration von 0.16% inkubiert; nach Entfernung des Neutralrots durch Mediumwechsel wurde die Anreicherung des Vitalfarbstoffes im Darm der Larven als Indiz für eine aktive Nahrungsaufnahme und damit als Vitalitätsnachweis bewertet. Die Medikation der Larven mit Iₖₛ-Kanalblockern führte in den Konzentrationen 200, 100, 50 und 25 µg/ml zu 100% Mortalität.

Die Wirkung von Iₖₛ-Kanalblockern gegen Ektoparasiten wurde im Flohlarventest (Katzenfloh = Ctenocephalides felis) überprüft. 5 mg Substanz wurden in 0.5 ml Aceton gelöst und in 500 mg Blutmehl eingemischt. Nachdem das Lösungsmittel verdampft war, wurden jeweils 2 g Quarzsand mit medikiertem Blutmehl so vermischt, daß Präparatkonzentrationen von 1000, 500, 250, 100 ppm resultierten. In das Gemisch aus medikiertem Blutmehl und Quarzsand wurden je medikierter Probe bzw. Lösungsmittelkontrolle 15 Floheier zugegeben, die anschließend bei 37°C und hoher Luftfeuchte inkubiert wurden. Die larvale Entwicklung, Verpuppung und Entwicklung zum adulten Stadium wurde im Abstand von 3 - 5 Tagen kontrolliert und die Mortalitätsraten dokumentiert. In Abhängigkeit von der Dosis konnte eine larvicide Wirkung von 100% im Vergleich zur Lösungsmittelkontrolle beobachtet werden.

Grundsätzlich sind bei der Behandlung von Helminthen und Ektoparasiten therapeutische, meta- und prophylaktische Maßnahmen zu unterscheiden; diese verschiedenen Behandlungsmethoden erfordern spezielle, galenische Formulierungen. Solche Formulierungen gewährleisten z. B. die kontinuierliche Gabe von subtherapeutischen bis therapeutischen Dosen von Anthelminthika bzw. von Ektoparasitiziden, die nach ihrem Freisetzungsmodus in "Sustained-" und "Pulse-Release Boli" unterschieden werden. Erstere unterscheidet man weiter in "Slow-Release Boli", das sind solche mit abnehmender Freisetzungsrate und "Continous-Release Boli", das sind solche mit gleichbleibendem Freisetzungsmodus. "Pulse Release Boli" setzen hingegen den gesamten Wirkstoff innerhalb weniger Stunden bis Tage frei. Neben der Release-Technologie, die auch die Mikroverkapselung von Wirkstoffen beinhaltet und die sich zunehmender Beliebtheit in der Veterinärmedizin erfreut, sind sogenannte "spot on" bzw. "pour on" Formulierungen für die äußere Anwendung am Tier üblich; die Verabreichung von Tabletten, Pasten oder Injektionslösungen sowie die Anwendung von Halsbändern, die Medikamente gegen z. B. Ektoparasiten enthalten, ist Stand der Technik.

### Aktivität gegen KQT1 in Xenopus Oozytes

| Struktur | Name | Inhibition bei 10 µM |
|---|---|---|
| 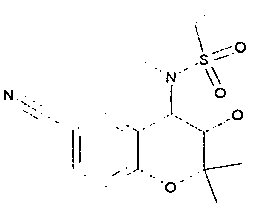 | 6-CYANO-4-TRANS-(N-ETHYLSULFONYL-N-METHYLAMINO)-3-HYDROXY-2,2-DIMETHYLCHROMAN | 40% |
| 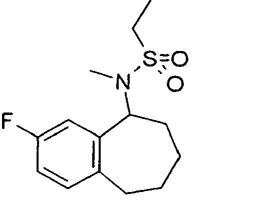 | N-ETHYLSULFONYL-N-METHYLAMINO-8-FLUORBENZOCYCLOHE PTAN | 78% |
| 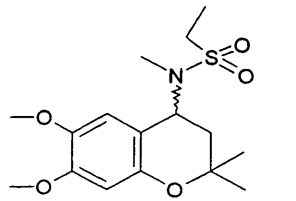 | 4-N-ETHYLSULFONYL-N-METHYLAMINO-6,7 DIMETHOXY-2,2 DIMETHYLCHROMAN | 30% |
| 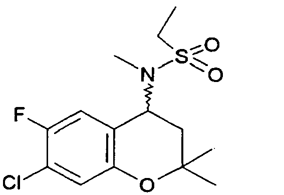 | 7-CHLOR-4-N-ETHYLSULFAMOYL-N-METHYLAMINO-6-FLUOR-2,2 DIMETHYLCHROMAN | 89% |
| 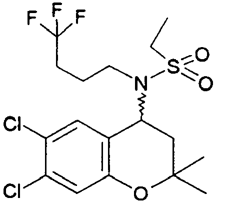 | 6,7-DICHLOR-4-[N-ETHYLSULFONYL-N-(4,4,4-TRIFLUORBUTYL)AMINO] -2,2-DIMETHYLCHROMAN | 88% |
| 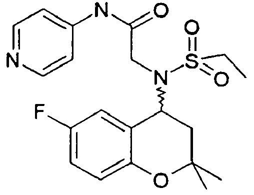 | 2-[ETHANESULFONYL-(6-FLUORO-2,2-DIMETHYL-CHROMAN-4-YL)-AMINO]-N-PYRIDIN-4-YL-ACETAMIDE | 35% |
| 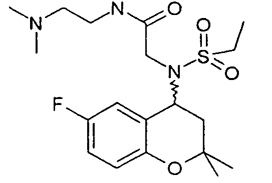 | N-(2-DIMETHYLAMINO-ETHYL)-2-[ETHANESULFONYL-(6-FLUORO-2,2-DIMETHYL-CHROMAN-4-YL)-AMINO]-ACETAMIDE | 22% |
| 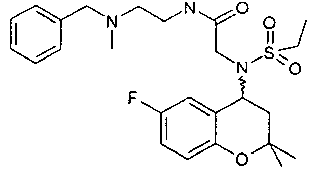 | N-[2-(BENZYL-METHYL-AMINO)-ETHYL]-2-[ETHANESULFONYL-(6-FLUORO-2,2-DIMETHYL-CHROMAN-4- YL)-AMINO]-ACETAMIDE | 36% |
| 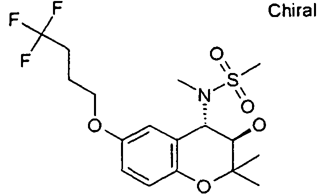 | (3R,4S)-(+)-N-[-3-HYDROXY-2,2-DIMETHYL-6-(4,4,4-TRIFLUORBUTOXY)-CHROMAN-4-YL]-N-METHYL-METHANSULFONAMID | 46% |
| 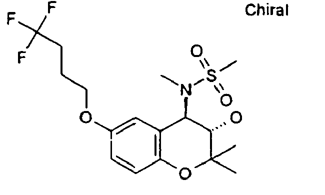 | (3S,4R)-(-)-N-[-3-HYDROXY-2,2-DIMETHYL-6-(4,4,4-TRIFLUORBUTOXY)-CHROMAN-4-YL]-N-METHYL-METHANSULFONAMID | 97% |
| 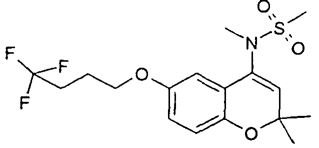 | N-[2,2-Dimethyl-6-(4,4,4-trifluoro-butoxy)-2H-chromen-4-yl]-N-methyl-methanesulfonamide | 92% |

## Patentansprüche

1. Verwendung von Inhibitoren des Iks-Kanals mit gleichzeitiger Wirkung als Inhibitoren des KQT1-Kanals für die Herstellung eines Medikaments zur Behandlung von Krankheiten, die durch Helminthen und Ektoparasiten hervorgerufen werden, **dadurch gekennzeichnet, dass** Verbindungen eingesetzt werden, die in folgenden Publikationen beschrieben werden:
1) EP 807 629 (HOE 96/F119)
Chromane der Formel I 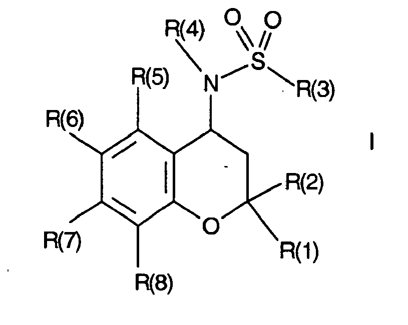 worin bedeuten:
R(1) und R(2)
unabhängig voneinander Wasserstoff, CₚF₂ₚ₊₁, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonylamino und Methylsulfonyl;
p 1, 2 oder 3;
oder
R(1) und R(2)
gemeinsam eine Alkylenkette mit 2, 3, 4, 5, 6, 7, 8, 9 oder 10 C-Atomen;
R(3) R(9)-CₙH₂ₙ[NR(11)]ₘ-;
R(9) Wasserstoff oder Cycloalkyl mit 3, 4, 5, 6, 7 oder 8 C-Atomen;
n Null, 1, 2, 3, 4, 5, 6, 7, 8, 9 oder 10;
m Null oder 1;
R(11) Wasserstoff oder Alkyl mit 1, 2, 3, 4, 5 oder 6 C-Atomen;
oder
R(11) gemeinsam mit R(9) eine Alkylengruppe mit 1, 2, 3, 4, 5, 6, 7 oder 8 C-Atomen;
wobei eine CH₂-Gruppe der Gruppe CₙH₂ₙ durch -O-, -SO_{q} oder -NR(10) ersetzt sein kann;
q Null, 1 oder 2;
R(10) Wasserstoff, Methyl oder Ethyl;
R(4) R(12)-CᵣH₂ᵣ;
R(12)
Wasserstoff, Cycloalkyl mit 3, 4, 5, 6, 7 oder 8 C-Atomen, Piperidyl, 1-Pyrrolidinyl, N-Morpholino, N-Methylpiperazino, CₚF₂ₚ₊₁, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, l, CF₃, Methyl, Methoxy, Sulfamoyl, Methylsulfonyl und Methylsulfonylamino;
r 1, 2, 3, 4, 5, 6, 7, 8, 9, 10, 11, 12, 13, 14, 15, 16, 17, 18, 19 oder 20;
wobei eine CH₂-Gruppe der Gruppe CᵣH₂ᵣ ersetzt sein kann durch -O-,
-HC=CH-, -C≡C-, -CO-, -CO-O-, -SO_{q}- oder -NR(10)-;
q Null, 1 oder 2;
R(10) Wasserstoff, Methyl oder Ethyl;
R(5), R(6), R(7) und R(8)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, -CN, -CF₃, -C₂F₅, -C₃F₇,
-N₃, -NO₂, -CONR(13)R(14), -COOR(15), R(16)-CₛH₂ₛ-Y- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(13) und R(14)
unabhängig voneinander Wasserstoff oder Alkyl mit 1, 2 oder 3 C-Atomen;
R(15) Wasserstoff, Methyl, Ethyl, Phenyl oder -CᵤH₂ᵤ-NR(13)R(14);
u 2 oder 3;
R(16) Wasserstoff, Cycloalkyl mit 3, 4, 5, 6, 7 oder 8 C-Atomen, -COOR(15), Thienyl, Imidazolyl, Pyridyl, Chinolyl, Isochinolyl, Piperidyl, 1-Pyrrolidinyl, N-Morpholino, N-Methylpiperazino, CₜF₂ₜ₊₁ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
s Null, 1, 2, 3, 4, 5 oder 6;
t 1, 2 oder 3;
Y SO_{q}, -CO-, -SO₂-NR(10)-, -O-, -NR(10)- oder -CO-NR(10);
wobei jedoch R(6) nicht -OCF₃ oder -OC₂F₅ sein kann;
und deren physiologisch verträgliche Salze;
oder
2) EP 847 996 (HOE 96/F341)
Verbindungen der Formel I, 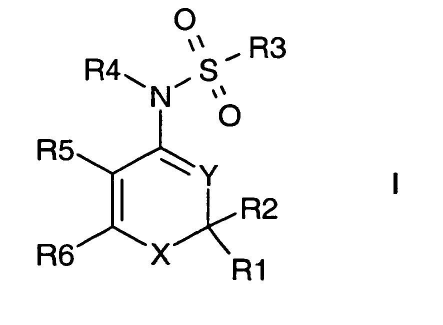 worin bedeuten:
X -O-, -S-, -SO-, -SO₂-, -NR(7)-, -CR(8a)R(8b)- oder -CO-;
R(7) Wasserstoff oder -(CₐH₂ₐ)-R(9),
wobei eine CH₂-Gruppe der Gruppe CₐH₂ₐ ersetzt sein kann durch -O-, - CH=CH-, -C≡C-, -CO-, -CO-O-, -O-CO-, -S-, -SO-, -SO₂-, NR(10)- oder - CONR(10)-;
R(10) Wasserstoff oder Alkyl mit 1, 2 oder 3 C-Atomen;
a Null, 1, 2, 3, 4, 5, 6, 7 oder 8;
R(9) Wasserstoff, CF₃, C₂F₅, C₃F₇, Cycloalkyl mit 3, 4, 5, 6, 7 oder 8 C-Atomen, Dimethylamino, Diethylamino, 1-Piperidyl, 1-Pyrrolidinyl, 4-Morpholinyl, 4-Methylpiperazin-1-yl, Pyridyl, Thienyl, Imidazolyl oder Phenyl,
wobei Pyridyl, Thienyl, Imidazolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(7) und R(1)
gemeinsam eine Bindung;
R(8a) Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(8b) Wasserstoff, Alkyl mit 1, 2 oder 3 C-Atomen, -OR(10), -COOR(10), CO-R(10);
R(10) Wasserstoff oder Alkyl mit 1, 2 oder 3 C-Atomen;
oder
einer der Reste R(8a) oder R(8b)
gemeinsam mit R(1) eine Bindung, sofern Y die Bedeutung von N besitzt;
Y N oder CR(11);
R(11) Wasserstoff oder Alkyl mit 1, 2 oder 3 C-Atomen;
R(1) und R(2)
unabhängig voneinander Wasserstoff, CF₃, C₂F₅, C₃F₇, F, Cl, Methoxy, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(1) und R(2)
gemeinsam eine Alkylenkette mit 2, 3, 4, 5, 6, 7, 8, 9 oder 10 C-Atomen;
R(3) R(12)-CₙH₂ₙ-NR(13)- oder R(12)-CₙH₂ₙ-,
wobei eine CH₂-Gruppe in den Gruppen CₙH₂ₙ ersetzt sein kann durch -O-, - CO-, -S-, -SO-, -SO₂- oder -NR(10a)-;
R(10a)
Wasserstoff, Methyl oder Ethyl;
R(12)
Wasserstoff, Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(13)
Wasserstoff oder Alkyl mit 1, 2, 3, 4, 5 oder 6 C-Atomen;
oder
R(12) und R(13)
gemeinsam eine Bindung, sofern n nicht kleiner als 3 ist;
oder
R(3) und R(4)
gemeinsam eine Alkylenkette mit 3, 4, 5, 6, 7 oder 8 C-Atomen,
wobei eine CH₂-Gruppe der Alkylenkette durch -O-, -CO-, -S-, -SO-, -SO₂- oder -NR(10a)- ersetzt sein kann;
R(10a)
Wasserstoff, Methyl oder Ethyl;
R(4) R(14)-CᵣH₂ᵣ,
wobei eine CH₂-Gruppe der Gruppe CᵣH₂ᵣ ersetzt sein kann durch -O-, - CH=CH-, -C≡C-, -CO-, -CO-O-, -O-CO-, -S-, -SO-, -SO₂-, -NR(10b)- oder - CONR(10b)-;
R(10b)
Wasserstoff oder Alkyl mit 1, 2 oder 3 C-Atomen;
R(14)
Methyl, CF₃, C₂F₅, C₃F₇, Cycloalkyl mit 3, 4, 5, 6, 7 oder 8 C-Atomen, -OH, - COOH, -NR(23)R(24), 1-Piperidyl, 1-Pyrrolidinyl, 4-Morpholinyl, 4-Methylpiperazin-1-yl, Pyridyl, Thienyl, Imidazolyl oder Phenyl,
wobei Pyridyl, Thienyl, Imidazolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(23) und R(24)
unabhängig voneinander Wasserstoff oder Alkyl mit 1, 2 oder 3 C-Atomen;
oder
R(23) und R(24)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
r Null, 1, 2, 3, 4, 5, 6, 7, 8, 9, 10, 11, 12, 13, 14, 15, 16, 17, 18, 19 oder 20;
R(5) und R(6)
gemeinsam eine Gruppe
-CR(15)=CR(16)-CR(17)=CR(18)-,
-CR(15)=CR(16)-CR(17)=N-,
-CR(15)=CR(16)-N=CR(18)-,
-CR(15)=N-CR(17)=N-,
-CR(15)=N-N=CR(18)-,
-N=CR(16)-CR(17)=N- oder
-S-C R(15)=CR(16)-,
wobei jeweils beide Verknüpfungsrichtungen möglich sind;
R(15), R(16), R(17) und R(18)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Z-CₛH₂ₛ-R(22), Thienyl oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Z -O-, -CO-, -CO-O-, -O-CO-, -S-, -SO-, -SO₂-, -SO₂-O-, -SO₂NR(10c), -NR(10c)- oder -CONR(10c)-;
R(10c) Wasserstoff oder Alkyl mit 1, 2 oder 3 C-Atomen;
s Null, 1, 2, 3, 4, 5 oder 6;
R(22) Wasserstoff, CF₃, C₂F₅, C₃F₇, Cycloalkyl mit 3, 4, 5, 6, 7 oder 8 C-Atomen, -NR(19)R(20), -COOR(21), 1-Piperidyl, 1-Pyrrolidinyl, 4-Morpholinyl, 4-Methylpiperazin-1-yl, Pyridyl, Thienyl, Imidazolyl, Chinolyl, Isochinolyl oder Phenyl,
wobei Pyridyl, Thienyl, Imidazolyl, Chinolyl, Isochinolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(19) und R(20)
unabhängig voneinander Wasserstoff oder Alkyl mit 1, 2 oder 3 C-Atomen;
oder
R(19) und R(20)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(21) Wasserstoff oder Alkyl mit 1, 2 oder 3 C-Atomen;
wobei jedoch nicht gleichzeitig Y gleich CR(11) und X gleich O sein kann;
sowie ihre physiologisch verträglichen Salze;
oder
3) EP 857 724 (HOE 97/F 024)
Verbindungen der Formel I 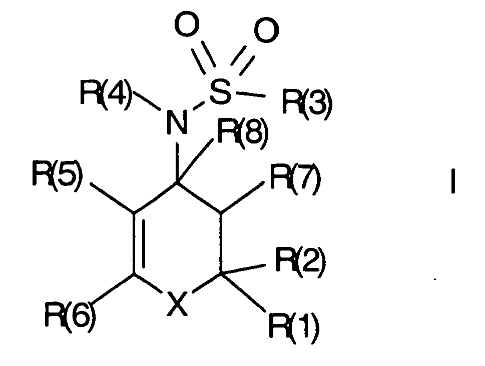 worin bedeuten:
X -[S(O)_{Null, 1 oder 2}]-, -NR(9)-, -[CR(9)R(23)]- oder -CO-;
R(9) Wasserstoff oder -(CₙH₂ₙ)-R(10);
n Null, 1, 2, 3, 4, 5, 6, 7 oder 8;
R(10) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
wobei eine CH₂-Gruppe der Gruppe CₙH₂ₙ ersetzt sein kann durch -O-,
-CH=CH-, -C≡C-, -CO-, -CO-O-, -[SO_{Null, 1 oder 2}]- oder -NR(11)-;
R(11) Wasserstoff, Methyl oder Ethyl;
oder
R(10) Pyridyl, Thienyl, Imidazolyl oder Phenyl,
welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
oder
R(9) gemeinsam mit R(1) eine Bindung;
R(23) Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
R(24) Wasserstoff, Methyl oder Ethyl;
R(1) und R(2)
unabhängig voneinander Wasserstoff, CF₃; C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, 6, 7, 8, 9 oder 10 C-Atomen;
R(3) R(12)-CₐH₂ₐ[NR(13)]ₘ-;
R(12) Wasserstoff oder Cycloalkyl mit 3, 4, 5, 6, 7 oder 8 C-Atomen, CF₃, C₂F₅ oder C₃F₇;
a Null, 1, 2, 3, 4, 5, 6, 7, 8, 9 oder 10;
m Null oder 1;
R(13) Wasserstoff oder Alkyl mit 1, 2, 3, 4, 5 oder 6 C-Atomen;
oder
R(12) und R(13)
gemeinsam eine Alkylengruppe mit 4, 5, 6, 7 oder 8 C-Atomen,
wobei eine CH₂-Gruppe der Alkylengruppe durch -O-, -[SO_{Null, oder 2}]-, -CO- oder -NR(11)- ersetzt sein kann;
R(11) Wasserstoff, Methyl oder Ethyl;
R(4) R(14)-CᵣH₂ᵣ;
r 1, 2, 3, 4, 5, 6, 7, 8, 9, 10, 11, 12, 13, 14, 15, 16, 17, 18, 19 oder 20;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null}, _{1 oder 2}]- oder -NR(11)-;
oder
R(3) und R(4)
gemeinsam eine Alkylenkette mit 3, 4, 5, 6, 7 oder 8 C-Atomen,
wobei eine CH₂-Gruppe der Alkylenkette durch -O-, -[SO_{Null}, _{1 oder2}]-, - CO- oder -NR(11)- ersetzt sein kann;
R(5) und R(6)
gemeinsam -CR(15)=CR(16)-CR(17)=CR(18)-, -CR(15)=CR(16)-CR(17)=N-, CR(15)=CR(16)-N=CR(18)-, -CR(15)=N-CR(17)=N-, -CR(15)=N-N=CR(18)-, -N=CR(16)-CR(17)=N- oder -S-CR(15)=CR(16)-;
R(15), R(16), R(17) und R(18)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(19) und R(20)
unabhängig voneinander Wasserstoff oder Alkyl mit 1, 2 oder 3 C-Atomen;
R(21) Wasserstoff, Methyl, Ethyl, Phenyl oder
-CᵤH₂ᵤ-NR(19)R(20);
u 2 oder 3;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
R(22) Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
s Null, 1, 2, 3, 4, 5 oder 6;
Z -[S(O)_{Null,1 oder 2}]-, -CO-, -SO₂-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
R(8) Wasserstoff oder Alkyl mit 1, 2, 3, 4, 5 oder 6 C-Atomen;
und deren physiologisch verträgliche Salze;
oder
4) EP 860 440 (HOE 97/F033)
Chroman-Derivate der Formel I 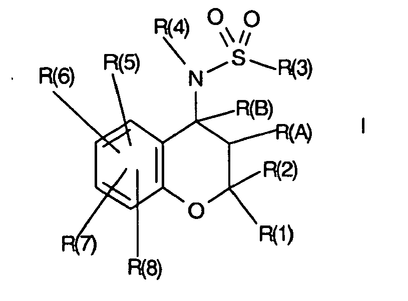 worin bedeuten:
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, 6, 7, 8, 9 oder 10 C-Atomen;
R(A) Hydroxy, Alkanoyloxy mit 1, 2, 3, 4, 5 oder 6 C-Atomen oder Alkylsulfonyloxy mit 1, 2, 3,4, 5 oder 6 C-Atomen;
R(B) Wasserstoff;
oder
R(A) und R(B)
zusammen eine Bindung;
R(3) R(9)-CₙH₂ₙ[NR(11)]ₘ-;
R(9) Wasserstoff oder Cycloalkyl mit 3, 4, 5, 6, 7 oder 8 C-Atomen;
n Null, 1, 2, 3, 4, 5, 6, 7, 8, 9 oder 10;
m Null oder 1;
R(11) Wasserstoff oder Alkyl mit 1, 2, 3, 4, 5 oder 6 C-Atomen;
oder
R(11) und R(9)
gemeinsam eine Alkylengruppe mit 1, 2, 3, 4, 5, 6, 7 oder 8 C-Atomen;
wobei eine CH₂-Gruppe der Gruppe CₙH₂ₙ durch -O-, SO_{Null, 1 oder 2}- oder -NR(10) ersetzt sein kann;
R(10) Wasserstoff, Methyl oder Ethyl;
R(4) R(12)-CᵣH₂ᵣ;
R(12) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
wobei Pyridyl, Thienyl, Imidazolyl oder Phenyl unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
r Null, 1, 2, 3, 4, 5, 6, 7, 8, 9, 10, 11, 12, 13, 14, 15, 16, 17, 18, 19 oder 20;
wobei eine CH₂-Gruppe der Gruppe CᵣH₂ᵣ ersetzt sein kann durch -O-, >CH=CH<, -C≡C-, -CO-, -CO-O-, SO_{Null}, _{1 oder 2}- oder -NR(10)-;
R(5), R(6), R(7) und R(8)
unabhängig voneinander Wasserstoff, F, Cl, Br, I, Alkyl mit 1, 2, 3, 4, 5 oder 6 C-Atomen, Cycloalkyl mit 3, 4, 5, 6, 7 oder 8 C-Atomen, -CN, -CF₃, -C₂F₅, -C₃F₇, -N₃, -NO₂, -CONR(13)R(14), -COOR(15), R(16)-CₛH₂ₛ-Y- oder Phenyl,
das unsubstituiert ist oder substituiert mit 1 oder 2 Substituenten ausgewählt aus der Gruppe bestehend aus F, Cl, Br, 1, CF₃, Methyl, Methoxy, Sulfamoyl und Methylsulfonyl;
R(13) und R(14)
unabhängig voneinander Wasserstoff oder Alkyl mit 1, 2 oder 3 C-Atomen;
R(15) Wasserstoff, Methyl, Ethyl, Phenyl oder -CᵤH₂ᵤNR(13)R(14);
u 2 oder 3;
R(16) Wasserstoff, Cycloalkyl mit 3, 4, 5, 6, 7 oder 8 C-Atomen, -COOR(15), Thienyl, Imidazolyl, Pyridyl, Chinolyl, Isochinolyl, Piperidyl, 1-Pyrrolidinyl, N-Morpholino, N-Methylpiperazino, CF₃, C₂F₅, C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
s Null, 1, 2, 3, 4, 5 oder 6;
Y -S-, -SO-, -SO₂-, -CO-, -SO₂-NR(10)-, -O-, -NR(10)- oder -CO-NR(10);
jedoch mit der Bedingung, daß zwei der Substituenten R(5), R(6), R(7) und R(8) nicht Wasserstoff sind;
und deren physiologisch verträgliche Salze;
oder
5) EP 861 836 (HOE 97/F038)
Verbindungen der Formel I, 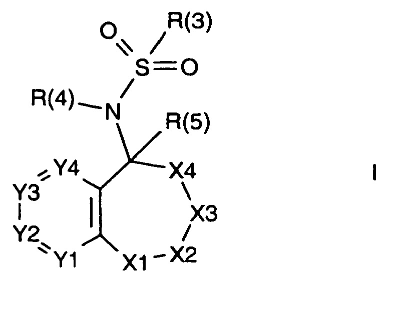 worin bedeuten:
X1 -O-, -S-, -SO-, -SO₂-, -CR(1)R(2)-, -NR(6)-, -CO- oder -CR(1)R(7)-;
R(1) und R(2)
unabhängig voneinander Wasserstoff, CF₃, C₂F₅, C₃F₇, Alkyl
mit 1, 2, 3, 4, 5 oder 6 C-Atomen oder Phenyl,
das unsubstituiert ist oder substituiert ist mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(1) und R(2)
gemeinsam eine Alkylenkette mit 2, 3, 4, 5, 6, 7, 8, 9 oder 10 C-Atomen;
R(6) Wasserstoff oder -CₙH₂ₙ-R(8),
wobei eine CH₂-Gruppe der Gruppe CₙH₂ₙ ersetzt sein kann durch -O-, -CH=CH-, -C=C-, -CO-, -CO-O-, -O-CO-, -S-, -SO-, -SO₂-, -NR(9)- oder -CONR(9)-;
R(9) Wasserstoff oder Alkyl mit 1, 2 oder 3 C-Atomen;
n Null, 1, 2, 3, 4, 5, 6, 7 oder 8;
R(8) Wasserstoff, CF₃, C₂F₅, C₃F₇, Cycloalkyl mit 3, 4, 5, 6, 7 oder 8 C-Atomen, Dimethylamino, Diethylamino, 1-Piperidyl, 1-Pyrrolidinyl, 4-Morpholinyl, 4-Methylpiperazin-1-yl, Pyridyl, Thienyl, Imidazolyl oder Phenyl,
wobei Pyridyl, Thienyl, Imidazolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
X2 -CR(1)R(2)- oder -CR(2)R(10)-;
oder
X2 sofern X3 und X1 -CR(1)R(2)- bedeuten, auch -O-, -S-, -SO-, -SO₂- oder -NR(6)-,
wobei die Reste R(1), R(2) und R(6)
wie bei X1 angegeben definiert sind, aber unabhängig von den Bedeutungen der Reste in X1 sind;
R(10) gemeinsam mit R(7) eine Bindung;
X3 -CR(1)R(2)-;
oder
X3 sofern X2 und X4 -CR(1)R(2)- bedeuten, auch -O-, -S-, -SO-, -SO₂- oder -NR(6)-,
wobei die Reste R(1), R(2) und R(6)
wie bei X1 angegeben definiert sind, aber unabhängig von den Bedeutungen der Reste in X1 sind;
X4 -CR(1)R(2)-, -NR(6)-, -NR(11)-, -CH(OR(30))- oder -CR(2)R(11)-,
wobei die Reste R(1), R(2) und R(6)
wie bei X1 angegeben definiert sind, aber unabhängig von den Bedeutungen der Reste in X1 sind;
R(30) Wasserstoff, Alkyl mit 1, 2 oder 3 C-Atomen oder Acyl mit 1, 2, 3 oder 4 C-Atomen;
R(11) gemeinsam mit R(5) eine Bindung;
Y1, Y2, Y3 und Y4
unabhängig voneinander -CR(12)- oder N,
wobei maximal 2 der Gruppen Y1, Y2, Y3 und Y4 gleichzeitig N bedeuten können;
die Reste R(12)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, C₂F₅, C₃F₇, N₃, NO₂, -Z-CₘH₂ₘ-R(13) oder Phenyl,
das unsubstituiert ist oder substituiert ist mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Z -O-, -CO-, -CO-O-, -O-CO-, -S-, -SO-, -SO₂-, -SO₂NR(14)-, -NR(14)- oder -CONR(14)-;
R(14) Wasserstoff oder Alkyl mit 1, 2 oder 3 C-Atomen;
m Null, 1, 2, 3, 4, 5 oder 6;
R(13) Wasserstoff, CF₃, C₂F₅, C₃F₇, Cycloalkyl mit 3, 4, 5, 6, 7 oder 8 C-Atomen, -NR(15)R(16), -CONR(15)R(16), -OR(30a), Phenyl, Thienyl oder einen N-haltigen Heterocyclus mit 1, 2, 3, 4, 5, 6, 7, 8 oder 9 C-Atomen,
wobei Phenyl, Thienyl und der N-haltige Heterocyclus unsubstituiert sind oder substituiert sind mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
unabhängig voneinander Wasserstoff oder Alkyl mit 1, 2 oder 3 C-Atomen;
oder
R(15) und R(16)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(30a)
Wasserstoff, Alkyl mit 1, 2 oder 3 C-Atomen oder Acyl mit 1, 2, 3 oder 4 C-Atomen;
oder
Y1 und Y2
gemeinsam ein S-Atom und Y3 und Y4 jeweils -CR(12)-;
die Reste R(12)
unabhängig voneinander wie bei Y1, Y2, Y3, Y4 definiert;
R(3) R(17)-CₓH₂ₓ-NR(18)- oder R(17)-CₓH₂ₓ-,
wobei eine CH₂-Gruppe in den Gruppen CₓH₂ₓ ersetzt sein kann durch -O-, -CO-, -S-, -SO-, -SO₂- oder -NR(19)-;
R(19) Wasserstoff, Methyl oder Ethyl;
R(17) Wasserstoff, Methyl, Cycloalkyl mit 3, 4, 5, 6, 7 oder 8 C-Atomen, CF₃, C₂F₅ oder C₃F₇;
x Null, 1, 2, 3, 4, 5, 6, 7, 8, 9 oder 10;
R(18) Wasserstoff oder Alkyl mit 1, 2, 3, 4, 5, 6, 7 oder 8 C-Atomen;
oder
R(18) und R(17)
gemeinsam eine Bindung, sofern x nicht kleiner als 3 ist;
oder
R(3) Phenyl,
das unsubstituiert ist oder substituiert ist mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(3) gemeinsam mit R(4)
eine Alkylenkette mit 3, 4, 5, 6, 7, oder 8 C-Atomen,
wobei eine CH₂-Gruppe der Alkylenkette durch -O-, -CO-, -S-, -SO- oder SO₂- ersetzt sein kann;
R(4) -CᵣH₂ᵣ-R(20),
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(21)- oder -CONR(21)-;
R(21) Wasserstoff oder Alkyl mit 1, 2 oder 3 C-Atomen;
r Null, 1, 2, 3, 4, 5, 6, 7, 8, 9, 10, 11, 12, 13, 14, 15, 16, 17, 18, 19 oder 20;
R(20) Wasserstoff, Methyl, CF₃, C₂F₅, C₃F₇, Cycloalkyl mit 3, 4, 5, 6, 7 oder 8 C-Atomen, -NR(22)R(23), Phenyl, Thienyl oder einen N-haltigen Heterocyclus mit 1, 2, 3, 4, 5, 6, 7, 8 oder 9 C-Atomen,
wobei Phenyl, Thienyl und der N-haltige Heterocyclus unsubstituiert sind oder substituiert sind mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(22) und R(23)
unabhängig voneinander Wasserstoff oder Alkyl mit 1, 2 oder 3 C-Atomen;
oder
R(22) und R(23)
gemeinsam eine Kette von 4 oder 5 Methylengruppen,
von denen eine CH₂-Gruppe durch -O-, -S-, -NH-, -N(CH₃)- oder -N(Benzyl)- ersetzt sein kann;
R(5) Wasserstoff oder gemeinsam mit R(11) eine Bindung;
in allen ihren stereoisomeren Formen und Gemische davon in beliebigen
Verhältnissen, sowie ihre physiologisch verträglichen Salze;
oder
6) HMR 97/L206 - EP 895 994
Verbindungen der Formel I 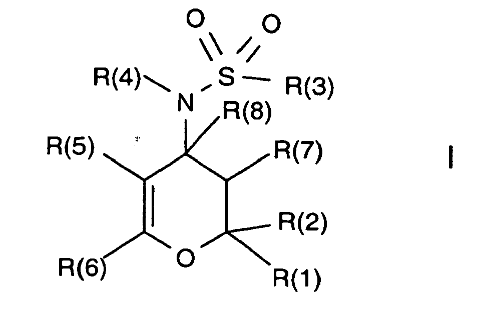 worin bedeuten:
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, 6, 7, 8, 9 oder 10 C-Atomen;
wobei eine CH₂-Gruppe der Alkylenkette durch -O-, -CO-, -S-, -SO-, -SO₂- oder -NR(10)- ersetzt sein kann;
R(10) Wasserstoff oder Alkyl mit 1, 2 oder 3 C-Atomen;
R(3) R(12)-CₐH₂ₐ[NR(13)]ₘ-;
R(12) Wasserstoff oder Cycloalkyl mit 3, 4, 5, 6, 7 oder 8 C-Atomen, CF₃, C₂F₅ oder C₃F₇;
a Null, 1, 2, 3, 4, 5, 6, 7, 8, 9 oder 10;
m Null oder 1;
R(13) Wasserstoff oder Alkyl mit 1, 2, 3, 4, 5 oder 6 C-Atomen;
oder
R(12) und R(13)
gemeinsam eine Alkylengruppe mit 4, 5, 6, 7 oder 8 C-Atomen,
wobei eine CH₂-Gruppe der Alkylengruppe durch -O-, -[SO_{Null, 1 oder 2}]-, -CO- oder -NR(10)- ersetzt sein kann;
R(10) Wasserstoff oder Alkyl mit 1, 2 oder 3 C-Atomen;
R(4) R(14)-CᵣH₂ᵣ;
r Null, 1, 2, 3, 4, 5, 6, 7, 8, 9, 10, 11, 12, 13, 14, 15, 16, 17, 18, 19 oder 20;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, - CH=CH-, -C=C-, -CO-, -CO-O-, -CO-NR(11)-, -[SO_{Null}, _{1 oder 2}]- oder -NR(11)-;
R(11) Wasserstoff oder -(CₐH₂ₐ)-R(10);
wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
R(10) Wasserstoff oder Alkyl mit 1, 2 oder 3 C-Atomen;
oder
R(3) und R(4)
gemeinsam eine Alkylenkette mit 3, 4, 5, 6, 7 oder 8 C-Atomen,
wobei eine CH₂-Gruppe der Alkylenkette durch -O-, -[SO_{Null, 1 oder 2}]-, -CO- oder -NR(11)- ersetzt sein kann;
R(11) Wasserstoff oder -(CₐH₂ₐ)-R(10),
wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
R(10) Wasserstoff oder Alkyl mit 1, 2 oder 3 C-Atomen;
R(5) und R(6)
-CR(15)=CR(16)-CR(17)=N-,
-CR(15)=CR(16)-N=CR(17)-,
-CR(15)=N-CR(17)=N-,
-CR(15)=N-N=CR(17)-,
-N=CR(16)-CR(17)=N- oder
-S-CR(15)=CR(16)-;
R(15), R(16) und R(17)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(21), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(19) Wasserstoff oder Alkyl mit 1, 2 oder 3 C-Atomen;
R(21) Wasserstoff, Methyl, Ethyl, Phenyl oder -CᵤH₂ᵤ-NR(19)R(20);
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(20)
Wasserstoff oder Alkyl mit 1, 2 oder 3 C-Atomen;
u 2 oder 3;
R(22) Wasserstoff, Cycloalkyl mit 3, 4, 5, 6, 7 oder 8 C-Atomen, -COOR(21), CONR(19)R(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅, C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
s Null, 1, 2, 3, 4, 5 oder 6;
Z -[S(O)_{Null, 1 oder 2}]-, -CO-, -SO_{(0, 1 oder 2)}-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
R(8) Wasserstoff oder Alkyl mit 1, 2, 3, 4, 5 oder 6 C-Atomen;
und deren physiologisch verträgliche Salze;
oder
7) HMR 97/L223 EP-OS 905 131
Verbindungen der Formel I, 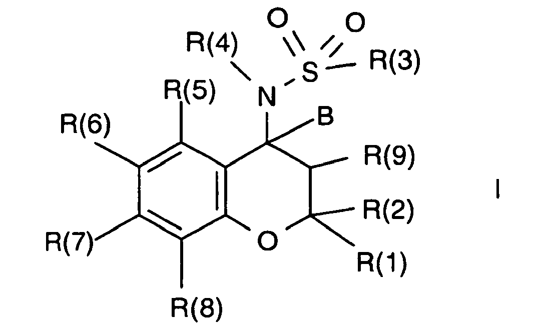 worin bedeuten:
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(1) und R(2)
gemeinsam eine Alkylenkette mit 2, 3, 4, 5, 6, 7, 8, 9 oder 10 C-Atomen;
R(3) R(10)-CₙH₂ₙ-NR(11)- oder R(10)-CₙH₂ₙ-,
wobei eine CH₂-Gruppe in den Gruppen CₙH₂ₙ ersetzt sein kann durch - O-, -CO-, -S-, -SO-, -SO₂- oder -NR(12a)-;
R(12a) Wasserstoff, Methyl oder Ethyl;
R(10) Wasserstoff, Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10) und R(11)
gemeinsam eine Bindung, sofern n nicht kleiner als 3 ist;
R(4) R(13)-CᵣH₂ᵣ-Z-C_{q}H_{2q}-;
q 0, 1, 2, 3, 4, 5, 6, 7 oder 8;
r 0, 1, 2, 3, 4, 5, 6, 7 oder 8;
Z -CO-NR(14)-,
-OCO-NR(14)-,
-O-CₓH₂ₓ-O-,
-O-CₓH₂ₓ-NR(14)-,
-O-CₓH₂ₓ-CO-O,
-CO-O-CₓH₂ₓ-O- oder
-CO-O-CₓH₂ₓ-NR(14)-,
wobei jeweils beide Verknüpfungsrichtungen möglich sind;
x 2, 3 oder 4;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) H, CF₃, C₂F₅, C₃F₇, Cycloalkyl mit 3, 4, 5, 6, 7 oder 8 C-Atomen,
-NR(15)R(16), -CONR(15)R(16), -C(=NR(17))NR(15)R(16), -OR(17), - COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(15) und R(16)
unabhängig voneinander Wasserstoff, Alkyl mit 1, 2, 3 oder 4 C-Atomen oder -C_{z}H_{2z}-phenyl,
worin Phenyl unsubstituiert ist oder substituiert mit 1 oder 2 Substituenten ausgewählt aus der Gruppe bestehend aus F, Cl, Br, CF₃, NO₂, CN OH, Methyl, Ethyl, Methoxy, Dimethylamino, Sulfamoyl, Methylsulfonyl, Aminosulfonyl und Methylsulfonylamino;
oder
R(15) und R(16)
gemeinsam eine Kette von 4 oder 5 Methylengruppen, von denen eine CH₂-Gruppe durch -O-, -S-, -NH-, -N(CH₃)- oder
-N(Benzyl)- ersetzt sein kann;
R(17) Wasserstoff oder Alkyl mit 1, 2 oder 3 C-Atomen;
R(5), R(6), R(7) und R(8)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18) oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-, -SO₂-O-, -SO₂NR(10c), - NR(1 0c)- oder -CONR(10c)-;
R(1 0c) Wasserstoff oder Alkyl mit 1, 2 oder 3 C-Atomen;
s Null, 1, 2, 3, 4, 5 oder 6;
R(18) Wasserstoff, CF₃, C₂F₅, C₃F₇, Cycloalkyl mit 3, 4, 5, 6, 7 oder 8 C-Atomen, -COOR(21), 1-Piperidyl, 1-Pyrrolidinyl, 4-Morpholinyl, 4-Methylpiperazin-1-yl, Pyridyl, Thienyl, Imidazolyl, Chinolyl, Isochinolyl oder Phenyl,
wobei Pyridyl, Thienyl, Imidazolyl, Chinolyl, Isochinolyl und Phenyl unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(21) Wasserstoff oder Alkyl mit 1, 2 oder 3 C-Atomen;
R(9) Wasserstoff, OR(10d) oder OCOR(10d);
R(10d) Wasserstoff oder Alkyl mit 1, 2 oder 3 C-Atomen;
B Wasserstoff;
oder
R(9) und B
gemeinsam eine Bindung;
sowie ihre physiologisch verträglichen Salze;
oder
8) HMR 97/L224 - EP-OS 906 911
Verbindungen der Formel I, 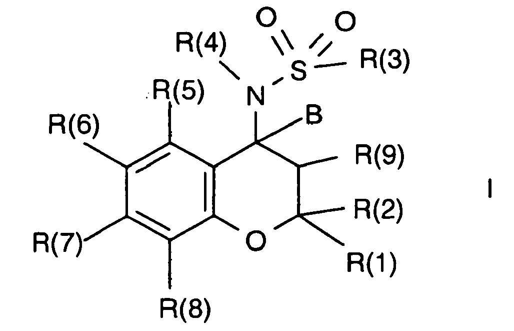 worin bedeuten:
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(1) und R(2)
gemeinsam eine Alkylenkette mit 2, 3, 4, 5, 6, 7, 8, 9 oder 10 C-Atomen;
R(3) R(10)-CₙH₂ₙ-NR(11)- oder R(10)-CₙH₂ₙ-,
wobei eine CH₂-Gruppe in den Gruppen CₙH₂ₙ ersetzt sein kann durch - O-, -CO-, -S-, -SO-, -SO₂- oder -NR(12a)-;
R(12a) Wasserstoff, Methyl oder Ethyl;
R(10) Wasserstoff, Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10) und R(11)
gemeinsam eine Bindung, sofern n nicht kleiner als 3 ist;
oder
R(3) gemeinsam mit R(4)
eine Alkylenkette mit 3, 4, 5, 6, 7 oder 8 C-Atomen,
wobei eine CH₂-Gruppe der Alkylenkette durch -O-, -CO-, -S-, -SO-, -SO₂- oder -NR(12a)- ersetzt sein kann;
R(12a) Wasserstoff, Methyl oder Ethyl;
R(4) R(13)-CᵣH₂ᵣ,
wobei eine CH₂-Gruppe der Gruppe CᵣH₂ᵣ ersetzt sein kann durch -O-, - CH=CH-, -C≡C-, -CO-, -CO-O-, -O-CO-, -S-, -SO-, -SO₂-, -NR(14)- oder -CONR(14)-;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) H, CF₃, C₂F₅, C₃F₇, Cycloalkyl mit 3, 4, 5, 6, 7 oder 8 C-Atomen,
-NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
unabhängig voneinander Wasserstoff oder Alkyl mit 1, 2, 3 oder 4 C-Atomen;
oder
R(15) und R(16)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff, Alkyl mit 1, 2 oder 3 C-Atomen, -CₓH₂ₓOR(12c);
R(12c) Wasserstoff, Methyl oder Ethyl;
x 2 oder 3;
r Null, 1, 2, 3, 4, 5, 6, 7, 8, 9, 10, 11, 12, 13, 14, 15, 16, 17, 18, 19 oder 20;
mindestens einer der Substituenten R(5), R(6), R(7) und R(8)
-Y-CₛH₂ₛ-R(18), Thienyl, Furyl oder ein N-haltiger Heterocyclus mit 1, 2, 3, 4, 5, 6, 7, 8 oder 9 C-Atomen,
wobei Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Methylamino, Dimethylamino, Ethylamino, Diethylamino, Sulfamoyl, Methylsulfonyl und Methylsulfonylamino;
Y -O-, -CO-, -O-CO-, -S-, -SO-, -SO₂-, -SO₂-O-, -SO₂NR(12d)-, -NR(12d)- oder -CONR(12d)-,
wobei die Anknüpfung an den Benzolkern jeweils über das links stehende Atom erfolgt;
R(12d) Wasserstoff, Methyl oder Ethyl;
s 1, 2, 3, 4, 5 oder 6;
R(18) substituiertes Phenyl, das einen oder zwei Substituenten trägt ausgewählt aus der Gruppe bestehend aus NO₂, CN, NH₂, N(Methyl)₂, OH, Ethyl, -COOH, -COOMethyl, -COOEthyl, -CONH₂, -CON(Methyl)₂ ;
oder
R(18) ein substituierter N-haltiger Heterocyclus mit 1, 2, 3, 4, 5, 6, 7, 8 oder 9 C-Atomen, der einen oder 2 Substituenten trägt ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(18) -OR(19), -SO₂R(19), -NR(19)R(20), -CONR(19)R(20);
R(19) und R(20)
unabhängig voneinander CₜH₂ₜ-R(21);
t Null, 1, 2, 3, 4, 5 oder 6;
R(21) Wasserstoff, CF₃, C₂F₅, C₃F₇, Cycloalkyl mit 3, 4, 5, 6, 7 oder 8 C-Atomen, NR(22)R(23), -OR(24), Phenyl, Thienyl oder ein N-haltiger Heterocyclus mit 1, 2, 3, 4, 5, 6, 7, 8 oder 9 C-Atomen,
wobei Phenyl, Thienyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(22) und R(23)
unabhängig voneinander Wasserstoff, Alkyl mit 1, 2 oder 3 C-Atomen;
oder
R(22) und R(23)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(24) Wasserstoff, Alkyl mit 1, 2 oder 3 C-Atomen;
und die jeweils übrigen Substituenten R(5), R(6), R(7) und R(8), die noch nicht durch die zuvor gegebene Definition vergeben sind,
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂, OR(12e) oder NR(12e)R(12f);
R(12e) und R(12f)
unabhängig voneinander Wasserstoff oder Alkyl mit 1, 2, 3 oder 4 C-Atomen;
R(9) Wasserstoff, OR(12g) oder OCOR(12g);
R(12g) Wasserstoff oder Alkyl mit 1, 2 oder 3 C-Atomen;
B Wasserstoff;
oder
R(9) und B
gemeinsam eine Bindung;
sowie ihre physiologisch verträglichen Salze;
oder
9) HMR 97/L232 - EP 913 396
Verbindungen der Formel I, 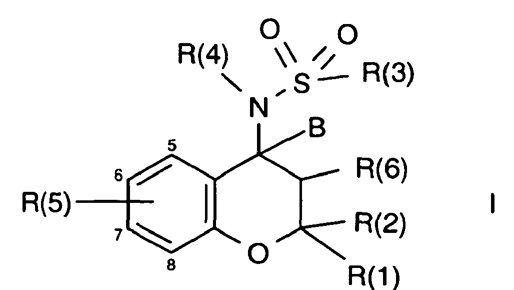 worin R(5) in einer der mit 5, 6, 7 und 8 **gekennzeichnet**en Positionen gebunden ist und worin bedeuten:
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(1) und R(2)
gemeinsam eine Alkylenkette mit 2, 3, 4, 5, 6, 7, 8, 9 oder 10 C-Atomen;
R(3) R(10)-CₙH₂ₙ-NR(11)- oder R(10)-CₙH₂ₙ-,
wobei eine CH₂-Gruppe in den Gruppen CₙH₂ₙ ersetzt sein kann durch - O-, -CO-, -S-, -SO-, -SO₂- oder -NR(12a)-;
R(12a) Wasserstoff, Methyl oder Ethyl;
R(10) Wasserstoff, Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10) und R(11)
gemeinsam eine Bindung, sofern n nicht kleiner als 3 ist;
oder
R(3) gemeinsam mit R(4)
eine Alkylenkette mit 3, 4, 5, 6, 7 oder 8 C-Atomen,
wobei eine CH₂-Gruppe der Alkylenkette durch -O-, -CO-, -S-, -SO-, -SO₂- oder -NR(12a)- ersetzt sein kann;
R(12a) Wasserstoff, Methyl oder Ethyl;
R(4) R(13)-CᵣH₂ᵣ,
wobei eine CH₂-Gruppe der Gruppe CᵣH₂ᵣ ersetzt sein kann durch -O-, - CH=CH-, -C≡C-, -CO-, -CO-O-, -O-CO-, -S-, -SO-, -SO₂- oder -NR(14)-; R(14) Wasserstoff oder Alkyl mit 1, 2 oder 3 C-Atomen;
R(13) CH₃, CF₃, C₂F₅, C₃F₇, Cycloalkyl mit 3, 4, 5, 6, 7 oder 8 C-Atomen, NR(15)R(16), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
gemeinsam eine Kette von 4 oder 5 Methylengruppen, von denen eine CH₂-Gruppe durch -O-, -S-, -NH-, -N(Methyl)- oder - N(Benzyl)- ersetzt sein kann;
r Null, 1, 2, 3, 4, 5, 6, 7, 8, 9, 10, 11, 12, 13, 14, 15, 16, 17, 18, 19 oder 20;
R(5) -Y-CₛH₂ₛ-R(18) oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -S- oder -NR(10c)-;
R(10c) Wasserstoff oder Alkyl mit 1, 2 oder 3 C-Atomen;
s 1, 2, 3, 4, 5, 6, 7 oder 8;
R(18) Wasserstoff, CF₃, C₂F₅, C₃F₇, Cycloalkyl mit 3, 4, 5, 6, 7 oder 8 C-Atomen, -COOR(21), NR(15a)R(16a), ein unsubstituierter N-haltiger Heterocyclus mit 1, 2, 3, 4, 5, 6, 7, 8 oder 9 C-Atomen, Phenyl oder Thienyl,
wobei Phenyl und Thienyl unsubstituiert sind oder substituiert mit 1 oder 2 Substituenten, ausgewählt aus der Gruppe bestehend aus F, Cl, Br, I, CF₃, Methyl, Methoxy, Sulfamoyl, Methylsulfonyl und Methylsulfonylamino;
R(15a) und R(16a)
gemeinsam eine Kette von 4 oder 5 Methylengruppen, von denen eine CH₂-Gruppe durch -O-, -S-, -NH-, -N(Methyl)- oder - N(Benzyl)- ersetzt sein kann;
R(21) Wasserstoff oder Alkyl mit 1, 2 oder 3 C-Atomen;
R(6) OR(10d) oder OCOR(10d);
R(10d) Wasserstoff oder Alkyl mit 1, 2 oder 3 C-Atomen;
B Wasserstoff;
oder
R(6) und B
gemeinsam eine Bindung;
sowie ihre physiologisch verträglichen Salze;
oder
10. HMR 97/L235 - EP 915 087
Verbindungen der Formel I, 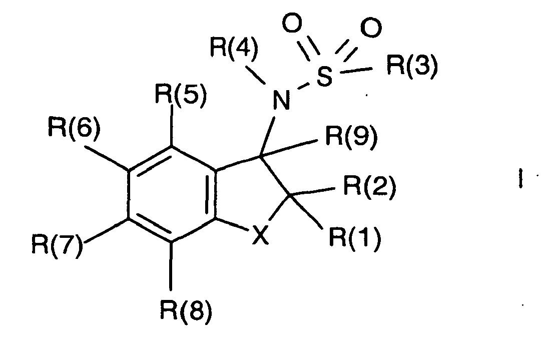 worin bedeuten:
R(1) und R(2)
unabhängig voneinander Wasserstoff, CF₃, Alkyl mit 1, 2, 3, 4, 5 oder 6 C-Atomen;
oder
R(2) und R(9)
gemeinsam eine Bindung;
oder R(2)
-OR(10a), wenn X -CR(22)R(23)- bedeutet;
R(10a) Wasserstoff, Acetyl oder Alkyl mit 1, 2 oder 3 C-Atomen;
R(3) R(10b)-CₙH₂ₙ-NR(11)- oder R(10b)-CₙH₂ₙ-,
wobei eine CH₂-Gruppe in den Gruppen CₙH₂ₙ ersetzt sein kann durch - O-, -CO-, -S-, -SO-, -SO₂- oder -NR(12a)-;
R(12a) Wasserstoff, Methyl oder Ethyl;
R(10b) Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10b) und R(11)
gemeinsam eine Bindung, sofern n größer als 2 ist;
oder
R(3) gemeinsam mit R(4)
eine Alkylenkette mit 3, 4, 5, 6, 7 oder 8 C-Atomen,
wobei eine CH₂-Gruppe der Alkylenkette durch -O-, -CO-, -S-, -SO-, -SO₂- oder -NR(12a)- ersetzt sein kann;
R(12a) Wasserstoff, Methyl oder Ethyl;
R(4) R(13)-CᵣH₂ᵣ,
wobei eine CH₂-Gruppe der Gruppe CᵣH₂ᵣ ersetzt sein kann durch -O-, CH=CH-, -C≡C-, -CO-, -CO-O-, -O-CO-, -S-, -SO-, -SO₂-, -NR(14)- oder -CONR(14)-;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) CH₃, CF₃, C₂F₅, C₃F₇, Cycloalkyl mit 3, 4, 5, 6, 7 oder 8 C-Atomen, -NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
unabhängig voneinander Wasserstoff oder Alkyl mit 1, 2, 3 oder 4 C-Atomen;
oder
R(15) und R(16)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff, Alkyl mit 1, 2 oder 3 C-Atomen, -C_{z}H_{2z}OR(12c); R(12c) Wasserstoff, Methyl oder Ethyl;
z 2 oder 3;
r Null, 1, 2, 3, 4, 5, 6, 7, 8, 9, 10, 11, 12, 13, 14, 15, 16, 17, 18, 19 oder 20;
R(5), R(6), R(7) und R(8)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18), Phenyl, Thienyl, Furyl oder ein N-haltiger Heterocyclus mit 1, 2, 3, 4, 5, 6, 7, 8 oder 9 C-Atomen,
wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-, -O-SO₂-, -SO₂NR(10c) - oder -CONR(10c)-, wobei die Verknüpfung mit dem Grundgerüst jeweils über das links stehende Atom erfolgt;
R(10c) Wasserstoff oder Alkyl mit 1, 2 oder 3 C-Atomen;
s Null, 1, 2, 3, 4, 5 oder 6;
R(18) Wasserstoff, Methyl, CF₃, C₂F₅, C₃F₇, Cycloalkyl mit 3, 4, 5, 6, 7 oder 8 C-Atomen, -OR(21), -COOR(21), -NR(15a)R(16a), -CONR(15a)R(16a), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15a) und R(16a)
unabhängig voneinander Wasserstoff oder Alkyl mit 1, 2, 3 oder 4 C-Atomen;
oder
R(15a) und R(16a)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(21) Wasserstoff oder Alkyl mit 1, 2 oder 3 C-Atomen;
R(9) Wasserstoff oder gemeinsam mit R(2) eine Bindung;
X -CR(22)R(23)-, -O-, -NR(24)-, -S-, -SO-, -SO₂-;
R(22) und R(23)
unabhängig voneinander Wasserstoff, CF₃, Alkyl mit 1, 2, 3, 4, 5 oder 6 C-Atomen;
R(24) Wasserstoff, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
sowie ihre physiologisch verträglichen Salze.

2. Verwendung nach Anspruch 1 von Inhibitoren des Iks-Kanals mit gleichzeitiger Wirkung als lnhibitoren des KQT1-Kanals für die Herstellung eines Medikaments zur Behandlung von Krankheiten, die durch Helminthen hervorgerufen werden.

## Claims

1. The use of inhibitors of the I_{KS} channel with simultaneous action as inhibitors of the KQT1 channel for preparing a medicament for treating diseases caused by helminths and ectoparasites, which comprises using compounds described in the following publications:
1) EP 807 629 (HOE 96/F119)
chromanes of the formula I 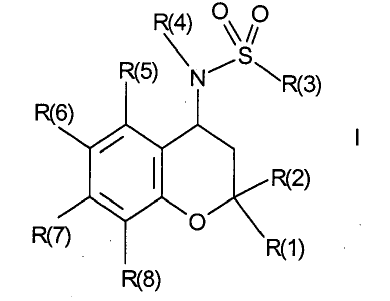 in which:
R(1) and R(2)
independently of one another are hydrogen, CₚF₂ₚ₊₁, alkyl having 1, 2, 3, 4, 5 or 6 C atoms or phenyl,
which is unsubstituted or substituted by 1 or 2 substituents selected from the group consisting of F, Cl, Br, I, CF₃, methyl, methoxy, sulfamoyl, methylsulfonylamino and methylsulfonyl;
p is 1, 2 or 3;
or
R(1) and R(2)
together are an alkylene chain having 2, 3, 4, 5, 6, 7, 8, 9 or 10 C atoms;
R(3) is R(9)-CₙH₂ₙ[NR(11)]ₘ-;
R(9) is hydrogen or cycloalkyl having 3, 4, 5, 6, 7 or 8 C atoms;
n is zero, 1, 2, 3, 4, 5, 6, 7, 8, 9 or 10;
m is zero or 1;
R(11) is hydrogen or alkyl having 1, 2, 3, 4, 5 or 6 C atoms;
or
R(11) together with R(9) is an alkylene group having 1, 2, 3, 4, 5, 6, 7 or 8 C atoms;
where a CH₂ group of the group CₙH₂ₙ can be replaced by -O-, -SO_{q}- or -NR(10)-;
q is zero, 1 or 2;
R(10) is hydrogen, methyl or ethyl;
R(4) is R(12)-CᵣH₂ᵣ;
R(12) is
hydrogen, cycloalkyl having 3, 4, 5, 6, 7 or 8 C atoms, piperidyl, 1-pyrrolidinyl, N-morpholino, N-methylpiperazino, CₚF₂ₚ₊₁, pyridyl, thienyl, imidazolyl or phenyl,
which is unsubstituted or substituted by 1 or 2 substituents selected from the group consisting of F, Cl, Br, I, CF₃, methyl, methoxy, sulfamoyl, methylsulfonyl and methylsulfonylamino;
r is 1, 2, 3, 4, 5, 6, 7, 8, 9, 10, 11, 12, 13, 14, 15, 16, 17, 18, 19 or 20;
where a CH₂ group of the group CᵣH₂ᵣ can be replaced by -O-, -HC=CH-, -C≡C-, -CO-, -CO-O-, -SO_{q}- or -NR(10)-;
q is zero, 1 or 2;
R(10) is hydrogen, methyl or ethyl;
R(5), R(6), R(7) and R(8)
independently of one another are hydrogen, F, Cl, Br, I, alkyl having 1, 2, 3 or 4 C atoms, cycloalkyl having 3, 4, 5, 6, 7 or 8 C atoms, -CN, -CF₃, -C₂F₅, -C₃F₇, -N₃, -NO₂, -CONR(13)R(14), -COOR(15), R(16)-CₛH₂ₛ-Y-or phenyl,
which is unsubstituted or substituted by 1 or 2 substituents selected from the group consisting of F, Cl, Br, I, CF₃, methyl, methoxy, sulfamoyl and methylsulfonyl;
R(13) and R(14)
independently of one another are hydrogen or alkyl having 1, 2 or 3 C atoms;
R(15) is hydrogen, methyl, ethyl, phenyl or -CᵤH₂ᵤ-NR(13)R(14);
u is 2 or 3;
R(16) is hydrogen, cycloalkyl having 3, 4, 5, 6, 7 or 8 C atoms, -COOR(15), thienyl, imidazolyl, pyridyl, quinolyl, isoquinolyl, piperidyl, 1-pyrrolidinyl, N-morpholino, N-methylpiperazino, CₜF₂ₜ₊₁ or phenyl,
which is unsubstituted or substituted by 1 or 2 substituents selected from the group consisting of F, Cl, Br, 1, CF₃, methyl, methoxy, sulfamoyl or methylsulfonyl;
s is zero, 1, 2, 3, 4, 5 or 6;
t is 1, 2 or 3;
Y is SO_{q}, -CO-, -SO₂-NR(10)-, -O-, -NR(10)- or -CO-NR(10);
but where R(6) may not be -OCF₃ or -OC₂F₅;
and their physiologically acceptable salts;
or
2) EP 847 996 (HOE 96/F341)
compounds of the formula I, 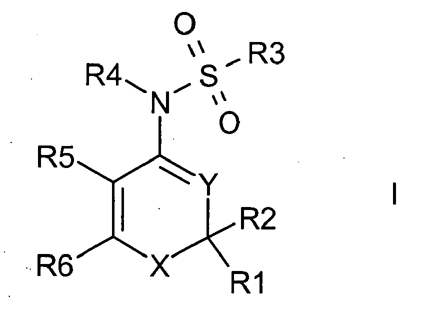 in which:
X is -O-, -S-, -SO-, -SO₂-, -NR(7)-, -CR(8a)R(8b)- or -CO-;
R(7) is hydrogen or-(CaH₂ₐ)-R(9),
where a CH₂ group of the group CₐH₂ₐ can be replaced by -O-, -CH=CH-, -C≡C-, -CO-, -CO-O-, -O-CO-; -S-, -SO-, -SO₂-, NR(10)- or -CONR(10)-;
R(10) is hydrogen or alkyl having 1, 2 or 3 C atoms;
a is zero, 1, 2, 3, 4, 5, 6, 7 or 8;
R(9) is hydrogen, CF₃, C₂F₅, C₃F₇, cycloalkyl having 3, 4, 5, 6, 7 or 8 C atoms, dimethylamino, diethylamino, 1-piperidyl, 1-pyrrolidinyl, 4-morpholinyl, 4-methylpiperazin-1-yl, pyridyl, thienyl, imidazolyl or phenyl,
where pyridyl, thienyl, imidazolyl and phenyl are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
or
R(7) and R(1)
together are a bond;
R(8a) is hydrogen, CF₃, C₂F₅, C₃F₇, alkyl having 1, 2, 3, 4, 5 or 6 C atoms or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino.;
R(8b) is hydrogen, alkyl having 1, 2 or 3 C atoms, -OR(10), -COOR(10), CO-R(10);
R(10) is hydrogen or alkyl having 1, 2 or 3 C atoms;
or
one of the radicals (8a) or R(8b)
together with R(1) is a bond, if Y is N;
Y is N or CR(11);
R(11) is hydrogen or alkyl having 1, 2 or 3 C atoms;
R(1) and R(2)
independently of one another are hydrogen, CF₃, C₂F₅, C₃F₇, F, Cl, methoxy, alkyl having 1, 2, 3, 4, 5 or 6 C atoms or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
or
R(1) and R(2)
together are an alkylene chain having 2, 3, 4, 5, 6, 7, 8, 9 or 10 C atoms;
R(3) is R(12)-CₙH₂ₙ-NR(13)- or R(12)-CₙH₂ₙ-,
where one CH₂ group in the groups CₙH₂ₙ can be replaced by -0-, - CO-, -S-, -SO-, -SO₂- or -NR(10a)-;
R(10a)
is hydrogen, methyl or ethyl;
R(12)
is hydrogen, methyl, cycloalkyl having 3, 4, 5, 6, 7 or 8 C atoms, CF₃, C₂F₅ or C₃F₇;
n is zero, 1, 2, 3, 4, 5, 6, 7, 8, 9 or 10;
R(13)
is hydrogen or alkyl having 1, 2, 3, 4, 5 or 6 C atoms;
or
R(12) and R(13)
together are a bond if n is not less than 3;
or
R(3) and R(4)
together are an alkylene chain having 3, 4, 5, 6, 7 or 8 C atoms, where one CH₂ group of the alkylene chain may be replaced by -O-, -CO-, -S-, -SO-, -SO₂- or -NR(10a)-;
R(10a)
is hydrogen, methyl or ethyl;
R(4) is R(14)-CᵣH₂ᵣ,
where one CH₂ group of the group CᵣH₂ᵣ can be replaced by -0-, - CH=CH-, -C≡C-, -CO-, -CO-O-, -O-CO-, -S-, -SO-, -SO₂-, -NR(10b)- or -CONR(10b)-;
R(10b)
is hydrogen or alkyl having 1, 2 or 3 C atoms;
R(14)
is methyl, CF₃, C₂F₅, C₃F₇, cycloalkyl having 3, 4, 5, 6, 7 or 8 C atoms, -OH, -COOH, -NR(23)R(24), 1-piperidyl, 1-pyrrolidinyl, 4-morpholinyl, 4-methylpiperazin-1-yl, pyridyl, thienyl, imidazolyl or phenyl,
where pyridyl, thienyl, imidazolyl and phenyl are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(23) and R(24)
independently of one another are hydrogen or alkyl having 1, 2 or 3 C atoms;
or
R(23) and R(24)
together are a chain of 4 or 5 methylene groups, one CH₂ group of which can be replaced by -O-, -S-, -NH-, -N(CH₃)- or -N(benzyl)-;
r is zero, 1, 2, 3, 4, 5, 6, 7, 8, 9, 10, 11, 12, 13, 14, 15, 16, 17, 18, 19 or 20;
R(5) and R(6)
together are a group
-CR(15)=CR(16)-CR(17)=CR(18)-,
-CR(15)=CR(16)-CR(17)=N-,
-CR(15)=CR(16)-N=CR(18)-,
-CR(15)=N-CR(17)=N-,
-CR(15)=N-N=CR(18)-,
-N=CR(16)-CR(17)=N- or
-S-CR(15)=CR(16)-,
where in each case both directions of attachment are possible;
R(15), R(16), R(17) and R(18)
independently of one another are hydrogen, F, Cl, Br, I, alkyl having 1, 2, 3, 4 or 5 C atoms, cycloalkyl having 3, 4, 5, 6, 7 or 8 C atoms, -CN, -CF₃, -C₂F₅, -C₃F₇, -N₃, -NO₂, -Z-CₛH₂ₛ-R(22), thienyl or phenyl,
which is unsubstituted or substituted by 1 or 2 substituents selected from the group consisting of F, Cl, Br, l, CF₃, NO₂, CN, NH₂, OH, methyl, ethyl, methoxy, dimethylamino, sulfamoyl, methylsulfonyl and methylsulfonylamino;
Z is -O-, -CO-, -CO-O-, -O-CO-, -S-, -SO-, -SO₂-, -SO₂-O-, -SO₂NR(10c), -NR(10c)- or -CONR(10c)-; R(10c) is hydrogen or alkyl having 1, 2 or 3 C atoms;
s is zero, 1, 2, 3, 4, 5 or 6;
R(22) is hydrogen, CF₃, C₂F₅, C₃F₇, cycloalkyl having 3, 4, 5, 6, 7 or 8 C atoms, -NR(19)R(20), -COOR(21), 1-piperidyl, 1-pyrrolidinyl, 4-morpholinyl, 4-methylpiperazin-1-yl, pyridyl, thienyl, imidazolyl, quinolyl, isoquinolyl or phenyl,
where pyridyl, thienyl, imidazolyl, quinolyl, isoquinolyl and phenyl are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(19) and R(20)
independently of one another are hydrogen or alkyl having 1, 2 or 3 C atoms;
or
R(19) and R(20)
together are a chain of 4 or 5 methylene groups, one CH₂ group of which can be replaced by -O-, -S-, -NH-, -N(CH₃)- or -N(benzyl)-;
R(21) is hydrogen or alkyl having 1, 2 or 3 C atoms;
but where Y may not be CR(11) at the same time as X is O;
and their physiologically acceptable salts;
or
3) EP 857 724 (HOE 97/F 024)
compounds of the formula I 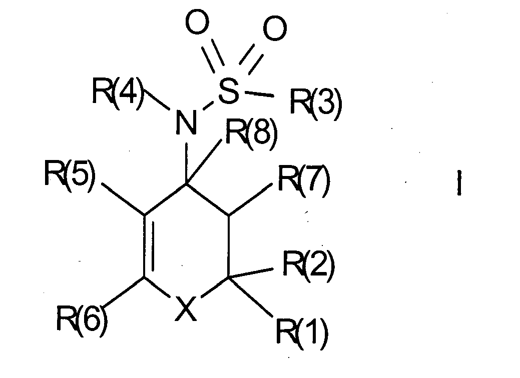 in which:
X is -[S(O)_{zero, 1 or 2}]-, -NR(9)-, -[CR(9)R(23)]- or -CO-;
R(9) is hydrogen or -(CₙH₂ₙ)-R(10);
n is zero, 1, 2, 3, 4, 5, 6, 7 or 8;
R(10) is hydrogen, cycloalkyl having 3, 4, 5, 6, 7 or 8 C atoms, piperidyl, 1-pyrrolidinyl, N-morpholino, N-methylpiperazino, CF₃, C₂F₅ or C₃F₇;
where one CH₂ group of the group CₙH₂ₙ can be replaced by -O-, -CH=CH-, -C≡C-, -CO-, -CO-O-, -[SO_{zero}, _{1 or 2}]- or -NR(11)-;
R(11) is hydrogen, methyl or ethyl;
or
R(10) is pyridyl, thienyl, imidazolyl or phenyl,
which are unsubstituted or substituted by 1 or 2 substituents selected from the group consisting of F, Cl, Br, I, CF₃, methyl, methoxy, sulfamoyl, methylsulfonyl and methylsulfonylamino;
or
R(9) together with R(1) is a bond;
R(23) is hydrogen, alkyl having 1, 2 or 3 C atoms, OH, O-alkyl having 1, 2 or 3 C atoms, COOH, COO-alkyl having 1, 2 or 3 C atoms or -CO-R(24);
R(24) is hydrogen, methyl or ethyl;
R(1) and R(2)
independently of one another are hydrogen, CF₃, C₂F₅, C₃F₇, alkyl
having 1, 2, 3, 4, 5 or 6 C atoms or phenyl,
which is unsubstituted or substituted by 1 or 2 substituents selected from the group consisting of F, Cl, Br, I, CF₃, methyl, methoxy, sulfamoyl and methylsulfonyl;
or
R(1) and R(2)
together are an alkylene chain having 2, 3, 4, 5, 6, 7, 8, 9 or 10 C atoms;
R(3) is R(12)-CₐH₂ₐ[NR(13)]ₘ-;
R(12) is hydrogen or cycloalkyl having 3, 4, 5, 6, 7 or 8 C atoms, CF₃, C₂F₅ or C₃F₇;
a is zero, 1, 2, 3, 4, 5, 6, 7, 8, 9 or 10;
m is zero or 1;
R(13) is hydrogen or alkyl having 1, 2, 3, 4, 5 or 6 C atoms;
or
R(12) and R(13)
together are an alkylene group having 4, 5, 6, 7 or 8 C atoms,
where one CH₂ group of the alkylene group can be replaced by -O-, -[SO_{zero}, _{1 or 2}]-, -CO- or -NR(11)-; R(11) is hydrogen, methyl or ethyl;
R(4) is R(14)-CᵣH₂ᵣ;
r is 1, 2, 3, 4, 5, 6, 7, 8, 9, 10, 11, 12, 13, 14, 15, 16, 17, 18, 19 or 20;
R(14) is hydrogen, cycloalkyl having 3, 4, 5, 6, 7 or 8 C atoms, piperidyl, 1-pyrrolidinyl, N-morpholino, N-methylpiperazino, CF₃, C₂F₅, C₃F₇, pyridyl, thienyl, imidazolyl or phenyl,
which is unsubstituted or substituted by 1 or 2 substituents selected from the group consisting of F, Cl, Br, I, CF₃, methyl, methoxy, sulfamoyl, methylsulfonyl and methylsulfonylamino;
where one CH₂ group of the group CᵣH₂ᵣ can be replaced by -O-, -CH=CH-, -C≡C-, -CO-, -CO-O-, -CO-NR(11)-, -[SO_{zero}, _{1 or 2}]- or -NR(11)-;
or
R(3) and R(4)
together are an alkylene chain having 3, 4, 5, 6, 7 or 8 C atoms,
where one CH₂ group of the alkylene chain can be replaced by -O-, -[SO_{zero}, _{1 or 2}]-, -CO- or -NR(11)-;
R(5) and R(6)
together are -CR(15)=CR(16)-CR(17)=CR(18)-, -CR(15)=CR(16)-CR(17)=N-, -CR(15)=CR(16)-N=CR(18)-, -CR(15)=N-CR(17)=N-, -CR(15)=N-N=CR(18)-, -N=CR(16)-CR(17)=N- or -S-CR(15)=CR(16)-; R(15), R(16), R(17) and R(18)
independently of one another are hydrogen, F, Cl, Br, I, alkyl having 1, 2, 3 or 4 C atoms, cycloalkyl having 3, 4, 5, 6, 7 or 8 C atoms, CN, CF₃, C₂F₅, C₃F₇, N₃, NO₂, -CONR(19)R(20), -COOR(21), R(22)-CₛH₂ₛ-Z- or phenyl,
which is unsubstituted or substituted by 1 or 2 substituents selected from the group consisting of F, Cl, Br, I, CF₃, methyl, methoxy, sulfamoyl and methylsulfonyl;
R(19) and R(20)
independently of one another are hydrogen or alkyl having 1, 2 or 3 C atoms;
R(21) is hydrogen, methyl, ethyl, phenyl or -CᵤH₂ᵤ-NR(19)R(20);
u is 2 or 3;
where the phenyl is unsubstituted or substituted by 1 or 2 substituents selected from the group consisting of F, Cl, Br, I, CF₃, methyl, methoxy, sulfamoyl or methylsulfonyl;
R(22) is hydrogen, cycloalkyl having 3, 4, 5, 6, 7 or 8 C atoms, -COOR(21), thienyl, imidazolyl, pyridyl, quinolyl, isoquinolyl, piperidyl, 1-pyrrolidinyl, N-morpholino, N-methylpiperazino, CF₃, C₂F₅ or C₃F₇ or phenyl,
which is unsubstituted or substituted by 1 or 2 substituents selected from the group consisting of F, Cl, Br, I, CF₃, methyl, methoxy, sulfamoyl or methylsulfonyl;
s is zero, 1, 2, 3, 4, 5 or 6;
Z is -[S(O)_{zero, 1 or 2]}-, -CO-, -SO₂-NR(11)-, -SO₂ -O-, -O-, -NR(11)- or -[CO-NR(11)]-;
R(7) is hydrogen, hydroxyl, alkoxy having 1, 2, 3 or 4 C atoms, acyloxy having 1, 2, 3 or 4 C atoms, Cl, Br, F, alkyl having 1, 2, 3 or 4 C atoms;
R(8) is hydrogen or alkyl having 1, 2, 3, 4, 5 or 6 C atoms;
and their physiologically acceptable salts;
or
4) EP 860 440 (HOE 97/F033)
chromane derivatives of the formula I 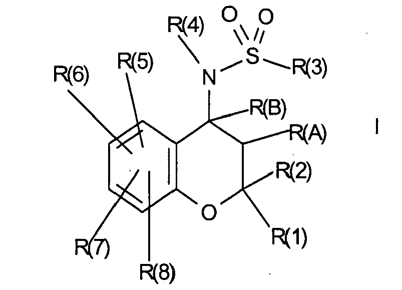 in which:
R(1) and R(2)
independently of one another are hydrogen, CF₃, C₂F₅, C₃F₇, alkyl having 1, 2, 3, 4, 5 or 6 C atoms or phenyl,
which is unsubstituted or substituted by 1 or 2 substituents selected from the group consisting of F, Cl, Br, I, CF₃, methyl, methoxy, sulfamoyl and methylsulfonyl;
or
R(1) and R(2)
together are an alkylene chain having 2, 3, 4, 5, 6, 7, 8, 9 or 10 C atoms;
R(A) is hydroxyl, alkanoyloxy having 1, 2, 3, 4, 5 or 6 C atoms or alkylsulfonyloxy having 1, 2, 3, 4, 5 or 6 C atoms;
R(B) is hydrogen;
or
R(A) and R(B)
together are a bond;
R(3) is R(9)-CₙH₂ₙ[NR(11)]ₘ-;
R(9) is hydrogen or cycloalkyl having 3, 4, 5, 6, 7 or 8 C atoms;
n is zero, 1, 2, 3, 4, 5, 6, 7, 8, 9 or 10;
m is zero or 1;
R(11) is hydrogen or alkyl having 1, 2, 3, 4, 5 or 6 C atoms;
or
R(11) and R(9)
together are an alkylene group having 1, 2, 3, 4, 5, 6, 7 or 8 C atoms;
where one CH₂ group of the group CₙH₂ₙ can be replaced by -O-, SO_{zero, 1 or 2}- or -NR(10);
R(10) is hydrogen, methyl or ethyl;
R(4) is R(12)-CᵣH₂ᵣ;
R(12) is hydrogen, cycloalkyl having 3, 4, 5, 6, 7 or 8 C atoms, piperidyl, 1-pyrrolidinyl, N-morpholino, N-methylpiperazino, CF₃, C₂F₅, C₃F₇, pyridyl, thienyl, imidazolyl or phenyl,
where pyridyl, thienyl, imidazolyl or phenyl are unsubstituted or substituted by 1 or 2 substituents selected from the group consisting of F, Cl, Br, I, CF₃, methyl, methoxy, sulfamoyl, methylsulfonyl and methylsulfonylamino;
r is zero, 1, 2, 3, 4, 5, 6, 7, 8, 9, 10, 11, 12, 13, 14, 15, 16, 17, 18, 19 or 20;
where one CH₂ group of the group CᵣH₂ᵣ can be replaced by -O-, >CH=CH<, -C≡C-, -CO-, -CO-O-, SO_{zero}, 1 or 2- or -NR(10)-;
R(5), R(6), R(7) and R(8)
independently of one another are hydrogen, F, Cl, Br, I, alkyl having 1, 2, 3, 4, 5 or 6 C atoms, cycloalkyl having 3, 4, 5, 6, 7 or 8 C atoms, -CN, -CF₃, -C₂F₅, -C₃F₇, -N₃, -NO₂, -CONR(13)R(14), -COOR(15), R(16)-CₛH₂ₛ-Y- or phenyl,
which is unsubstituted or substituted by 1 or 2 substituents selected from the group consisting of F, Cl, Br, I, CF₃, methyl, methoxy, sulfamoyl and methylsulfonyl;
R(13) and R(14)
independently of one another are hydrogen or alkyl having 1, 2 or 3 C atoms;
R(15) is hydrogen, methyl, ethyl, phenyl or -CᵤH₂ᵤ-NR(13)R(14);
u is 2 or 3;
R(16) is hydrogen, cycloalkyl having 3, 4, 5, 6, 7 or 8 C atoms, -COOR(15), thienyl, imidazolyl, pyridyl, quinolyl, isoquinolyl, piperidyl, 1-pyrrolidinyl, N-morpholino, N-methylpiperazino, CF₃, C₂F₅, C₃F₇ or phenyl,
which is unsubstituted or substituted by 1 or 2 substituents selected from the group consisting of F, Cl, Br, I, CF₃, methyl, methoxy, sulfamoyl and methylsulfonyl;
s is zero, 1, 2, 3, 4, 5 or 6;
Y -S-, -SO-, -SO₂-, -CO-, -SO₂-NR(10)-, -O-, -NR(10)- or -CO-NR(10);
but with the proviso that two of the substituents R(5), R(6), R(7) and R(8)
are not hydrogen;
and their physiologically acceptable salts;
or
5) EP 861 836 (HOE 97/F038)
compounds of the formula I 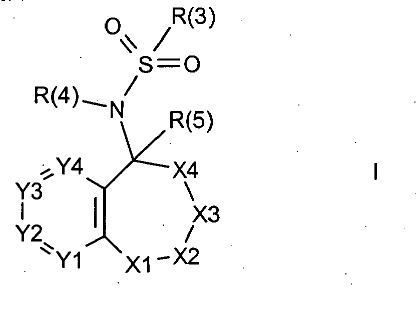 in which:
X1 is -O-, -S-, -SO-, -SO₂-, -CR(1)R(2)-, -NR(6)-, -CO- or -CR(1)R(7)-;
R(1) and R(2)
independently of one another are hydrogen, CF₃, C₂F₅, C₃F₇, alkyl having 1, 2, 3, 4, 5 or 6 C atoms or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
or
R(1) and R(2)
together are an alkylene chain having 2, 3, 4, 5, 6, 7, 8, 9 or 10 C atoms;
R(6) is hydrogen or -CₙH₂ₙ-R(8),
where one CH₂ group of the group CₙH₂ₙ can be replaced by -O-, -CH=CH-, -C≡C-, -CO-, -CO-O-, -O-CO-, -S-, -SO-, -SO₂-, -NR(9)- or -CONR(9)-;
R(9) is hydrogen or alkyl having 1, 2 or 3 C atoms;
n is zero, 1, 2, 3, 4, 5, 6, 7 or 8;
R(8) is hydrogen, CF₃, C₂F₅, C₃F₇, cycloalkyl having 3, 4, 5, 6, 7 or 8 C atoms, dimethylamino, diethylamino, 1-piperidyl, 1-pyrrolidinyl, 4-morpholinyl, 4-methylpiperazin-1-yl, pyridyl, thienyl, imidazolyl or phenyl,
where pyridyl, thienyl, imidazolyl and phenyl are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
X2 is -CR(1)R(2)- or -CR(2)R(10)-;
or
X2 is, if X3 and X1 are -CR(1)R(2)-, also -O-, -S-, -SO-, -SO₂- or -NR(6)-,
where the radicals R(1), R(2) and R(6)
are as defined under X1 but independent from the meanings of the radicals in X1;
R(10) together with R(7) is a bond;
X3 is -CR(1)R(2)-;
or
X3 is, if X2 and X4 are -CR(1)R(2)-, also -O-, -S-, -SO-, -SO₂- or -NR(6)-,
where the radicals R(1), R(2) and R(6)
are as defined under X1 but independent from the meanings of the radicals in X1;
X4. is -CR(1)R(2)-, -NR(6)-, -NR(11)-, -CH(OR(30))- or -CR(2)R(11)-,
where the radicals R(1), R(2) and R(6)
are as defined under X1 but independent from the meanings of the radicals in X1;
R(30) is hydrogen, alkyl-having 1, 2 or 3 C atoms or acyl having 1, 2, 3 or 4 C atoms;
R(11) together with R(5) is a bond;
Y1, Y2, Y3 and Y4
independently of one another are -CR(12)- or N,
where at most 2 of the groups Y1, Y2, Y3 and Y4 may simultaneously be N;
the radicals R(12)
independently of one another are hydrogen, F, Cl, Br, I, alkyl having 1, 2, 3, 4 or 5 C atoms, cycloalkyl having 3, 4, 5, 6, 7 or 8 C atoms, CN, CF₃, C₂F₅, C₃F₇, N₃, NO₂, -Z-CₘH₂ₘ-R(13) or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Z is -O-, -CO-, -CO-O-, -O-CO-, -S-, -SO-, -SO₂-, -SO₂NR(14)-, -NR(14)- or -CONR(14)-;
R(14) is hydrogen or alkyl having 1, 2 or 3 C atoms;
m is zero, 1, 2, 3, 4, 5 or 6;
R(13) is hydrogen, CF₃, C₂F₅, C₃F₇, cycloalkyl having 3, 4, 5, 6, 7 or 8 C atoms, -NR(15)R(16), -CONR(15)R(16), -OR(30a), phenyl, thienyl or an N-containing heterocycle having 1, 2, 3, 4, 5, 6, 7, 8 or 9 C atoms,
where phenyl, thienyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15) and R(16)
independently of one another are hydrogen or alkyl having 1, 2 or 3 C atoms;
or
R(15) and R(16)
together are a chain of 4 or 5 methylene groups, one CH₂ group of which can be replaced by -O-, -S-, -NH-, -N(CH₃)- or -N(benzyl)-;
R(30a)
is hydrogen, alkyl having 1, 2 or 3 C atoms or acyl having 1, 2, 3 or 4 C atoms;
or
Y1 and Y2
together are an S atom and Y3 and Y4 are each -CR(12)-;
the radicals R(12)
are independently of one another as defined under Y1, Y2, Y3, Y4;
R(3) is R(17)-CₓH₂ₓ-NR(18)- or R(17)-CₓH₂ₓ-,
where one CH₂ group in the groups CₓH₂ₓ can be replaced by -O-, -CO-, -S-, -SO-, -SO₂- or -NR(19)-;
R(19) is hydrogen, methyl or ethyl;
R(17) is hydrogen, methyl, cycloalkyl having 3, 4, 5, 6, 7 or 8 C atoms, CF₃, C₂F₅ or C₃F₇;
x is zero, 1, 2, 3, 4, 5, 6, 7, 8, 9 or 10;
R(18) is hydrogen or alkyl having 1, 2, 3, 4, 5, 6, 7 or 8 C atoms;
or
R(18) and R(17)
together are a bond if x is not less than 3;
or
R(3) is phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
or
R(3) together with R(4)
are an alkylene chain having 3, 4, 5, 6, 7 or 8 C atoms,
where one CH₂ group of the alkylene chain can be replaced by -O-, -CO-, -S-, -SO- or SO₂-;
R(4) is -CᵣH₂ᵣR(20),
where one CH₂ group of the group CᵣH₂ᵣ can be replaced by -O-, -CH=CH-, -C≡C-, -CO-, -CO-O-, -O-CO-, -S-, -SO-, -SO₂-, -NR(21)- or -CONR(21)-;
R(21) is hydrogen or alkyl having 1, 2 or 3 C atoms;
r is zero, 1, 2, 3, 4, 5, 6, 7, 8, 9, 10, ,11, 12, 13, 14, 15, 16, 17, 18, 19 or 20;
R(20) is hydrogen, methyl, CF₃, C₂F₅, C₃F₇, cycloalkyl having 3, 4, 5, 6, 7 or 8 C atoms, -NR(22)R(23), phenyl, thienyl or an N-containing heterocycle having 1, 2, 3, 4, 5, 6, 7, 8 or 9 C atoms,
where phenyl, thienyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(22) and R(23)
independently of one another are hydrogen or alkyl having 1, 2 or 3 C atoms;
or
R(22) and R(23)
together are a chain of 4 or 5 methylene groups,
one CH₂ group of which can be replaced by -O-, -S-, -NH-, -N(CH₃)- or -N(benzyl)-;
R(5) is hydrogen or together with R(11) is a bond;
in all their stereoisomeric forms and mixtures thereof in any ratios, and their physiologically acceptable salts;
or
6) HMR 97/L206 - EP 895 994
compounds of the formula I 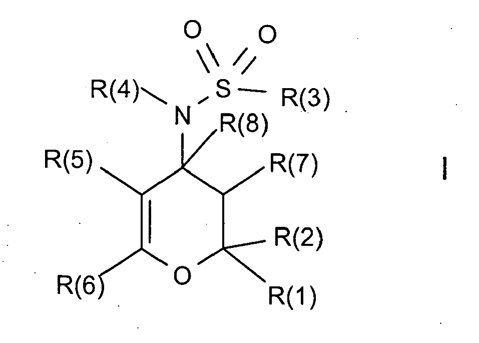 in which:
R(1) and R(2)
independently of one another are hydrogen, CF₃, C₂F₅, C₃F₇, alkyl having 1, 2, 3, 4, 5 or 6 C atoms or phenyl,
which is unsubstituted or substituted by 1 or 2 substituents selected from the group consisting of F, Cl, Br, I, CF₃, methyl, methoxy, sulfamoyl and methylsulfonyl;
or
R(1) and R(2)
together are an alkylene chain having 2, 3, 4, 5, 6, 7, 8, 9 or 10 C atoms;
where one CH₂ group of the alkylene chain can be replaced by -O-, -CO-, -S-, -SO-, -SO₂- or -NR(10)-;
R(10) is hydrogen or alkyl having 1, 2 or 3 C atoms;
R(3) is R(12)-CₐH₂ₐ[NR(13)]ₘ-;
R(12) is hydrogen or cycloalkyl having 3, 4, 5, 6, 7 or 8 C atoms, CF₃, C₂F₅ or C₃F₇;
a is zero, 1, 2, 3, 4, 5, 6, 7, 8, 9 or 10;
m is zero or 1;
R(13) is hydrogen or alkyl having 1, 2, 3, 4, 5 or 6 C atoms;
or
R(12) and R(13)
together are an alkylene group having 4, 5, 6, 7 or 8 C atoms, where one CH₂ group of the alkylene group can be replaced by -O-, -[SO_{zero, 1 or 2}]-, -CO- or -NR(10)-; R(10) is hydrogen or alkyl having 1, 2 or 3 C atoms;
R(4) R(14)-CᵣH₂ᵣ;
r is zero, 1, 2, 3, 4, 5, 6, 7, 8, 9, 10, 11, 12, 13, 14, 15, 16, 17, 18, 19 or 20;
R(14) is hydrogen, cycloalkyl having 3, 4, 5, 6, 7 or 8 C atoms, piperidyl, 1-pyrrolidinyl, N-morpholino, N-methylpiperazino, CF₃, C₂F₅, C₃F₇, pyridyl, thienyl, imidazolyl or phenyl,
which is unsubstituted or substituted by 1 or 2 substituents selected from the group consisting of F, Cl, Br, I, CF₃, methyl, methoxy, sulfamoyl, methylsulfonyl and methylsulfonylamino;
where one CH₂ group of the group CᵣH₂ᵣ can be replaced by -O-, -CH=CH-, -C≡C-, -CO-, -CO-O-, -CO-NR(11)-, -[SO_{zero, 1 or 2}]- or -NR(11)-;
R(11) is hydrogen or -(CₐH₂ₐ)-R(10);
where one CH₂ group of the group CₐH₂ₐ can be replaced by -O-, -CH=CH-, -C≡C-, -CO-, -CO-O-, -O-CO-, -S-, -SO-, -SO₂-, NR(10)- or -CONR(10)-;
R(10) is hydrogen or alkyl having 1, 2 or 3 C atoms;
or
R(3) and R(4)
together are an alkylene chain having 3, 4, 5, 6, 7 or 8 C atoms,
where one CH₂ group of the alkylene chain can be replaced by -O-, -[SO_{zero, 1 or 2}]-, -CO- or -NR(11)-;
R(11) is hydrogen or -(CₐH₂ₐ)-R(10),
where one CH₂ group of the group CₐH₂ₐ can be replaced by -O-, -CH=CH-, -C≡C-, -CO-, -CO-O-, -O-CO-, -S-, -SO-, -SO₂-, NR(10)- or -CONR(10)-;
R(10) is hydrogen or alkyl having 1, 2 or 3 C atoms;
R(5) and R(6) are
-CR(15)=CR(16)-CR(17)=N-,
-CR(15)=CR(16)-N=CR(17)-,
-CR(15)=N-CR(17)=N-,
-CR(15)=N-N=CR(17)-,
-N=CR(16)-CR(17)=N- or
-S-CR(15)=CR(16)-;
R(15), R(16) and R(17)
independently of one another are hydrogen, F, Cl, Br, I, alkyl having 1, 2, 3 or 4 C atoms, cycloalkyl having 3, 4, 5, 6, 7 or 8 C atoms, CN, CF₃, C₂F₅, C₃F₇, N₃, NO₂, -CONR(19)R(21), -COOR(21), R(22)-CₛH₂ₛ-Z- or phenyl,
which is unsubstituted or substituted by 1 or 2 substituents selected from the group consisting of F, Cl, Br, I, CF₃, methyl, methoxy, sulfamoyl and methylsulfonyl;
R(19) is hydrogen or alkyl having 1, 2 or 3 C atoms;
R(21) is hydrogen, methyl, ethyl, phenyl or -CuH₂ᵤ-NR(19)R(20);
where the phenyl is unsubstituted or substituted by 1 or 2 substituents selected from the group consisting of F, Cl, Br, I, CF₃, methyl, methoxy, sulfamoyl and methylsulfonyl;
R(20)
is hydrogen or alkyl having 1, 2 or 3 C atoms;
u is 2 or 3;
R(22) is hydrogen, cycloalkyl having 3, 4, 5, 6, 7 or 8 C atoms, -COOR(21), CONR(19)R(21), thienyl, imidazolyl, pyridyl, quinolyl, isoquinolyl, piperidyl, 1-pyrrolidinyl, N-morpholino, N-methylpiperazino, CF₃, C₂F₅, C₃F₇ or phenyl,
which is unsubstituted or substituted by 1 or 2 substituents selected from the group consisting of F, Cl, Br, l, CF₃, methyl, methoxy, sulfamoyl and methylsulfonyl;
s is zero, 1, 2, 3, 4, 5 or 6;
Z is -[S(O)_{zero, 1 or 2}]-, -CO-, -SO_{(0, 1 or 2)}-NR(11)-, -SO₂-O-, -O-, -NR(11)- or -[CO-NR(11)]-;
R(7) is hydrogen, hydroxyl, alkoxy having 1, 2, 3 or 4 C atoms, acyloxy having 1, 2, 3 or 4 C atoms, Cl, Br, F, alkyl having 1, 2, 3 or 4 C atoms;
R(8) is hydrogen or alkyl having 1, 2, 3, 4, 5 or 6 C atoms;
and their physiologically acceptable salts;
or
7) HMR 97/L223 EP-A 905 131
compounds of the formula I 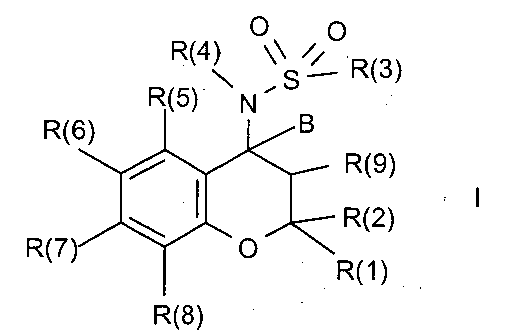 in which:
R(1) and R(2)
independently of one another are hydrogen, CF₃, C₂F₅, C₃F₇, alkyl having 1, 2, 3, 4, 5 or 6 C atoms or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
or
R(1) and R(2)
together are an alkylene chain having 2, 3, 4, 5, 6, 7, 8, 9 or 10 C atoms;
R(3) is R(10)-CₙH₂ₙ-NR(11)-orR(10)-CₙH₂ₙ-,
where one CH₂ group in the groups CₙH₂ₙ can be replaced by -O-, -CO-, -S-, -SO-, -SO₂- or -NR(12a)-;
R(12a) is hydrogen, methyl or ethyl;
R(10) is hydrogen, methyl, cycloalkyl having 3, 4, 5, 6, 7 or 8 C
atoms, CF₃, C₂F₅ or C₃F₇;
n is zero, 1, 2, 3, 4, 5, 6, 7, 8, 9 or 10;
R(11) is hydrogen or alkyl having 1, 2, 3, 4, 5 or 6 C atoms;
or
R(10) and R(11)
together are a bond if n is not less than 3;
R(4) is R(13)-CᵣH₂ᵣ-Z-C_{q}H_{2q}-;
q is 0, 1, 2, 3, 4, 5, 6, 7 or 8;
r is 0, 1, 2, 3, 4, 5, 6, 7 or 8;
Z is -CO-NR(14)-,
-OCO-NR(14)-,
-O-CₓH₂ₓ-O-,
-O-CₓH₂ₓ-NR(14)-,
-O-CₓH₂ₓ-CO-O,
-CO-O-CₓH₂ₓ-O- or
-CO-O-CₓH₂ₓ-NR(14)-,
where in each case both directions of attachment are possible;
x is 2, 3 or 4;
R(14) is hydrogen, alkyl having 1, 2 or 3 C atoms, -CyH_{2y}-OR(12b), -CyH₂y-NR(12b)₂;
R(12b) is hydrogen, methyl or ethyl;
y is 2 or 3;
R(13) is H, CF₃, C₂F₅, C₃F₇, cycloalkyl having 3, 4, 5, 6, 7 or 8 C atoms, -NR(15)R(16), -CONR(15)R(16), -C(=NR(17))NR(15)R(16), -OR(17), -COOR(17), phenyl or an N-containing heterocycle having 1, 2, 3, 4, 5, 6, 7, 8 or 9 C atoms,
where phenyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl, aminosulfonyl and methylsulfonylamino;
R(15) and R(16)
independently of one another are hydrogen, alkyl having 1, 2, 3 or 4 C atoms or -C_{z}H_{2z}-phenyl,
where phenyl is unsubstituted or substituted by 1 or 2 substituents selected from the group consisting of F, Cl, Br, CF₃, NO₂, CN, OH, methyl, ethyl, methoxy, dimethylamino, sulfamoyl, methylsulfonyl, aminosulfonyl and methylsulfonylamino;
or
R(15) and R(16)
together are a chain of 4 or 5 methylene groups, one CH₂ group of which can be replaced by -O-, -S-, -NH-, -N(CH₃)- or -N(benzyl)-;
R(17) is hydrogen or alkyl having 1, 2 or 3 C atoms;
R(5), R(6), R(7) and R(8)
independently of one another are hydrogen, F, Cl, Br, I, alkyl having 1, 2, 3, 4 or 5 C atoms, cycloalkyl having 3, 4, 5, 6, 7 or 8 C atoms, -CN, -CF₃, -C₂F₅, -C₃F₇, -N₃, -NO₂, -Y-CₛH₂ₛ-R(18) or phenyl,
which is unsubstituted or substituted by 1 or 2 substituents selected from the group consisting of F, Cl, Br, l, CF₃, NO₂, CN, NH₂, OH, methyl, ethyl, methoxy, dimethylamino, sulfamoyl, methylsulfonyl and methylsulfonylamino;
Y is -O-, -CO-, -CO-O-, -O-CO-, -S-, -SO-, -SO₂-, -SO₂-O-, -SO₂NR(10c), -NR(10c)- or -CONR(10c)-;
R(10c) is hydrogen or alkyl having 1, 2 or 3 C atoms;
s is zero, 1, 2, 3, 4, 5 or 6;
R(18) is hydrogen, CF₃, C₂F₅, C₃F₇, cycloalkyl having 3, 4, 5, 6, 7 or 8 C atoms, -COOR(21), 1-piperidyl, 1-pyrrolidinyl, 4-morpholinyl, 4-methylpiperazin-1-yl, pyridyl, thienyl, imidazolyl, quinolyl, isoquinolyl or phenyl,
where pyridyl, thienyl, imidazolyl, quinolyl, isoquinolyl and phenyl are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(21) is hydrogen or alkyl having 1, 2 or 3 C atoms;
R(9) is hydrogen, OR(10d) or OCOR(10d);
R(10d) is hydrogen or alkyl having 1, 2 or 3 C atoms;
B is hydrogen;
or
R(9) and B
together are a bond;
and their physiologically acceptable salts;
or
8) HMR 97/L224 - EP-A 906 911
compounds of the formula I 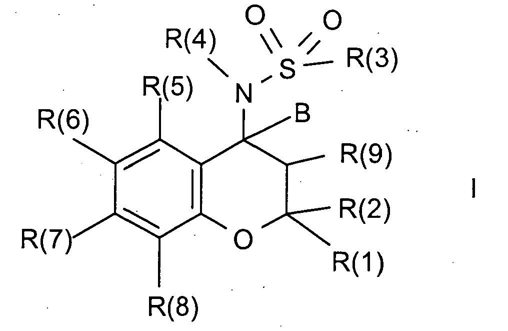 in which:
R(1) and R(2)
independently of one another are hydrogen, CF₃, C₂F₅, C₃F₇, alkyl having 1, 2, 3, 4, 5 or 6 C atoms or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
or
R(1) and R(2)
together are an alkylene chain having 2, 3, 4, 5, 6, 7, 8, 9 or 10 C atoms;
R(3) is R(10)-CₕH₂ₙ-NR(11)- or R(10)-CₙH₂ₙ-,
where one CH₂ group in the groups CₙH₂ₙ can be replaced by -O-, -CO-, -S-, -SO-, -SO₂- or -NR(12a)-;
R(12a) is hydrogen, methyl or ethyl;
R(10) is hydrogen, methyl, cycloalkyl having 3, 4, 5, 6, 7 or 8 C
atoms, CF₃, C₂F₅ or C₃F₇;
n is zero, 1, 2, 3, 4, 5, 6, 7, 8, 9 or 10;
R(11) is hydrogen or alkyl having 1, 2, 3, 4, 5 or 6 C atoms;
or
R(10) and R(11)
together are a bond if n is not less than 3;
or
R(3) together with R(4)
is an alkylene chain having 3, 4, 5, 6, 7 or 8 C atoms,
where one CH₂ group of the alkylene chain can be replaced by -O-, -CO-, -S-, -SO-, -SO₂- or -NR(12a)-;
R(12a) is hydrogen, methyl or ethyl;
R(4) is R(13)-CᵣH₂ᵣ,
where one CH₂ group of the group CᵣH₂ᵣ can be replaced by -O-, -CH=CH-, -C≡C-, -CO-, -CO-O-, -O-CO-, -S-, -SO-, -SO₂-, -NR(14)- or -CONR(14)-;
R(14) is hydrogen, alkyl having 1, 2 or 3 C atoms, -C_{y}H_{2y}-OR(12b), -C_{y}H_{2y}-NR(12b)₂;
R(12b) is hydrogen, methyl or ethyl;
y is 2 or 3;
R(13) is H, CF₃, C₂F₅, C₃F₇, cycloalkyl having 3, 4, 5, 6, 7 or 8 C atoms, -NR(15)R(16), -CONR(15)R(16), -OR(17), -COOR(17), phenyl or an N-containing heterocycle having 1, 2, 3, 4, 5, 6, 7, 8 or 9 C atoms,
where phenyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15) and R(16)
independently of one another are hydrogen or alkyl having 1, 2, 3 or 4 C atoms;
or
R(15) and R(16)
together are a chain of 4 or 5 methylene groups, one CH₂ group of which can be replaced by -O-, -S-, -NH-, -N(CH₃)- or -N(benzyl)-;
R(17) is hydrogen, alkyl having 1, 2 or 3 C atoms, -CₓH₂ₓOR(12c);
R(12c) is hydrogen, methyl or ethyl;
x is 2 or 3;
r is zero, 1, 2, 3, 4, 5, 6, 7, 8, 9, 10, 11, 12, 13, 14, 15, 16, 17, 18, 19 or 20;
at least one of the substituents R(5), R(6), R(7) and R(8)
is -Y-CₛH₂ₛ-R(18), thienyl, furyl or an N-containing heterocycle having 1, 2, 3, 4, 5, 6, 7, 8 or 9 C atoms,
where thienyl, furyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, methylamino, dimethylamino, ethylamino, diethylamino, sulfamoyl, methylsulfonyl and methylsulfonylamino;
Y is -O-, -CO-, -O-CO-, -S-, -SO-, -SO₂-, -SO₂-O-, -SO₂NR(12d)-, -NR(12d)- or -CONR(12d)-,
where the attachment to the benzene ring is in each case effected through the atom on the left;
R(12d) is hydrogen, methyl or ethyl;
s is 1, 2, 3, 4, 5 or 6;
R(18) is substituted phenyl which carries one or two substituents selected from the group consisting of NO₂, CN, NH₂, N(methyl)₂, OH, ethyl, -COOH, -COOmethyl, -COOethyl, -CONH₂, -CON(methyl)₂ ;
or
R(18) is a substituted N-containing heterocycle having 1, 2, 3, 4, 5, 6, 7, 8 or 9 C atoms which carries one or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
or
R(18) is -OR(19), -SO₂R(19), -NR(19)R(20), -CONR(19)R(20);
R(19) and R(20)
independently of one another are CₜH₂ₜ-R(21);
t is zero, 1, 2, 3, 4, 5 or 6;
R(21) is hydrogen, CF₃, C₂F₅, C₃F₇, cycloalkyl having 3, 4, 5, 6, 7 or 8 C atoms, NR(22)R(23), -OR(24), phenyl, thienyl or an N-containing heterocycle having 1, 2, 3, 4, 5, 6, 7, 8 or 9 C atoms,
where phenyl, thienyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(22) and R(23)
independently of one another are hydrogen, alkyl having 1, 2 or 3 C atoms;
or
R(22) and R(23)
together are a chain of 4 or 5 methylene groups, one CH₂ group of which can be replaced by -O-, -S-, -NH-, -N(CH₃)- or -N(benzyl)-;
R(24) is hydrogen, alkyl having 1, 2 or 3 C atoms;
and the in each case other substituents R(5), R(6), R(7) and R(8) which have not been used in the definition above
independently of one another are hydrogen, F, Cl, Br, I, alkyl having 1, 2, 3, 4 or 5 C atoms, cycloalkyl having 3, 4, 5, 6, 7 or 8 C atoms, CN, CF₃, NO₂, OR(12e) or NR(12e)R(12f);
R(12e) and R(12f)
independently of one another are hydrogen or alkyl having 1, 2, 3 or 4 C atoms;
R(9) is hydrogen, OR(12g) or OCOR(12g);
R(12g) is hydrogen or alkyl having 1, 2 or 3 C atoms;
B is hydrogen;
or
R(9) and B
together are a bond;
and their physiologically acceptable salts;
or
9) HMR 97/L232 - EP 913 396
compounds of the formula I 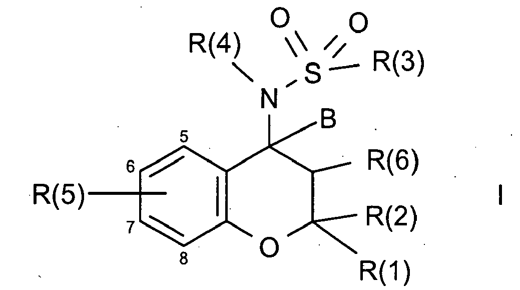 in which R(5) is attached to one of the positions labeled 5, 6, 7 and 8 and in which:
R(1) and R(2)
independently of one another are hydrogen, CF₃, C₂F₅, C₃F₇, alkyl having 1, 2, 3, 4, 5 or 6 C atoms or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
or
R(1) and R(2)
together are an alkylene chain having 2, 3, 4, 5, 6, 7, 8, 9 or 10 C atoms;
R(3) is R(10)-CₙH₂ₙ-NR(11)- or R(10)-CₙH₂ₙ-,
where one CH₂ group in the groups CₙH₂ₙ can be replaced by -O-, -CO-, -S-, -SO-, -SO₂- or -NR(12a)-;
R(12a) is hydrogen, methyl or ethyl;
R(10) is hydrogen, methyl, cycloalkyl having 3, 4, 5, 6, 7 or 8 C
atoms, CF₃, C₂F₅ or C₃F₇;
n is zero, 1, 2, 3, 4, 5, 6, 7, 8, 9 or 10;
R(11) is hydrogen or alkyl having 1, 2, 3, 4, 5 or 6 C atoms;
or
R(10) and R(11)
together are a bond if n is not less than 3;
or
R(3) together with R(4)
is an alkylene chain having 3, 4, 5, 6, 7 or 8 C atoms,
where one CH₂ group of the alkylene chain can be replaced by -O-, -CO-, -S-, -SO-, -SO₂- or -NR(12a)-;
R(12a) is hydrogen, methyl or ethyl;
R(4) is R(13)-CᵣH₂ᵣ,
where one CH₂ group of the group CᵣH₂ᵣ can be replaced by -O-, -CH=CH-, -C≡C-, -CO-, -CO-O-, -O-CO-, -S-, -SO-, -SO₂- or -NR(14)-;
R(14) is hydrogen or alkyl having 1; 2 or 3 C atoms;
R(13) is CH₃, CF₃, C₂F₅, C₃F₇, cycloalkyl having 3, 4, 5, 6, 7 or 8 C atoms, NR(15)R(16), phenyl or an N-containing heterocycle having 1, 2, 3, 4, 5, 6, 7, 8 or 9 C atoms,
where phenyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15) and R(16)
together are a chain of 4 or 5 methylene groups, one CH₂ group of which can be replaced by -O-, -S-, -NH-, -N(methyl)- or -N(benzyl)-;
r is zero, 1, 2, 3, 4, 5, 6, 7, 8, 9, 10, 11, 12, 13, 14, 15, 16, 17, 18, 19 or 20;
R(5) is -Y-CₛH₂ₛ-R(18) or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Y is -O-, -S- or -NR(10c)-;
R(10c) is hydrogen or alkyl having 1, 2 or 3 C atoms;
s is 1, 2, 3, 4, 5, 6, 7 or 8;
R(18) is hydrogen, CF₃, C₂F₅, C₃F₇, cycloalkyl having 3, 4, 5, 6, 7 or 8 C atoms, -COOR(21), NR(15a)R(16a), an unsubstituted N-containing heterocycle having 1, 2, 3, 4, 5, 6, 7, 8 or 9 C atoms, phenyl or thienyl,
where phenyl and thienyl are unsubstituted or substituted by 1 or 2 substituents selected from the group consisting of F, Cl, Br, I, CF₃, methyl, methoxy, sulfamoyl, methylsulfonyl and methylsulfonylamino;
R(15a) and R(16a)
together are a chain of 4 or 5 methylene groups, one CH₂ group of which can be replaced by -O-, -S-, -NH-, -N(methyl)- or -N(benzyl)-;
R(21) is hydrogen or alkyl having 1, 2 or 3 C atoms;
R(6) is OR(10d) or OCOR(10d);
R(10d) is hydrogen or alkyl having 1, 2 or 3 C atoms;
B is hydrogen;
or
R(6) and B
together are a bond;
and their physiologically acceptable salts;
or
10. HMR 97/L235 - EP 915 087
compounds of the formula I 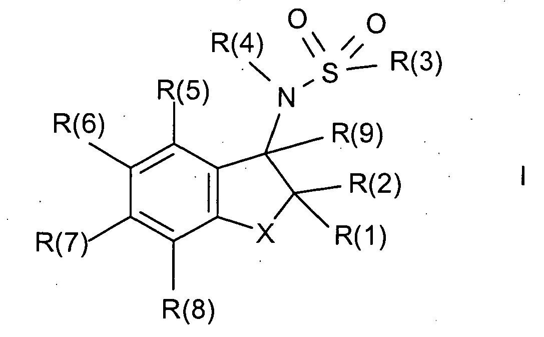 in which:
R(1) and R(2)
independently of one another are hydrogen, CF₃, alkyl having 1, 2, 3, 4, 5 or 6 C atoms;
or
R(2) and R(9)
together are a bond;
or R(2)
is -OR(10a), if X is -CR(22)R(23)-;
R(10a) is hydrogen, acetyl or alkyl having 1, 2 or 3 C atoms;
R(3) is R(10b)-CₙH₂ₙ-NR(11)- or R(1 0b)-CₙH₂ₙ-,
where one CH₂ group in the groups CₙH₂ₙ can be replaced by -O-, -CO-, -S-, -SO-, -SO₂- or -NR(12a)-;
R(12a) is hydrogen, methyl or ethyl;
R(10b) is methyl, cycloalkyl having 3, 4, 5, 6, 7 or 8 C atoms, CF₃, C₂F₅ or C₃F₇;
n is zero, 1, 2, 3, 4, 5, 6, 7, 8, 9 or 10;
R(11) is hydrogen or alkyl having 1, 2, 3, 4, 5 or 6 C atoms;
or
R(10b) and R(11)
together are a bond if n is greater than 2; or
R(3) together with R(4)
is an alkylene chain having 3, 4, 5, 6, 7 or 8 C atoms,
where one CH₂ group of the alkylene chain can be replaced by -O-, -CO-, -S-, -SO-, -SO₂- or -NR(12a)-;
R(12a) is hydrogen, methyl or ethyl;
R(4) is R(13)-CᵣH₂ᵣ,
where one CH₂ group of the group CᵣH₂ᵣ can be replaced by -O-, -CH=CH-, -C≡C-, -CO-, -CO-O-, -O-CO-, -S-, -SO-, -SO₂-, -NR(14)- or -CONR(14)-;
R(14) is hydrogen, alkyl having 1, 2 or 3 C atoms, -C_{y}H_{2y}-OR(12b), -C_{y}H_{2y}-NR(12b)₂;
R(12b) is hydrogen, methyl or ethyl;
y is 2 or 3;
R(13) is CH₃, CF₃, C₂F₅, C₃F₇, cycloalkyl having 3, 4, 5, 6, 7 or 8 C atoms, -NR(15)R(16), -CONR(15)R(16), -OR(17), -COOR(17), phenyl or an N-containing heterocycle having 1, 2, 3, 4, 5, 6, 7, 8 or 9 C atoms,
where phenyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15) and R(16)
independently of one another are hydrogen or alkyl having 1, 2, 3 or 4 C atoms;
or
R(15) and R(16)
together are a chain of 4 or 5 methylene groups, one CH₂ group of which can be replaced by -O-, -S-, -NH-, -N(CH₃)- or -N(benzyl)-;
R(17) is hydrogen, alkyl having 1, 2 or 3 C atoms, -C_{z}H_{2z}OR(12c);
R(12c) is hydrogen, methyl or ethyl;
z is 2 or 3;
r is zero, 1, 2, 3, 4, 5, 6, 7, 8, 9, 10, 11, 12, 13, 14, 15, 16, 17, 18, 19 or 20;
R(5), R(6), R(7) and R(8)
independently of one another are hydrogen, F, Cl, Br, I, alkyl having 1, 2, 3, 4 or 5 C atoms, cycloalkyl having 3, 4, 5, 6, 7 or 8 C atoms, -CN, -CF₃, -C₂F₅, -C₃F₇, -N₃, -NO₂, -Y-CₛH₂ₛ-R(18), phenyl, thienyl, furyl or an N-containing heterocycle having 1, 2, 3, 4, 5, 6, 7, 8 or 9 C atoms,
where phenyl, thienyl, furyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Y is -O-, -CO-, -CO-O-, -O-CO-, -S-, -SO-, -SO₂-, -O-SO₂-, -SO₂NR(10c)- or -CONR(10c)-, where the attachment to the skeleton is in each case effected through the atom on the left; R(10c) is hydrogen or alkyl having 1, 2 or 3 C atoms;
s is zero, 1, 2, 3, 4, 5 or 6;
R(18) is hydrogen, methyl, CF₃, C₂F₅, C₃F₇, cycloalkyl having 3, 4, 5, 6, 7 or 8 C atoms, -OR(21), -COOR(21), -NR(15a)R(16a), -CONR(15a)R(16a), phenyl or an N-containing heterocycle having 1, 2, 3, 4, 5, 6, 7, 8 or 9 C atoms,
where phenyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15a) and R(16a)
independently of one another are hydrogen or alkyl having 1, 2, 3 or 4 C atoms;
or
R(15a) and R(16a)
together are a chain of 4 or 5 methylene groups, one CH₂ group of which can be replaced by -O-, -S-, -NH-, -N(CH₃)- or -N(benzyl)-;
R(21) is hydrogen or alkyl having 1, 2 or 3 C atoms;
R(9) is hydrogen or together with R(2) a bond;
X is -CR(22)R(23)-, -O-, -NR(24)-, -S-, -SO-, -SO₂-;
R(22) and R(23)
independently of one another are hydrogen, CF₃, alkyl having 1, 2, 3, 4, 5 or 6 C atoms;
R(24) is hydrogen, alkyl having 1, 2, 3, 4, 5 or 6 C atoms or phenyl, which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
and their physiologically acceptable salts.
2. The use as claimed in claim 1 of inhibitors of the I_{KS} channel with simultaneous action as inhibitors of the KQT1 channel for preparing a medicament for treating diseases caused by helminths.

## Revendications

1. Utilisation d'inhibiteurs du canal Iₖₛ à action simultanée en tant qu'inhibiteurs du canal KQT1, pour la fabrication d'un médicament destiné au traitement de maladies qui sont provoquées par des helminthes et des ectoparasites, **caractérisée en ce qu'**on utilise des composés qui sont décrits dans les publications suivantes :
1) EP 807 629 (HOE 96/F119)
chromanes de formule I 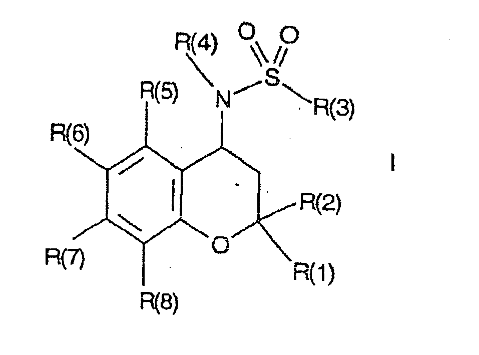 dans laquelle :
R(1) et R(2)
représentent, indépendamment l'un de l'autre, CₚF₂ₚ₊₁, un groupe alkyle ayant 1, 2, 3, 4, 5 ou 6 atomes de carbone ou phényle,
qui est non substitué ou porte 1 ou 2 substituants choisis dans l'ensemble constitué par F, Cl, Br, I, CF₃, les groupes méthyle, méthoxy, sulfamoyle, méthylsulfonylamino et méthylsulfonyle ;
p est 1, 2 ou 3 ;
ou
R(1) et R(2)
forment ensemble une chaîne alkylène ayant 2, 3, 4, 5, 6, 7, 8, 9 ou 10 atomes de carbone ;
R(3) représente R(9)-CₙH₂ₙ[NR(11)]ₘ- ;
R(9) représente un atome d'hydrogène ou un groupe cycloalkyle ayant 3, 4, 5, 6, 7 ou 8 atomes de carbone ;
n est zéro, 1, 2, 3, 4, 5, 6, 7, 8, 9 ou 10 ;
m est zéro ou 1 ;
R(11) représente un atome d'hydrogène ou un groupe alkyle ayant 1, 2, 3, 4, 5 ou 6 atomes de carbone ;
ou
R(11) forme conjointement avec R(9) un groupe alkylène ayant 1, 2, 3, 4, 5, 6, 7 ou 8 atomes de carbone ;
un groupe CH₂ du groupe CₙH₂ₙ pouvant être remplacé par -O-, -SO_{q}- ou -NR(10) ;
q étant zéro, 1 ou 2 ;
R(10) représentant un atome d'hydrogène, le groupe méthyle ou éthyle ;
R(4) représente R(12)-CᵣH₂ᵣ ;
(R12)
représente un atome d'hydrogène, un groupe cycloalkyle ayant 3, 4, 5, 6, 7 ou 8 atomes de carbone, pipéridyle, 1-pyrrolidinyle, N-morpholino, N-méthylpipérazino, CₚF₂ₚ₊₁, pyridyle, thiényle, imidazolyle ou phényle,
qui est non substitué ou porte 1 ou 2 substituants choisis dans l'ensemble constitué par F, Cl, Br, I, CF₃, les groupes méthyle, méthoxy, sulfamoyle, méthylsulfonylamino et méthylsulfonyle ;
r est 1, 2, 3, 4, 5, 6, 7, 8, 9, 10, 11, 12, 13, 14, 15, 16, 17, 18, 19 ou 20 ;
un groupe CH₂ du groupe CᵣH₂ᵣ pouvant être remplacé par -O-, -HC=CH-, -C=C-, -CO-, -CO-O-, -SO_{q}- ou -NR(10)- ;
q est zéro, 1 ou 2 ;
R(10) représente un atome d'hydrogène, le groupe méthyle ou éthyle ;
R(5), R(6), R(7) et R(8)
représentent, indépendamment les uns des autres, un atome d'hydrogène, F, Cl, Br, I, un groupe alkyle ayant 1, 2, 3 ou 4 atomes de carbone, un groupe cycloalkyle ayant 3, 4, 5, 6, 7 ou 8 atomes de carbone, -CN, -CF₃, -C₂F₅, -C₃F₇, -N₃, -NO₂, -CONR(13)R(14), -COOR(15),
R(16)-CₛH₂ₛ-Y- ou phényle,
qui est non substitué ou porte 1 ou 2 substituants choisis dans l'ensemble constitué par F, Cl, Br, I, CF₃, les groupes méthyle, méthoxy, sulfamoyle et méthylsulfonyle ;
R(13) et R(14)
représentent, indépendamment l'un de l'autre, un atome d'hydrogène ou un groupe alkyle ayant 1, 2 ou 3 atomes de carbone ;
R(15) représente un atome d'hydrogène, un groupe méthyle, éthyle, phényle ou -CᵤH₂ᵤ-NR(13)R(14) ; u est 2. ou 3 ;
R(16) représente un atome d'hydrogène, un groupe cycloalkyle ayant 3, 4, 5, 6, 7 ou 8 atomes de carbone, -COOR(15), thiényle, imidazolyle, pyridyle, quinolyle, isoquinolyle, pipéridyle, 1-pyrrolidinyle, N-morpholino, N-méthylpipérazino, CₜF₂ₜ₊₁ ou phényle,
qui est non substitué ou porte 1 ou 2 substituants choisis dans l'ensemble constitué par F, Cl, Br, I, CF₃, les groupes méthyle, méthoxy, sulfamoyle, méthylsulfonyle ;
s est zéro, 1, 2, 3, 4, 5 ou 6 ;
t est 1, 2 ou 3 ;
Y représente SOq, -CO-, -SO₂NR(10)-, -O-, -NR(10)- ou -CO-NR(10)- ;
R(6) ne pouvant pas être -OCF₃ ni -OC₂F₅ ;
et leurs sels physiologiquement acceptables;
ou
2) EP 847 996 (HOE 96/F341)
composés de formule I, 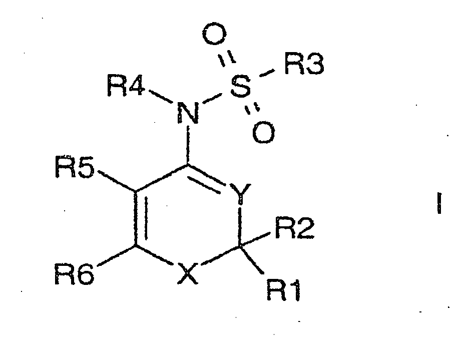 dans laquelle .
X représente -O-, -S-, -SO-, -SO₂-, -NR(7)-, - CR(8a)R(8b)- ou -CO- ;
R(7) représente un atome d'hydrogène ou -(CₐH₂ₐ)-R(9),
un groupe CH₂ du groupe CₐH₂ₐ pouvant être remplacé par -O-, -CH=CH-, -C≡C-, -CO-, -CO-O-, -O-CO-, -S-, -SO-, -SO₂-, -NR(10)- ou -CONR(10)-;
R(10) représente un atome d'hydrogène ou un groupe alkyle ayant 1, 2 ou 3 atomes de carbone ;
a est zéro, 1, 2, 3, 4, 5, 6, 7 ou 8 ;
R(9) représente un atome d'hydrogène, CF₃, C₂F₅, C₃F₇, un groupe cycloalkyle ayant 3, 4, 5, 6, 7 ou 8 atomes de carbone, diméthylamino, diéthylamino, 1-pipéridyle, 1-pyrrolidinyle, 4-morpholinyle, N-méthylpipérazin-1-yle, pyridyle, thiényle, imidazolyle ou phényle,
les groupes pyridyle, thiényle, imidazolyle et phényle étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
ou
R(7) et R(1)
forment ensemble une liaison;
R(8a) représente un atome d'hydrogène, CF₃, C₂F₅, C₃F₇, un groupe alkyle ayant 1, 2, 3, 4, 5 ou 6 atomes d'hydrogène ou phényle,
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(8b) représente un atome d'hydrogène, un groupe alkyle ayant 1, 2 ou 3 atomes de carbone, -OR(10), -COOR(10), COR(10) ;
R(10) représente un atome d'hydrogène ou un groupe alkyle ayant 1, 2 ou 3 atomes de carbone ;
ou
l'un des radicaux R(8a) et R(8b)
forment conjointement avec R(1) une liaison, lorsque Y a la signification de N ;
Y représente N ou CR(11) ;
R(11) représente un atome d'hydrogène ou un groupe alkyle ayant 1, 2 ou 3 atomes de carbone ;
R(1) et R(2)
représentent, indépendamment l'un de l'autre, un atome d'hydrogène, CF₃, C₂F₅, C₃F₇, F, Cl, un groupe méthoxy, alkyle ayant 1, 2, 3, 4, 5 ou 6 atomes de carbone ou phényle,
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
ou
R(1) et R(2)
forment ensemble une chaîne alkylène ayant 2, 3, 4, 5, 6, 7, 8, 9 ou 10 atomes de carbone ;
R(3) représente R(12)-CₙH₂ₙ-NR(13)- ou R(12)-CₙH₂ₙ-, un groupe CH₂ dans les groupes CₙH₂ₙ pouvant être remplacé par -O-, -CO-, -S-, -SO-, -SO₂- ou -NR(10a)- ;
R(10a)
représente un atome d'hydrogène, le groupe méthyle ou éthyle ;
R(12)
représente un atome d'hydrogène, un groupe méthyle, cycloalkyle ayant 3, 4, 5, 6, 7 ou 8 atomes de carbone, CF₃, C₂F₅ ou C₃F₇ ;
n est zéro, 1, 2, 3, 4, 5, 6, 7, 8, 9 ou 10 ;
R(13)
représente un atome d'hydrogène ou un groupe alkyle ayant 1, 2, 3, 4, 5 ou 6 atomes de carbone ;
ou
R(12) et R(13)
forment ensemble une liaison, lorsque n n'est pas inférieur à 3 ;
ou
R(3) et R(4) forment ensemble une chaîne alkylène ayant 3, 4, 5, 6, 7 ou 8 atomes de carbone, un groupe CH₂ de la chaîne alkylène pouvant être remplacé par -O-, -CO-, -S-, -SO-, -SO₂- ou -NR(10a)- ;
R(10a)
représente un atome d'hydrogène, un groupe méthyle ou éthyle ;
R(4) représente R(14)-CᵣH₂ᵣ,
un groupe CH₂ du groupe CᵣH₂ᵣ pouvant être remplacé par -O-, -CH=CH-, -C≡C-, -CO-, -CO-O-, -O-CO-, -S-, -SO-, -SO₂-, -NR(10b)- ou -CONR(10b)- ;
R(10b)
représente un atome d'hydrogène ou un groupe alkyle ayant 1, 2 ou 3 atomes de carbone ;
R(14)
représente un groupe méthyle, CF₃, C₂F₅, C₃F₇, cycloalkyle ayant 3, 4, 5, 6, 7 ou 8 atomes de carbone, -OH, -COOH, -NR(23)R(24), 1-pipéridyle, 1-pyrrolidinyle, 4-morpholinyle, 4-méthylpipérazin-1-yle, pyridyle, thiényle, imidazolyle ou phényle, les groupes pyridyle, thiényle, imidazolyle et phényle étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(23) et R(24)
représentent, indépendamment l'un de l'autre, un atome d'hydrogène ou un groupe alkyle ayant 1, 2 ou 3 atomes de carbone ;
ou
R(23) et R(24)
forment ensemble une chaîne de 4 ou 5 groupes méthylène, dont un groupe CH₂ peut être remplacé par -O-, -S-, -NH-, -N(CH₃)- ou -N(benzyle)- ;
r est zéro, 1, 2, 3, 4, 5, 6, 7, 8, 9, 10, 11, 12, 13, 14, 15, 16, 17, 18, 19 ou 20 ;
R(5) et R(6)
forment ensemble un groupe
-CR(15)=CR(16)-CR(17)=CR(18)-,
-CR(15)=CR(16)-CR(17)=N-,
-CR(15)=CR(16)-N=CR(18)-,
-CR(15)=N-CR(17)=N-,
-CR(15)=N-N=CR(18)-,
-N=CR(16)=CR(17)=N- ou
-S-CR(15)=CR(16)-,
les deux sens de liaison étant chaque fois possibles ;
R(15), R(16), R(17) et R(18)
représentent, indépendamment les uns des autres, un atome d'hydrogène, F, Cl, Br, I, un groupe alkyle ayant 1, 2, 3, 4 ou 5 atomes de carbone, cycloalkyle ayant 3, 4, 5, 6, 7 ou 8 atomes de carbone, -CN, -CF₃, -C₂F₅, -C₃F₇, -N₃, -NO₂, -Z-CₛH₂ₛ-R(22), thiényle ou phényle,
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
Z représente -O-, -CO-, -CO-O-, -O-CO-, -S-, -SO-, -SO₂-, -SO₂-O-, -SO₂NR(10c), -NR(10c)- ou -CONR(10c)- ;
R(10c) représente un atome d'hydrogène ou un groupe alkyle ayant 1, 2 ou 3 atomes de carbone;
s est zéro, 1, 2, 3, 4, 5 ou 6 ;
R(22) représente un atome d'hydrogène, CF₃, C₂F₅, C₃F₇, un groupe cycloalkyle ayant 3, 4, 5, 6, 7 ou 8 atomes de carbone, NR(19)R(20), -COOR(21), 1-pipéridyle, 1-pyrrolidinyle, 4-morpholinyle, 4-méthylpipérazin-1-yle, pyridyle, thiényle, imidazolyle, quinolyle, isoquinolyle ou phényle,
les groupes pyridyle, thiényle, imidazolyle quinolyle, isoquinolyle et phényle étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(19) et R(20)
représentent, indépendamment l'un de l'autre, un atome d'hydrogène ou un groupe alkyle ayant 1, 2 ou 3 atomes de carbone ;
ou
R(19) et R(20)
forment ensemble une chaîne de 4 ou 5 groupes méthylène, dont un groupe CH₂ peut être remplacé par -O-, -S-, -NH-, -N(CH₃)- ou -N(benzyle)- ;
R(21) représente un atome d'hydrogène ou un groupe alkyle ayant 1, 2 ou 3 atomes de carbone ;
Y et X ne pouvant toutefois pas être simultanément, respectivement, CR(11) et O.
ou
3) EP 857 724 (HOE 97/F024)
composés de formule I 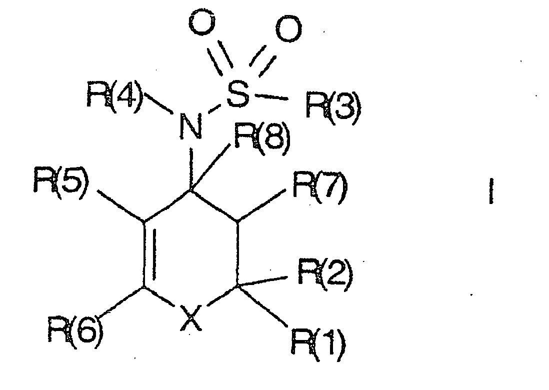 dans laquelle :
X représente -[S(O)_{zéro, 1 ou 2}]-, -NR(9)-, -[CR(9)R(23)]- ou -CO-;
R(9) représente un atome d'hydrogène ou -(CₙH₂ₙ)-R(10) ;
n est zéro, 1, 2, 3, 4, 5, 6, 7 ou 8 ;
R(10) représente un atome d'hydrogène, un groupe cycloalkyle ayant 3, 4, 5, 6, 7 ou 8 atomes de carbone, pipéridyle, 1-pyrrolidinyle, N-morpholino, N-méthylpipérazino, CF₃, C₂F₅, C₃F₇ ;
un groupe CH₂ du groupe CₙH₂ₙ pouvant être remplacé par -O-, -CH=CH-, -C≡C-, -CO-, -CO-O-, -[SO_{zéro, 1 ou 2}]- ou -NR(11)- ;
R(11) représente un atome d'hydrogène, le groupe méthyle ou éthyle ;
ou
R(10) représente un groupe pyridyle, thiényle, imidazolyle ou phényle,
qui est non substitué ou porte 1 ou 2 substituants choisis dans l'ensemble constitué par F, Cl, Br, I, CF₃, les groupes méthyle, méthoxy, sulfamoyle, méthylsulfonyle et méthylsulfonylamino ;
ou
R(9) forme, conjointement avec R(1), une liaison,
R(23) représente un atome d'hydrogène, un groupe alkyle ayant 1, 2 ou 3 atomes de carbone, OH, O-alkyle ayant 1, 2 ou 3 atomes de carbone, COOH, COO-alkyle ayant 1, 2 ou 3 atomes de carbone ou -CO-R(24) ;
R(24) représente un atome d'hydrogène, le groupe méthyle ou éthyle ;
R(1) et R(2)
représentent, indépendamment l'un de l'autre, un atome d'hydrogène, CF₃, C₂F₅, C₃F₇, un groupe alkyle ayant 1, 2, 3, 4, 5 ou 6 atomes de carbone ou phényle,
qui est non substitué ou porte 1 ou 2 substituants choisis dans l'ensemble constitué par F, Cl, Br, I, CF₃, les groupes méthyle, méthoxy, sulfamoyle et méthylsulfonyle ;
ou
R(1) et R(2)
forment ensemble une chaîne alkylène ayant 2, 3, 4, 5, 6, 7, 8, 9 ou 10 atomes de carbone ;
R(3) représente R(12)-CₐH₂ₐ[NR(13)]ₘ- ;
R(12) représente un atome d'hydrogène ou un groupe cycloalkyle ayant 3, 4, 5, 6, 7 ou 8 atomes de carbone, CF₃, C₂F₅ ou C₃F₇ ;
a est zéro, 1, 2, 3, 4, 5, 6, 7, 8, 9 ou 10 ;
m est zéro ou 1 ;
R(13) représente un atome d'hydrogène ou un groupe alkyle ayant 1, 2, 3, 4, 5 ou 6 atomes de carbone
ou
R(12) et R(13)
forment ensemble un groupe alkylène ayant 4, 5, 6, 7 ou 8 atomes de carbone, un groupe CH₂ du groupe alkylène pouvant être remplacé par -O-, -[SO_{zéro, 1 ou 2}]-, -CO- ou -NR (11) ;
R(11) représente un atome d'hydrogène, le groupe méthyle ou éthyle ;
R(4) représente R(14)-CᵣH₂ᵣ ;
r est 1, 2, 3, 4, 5, 6, 7, 8, 9, 10, 11, 12, 13, 14, 15, 16, 17, 18, 19 ou 20 ;
R(14) représente un atome d'hydrogène, un groupe cycloalkyle ayant 3, 4, 5, 6, 7 ou 8 atomes de carbone, pipéridyle, 1-pyrrolidinyle, N-morpholino, N-méthylpipérazino, CF₃, C₂F₅, C₃F₇, pyridyle, thiényle, imidazolyle ou phényle,
qui est non substitué ou porte 1 ou 2 substituants choisis dans l'ensemble constitué par F, Cl, Br, I, CF₃, les groupes méthyle, méthoxy, sulfamoyle, méthylsulfonyle et méthylsulfonylamino ;
un groupe CH₂ du groupe CᵣH₂ᵣ pouvant être remplacé par -O-, -CH=CH-, -C≡C-, -CO-, -CO-O-, -CO-NR(11)-, - [SO_{zéro, 1 ou 2}]- ou -NR(11)- ;
ou
R(3) et R(4)
forment ensemble une chaîne alkylène ayant 3, 4, 5, 6, 7 ou 8 atomes de carbone, un groupe CH₂ de la chaîne alkylène pouvant être remplacé par -O-, -[SO_{zéro, 1 ou 2}]-, -CO- ou -NR (11) ;
R(5) et R(6)
forment ensemble un groupe
-CR(15)=CR(16)-CR(17)=CR(18)-,
-CR(15)=CR(16)-CR(17)=N-,
-CR(15)=CR(16)-N=CR(18)-,
-CR(15)=N-CR(17)=N-, -CR(15)=N-N=CR(18)-,
-N=CR(16)-CR(17)=N- ou -S-CR(15)=CR(16)-,
R(15), R(16), R(17) et R(18)
représentent, indépendamment les uns des autres, un atome d'hydrogène, F, Cl, Br, I, un groupe alkyle ayant 1, 2, 3 ou 4 atomes de carbone, cycloalkyle ayant 3, 4, 5, 6, 7 ou 8 atomes de carbone, CN, CF₃, C₂F₅, C₃F₇, N₃, NO₂, -CONR(19)R(20), -COOR(21), R(22)-CₛH₂ₛ-Zou phényle,
qui est non substitué ou porte 1 ou 2 substituants choisis dans l'ensemble constitué par F, Cl, Br, I, CF₃, les groupes méthyle, méthoxy, sulfamoyle, et méthylsulfonyle ;
R(19) et R(20)
représentent, indépendamment l'un de l'autre, un atome d'hydrogène ou un groupe alkyle ayant 1, 2 ou 3 atomes de carbone ;
R(21) représente un atome d'hydrogène, le groupe méthyle, éthyle, phényle ou -CᵤH₂ᵤ-NR(19)R(20) ;
u est 2 ou 3 ;
le groupe phényle étant non substitué ou portant 1 ou 2 substituants choisis dans l'ensemble constitué par F, Cl, Br, I, CF₃, les groupes méthyle, méthoxy, sulfamoyle, et méthylsulfonyle ;
R(22) représente un atome d'hydrogène, un groupe cycloalkyle ayant 3, 4, 5, 6, 7 ou 8 atomes de carbone, -COOR(21), thiényle, imidazolyle, pyridyle, quinolyle, isoquinolyle, pipéridyle, 1-pyrrolidinyle, N-morpholino, N-méthylpipérazino, CF₃, C₂F₅ ou C₃F₇ ou phényle,
qui est non substitué ou porte 1 ou 2 substituants choisis dans l'ensemble constitué par F, Cl, Br, I, CF₃, les groupes méthyle, méthoxy, sulfamoyle et méthylsulfonyle ;
s est zéro, 1, 2, 3, 4, 5 ou 6 ;
Z représente -[S(O)_{zéro}, _{1 ou 2}]-, -CO-, -SO₂-NR(11)-, -SO₂-O-, -O-, -NR(11)- ou -[CO-NR(11)]- ;
R(7) représente un atome d'hydrogène, un groupe hydroxy, alcoxy ayant 1, 2, 3 ou 4 atomes de carbone, acyloxy ayant 1, 2, 3 ou 4 atomes de carbone, Cl, Br, F, un groupe alkyle ayant 1, 2, 3 ou 4 atomes de carbone ;
R(8) représente un atome d'hydrogène ou un groupe alkyle ayant 1, 2, 3, 4, 5 ou 6 atomes de carbone ;
et leurs sels physiologiquement acceptables ;
ou
4) EP 860 440 (HOE 97/F033)
dérivés de chromane de formule I 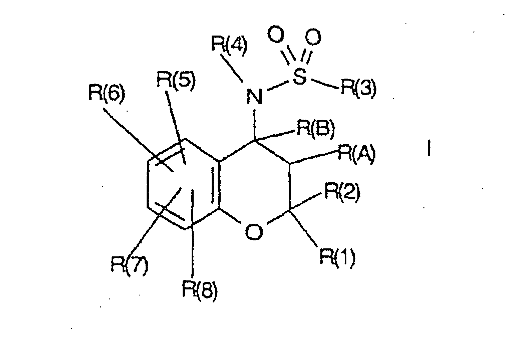 dans laquelle :
R(1) et R(2)
représentent, indépendamment l'un de l'autre, un atome d'hydrogène, CF₃, C₂F₅, C₃F₇ un groupe alkyle ayant 1, 2, 3, 4, 5 ou 6 atomes de carbone ou phényle,
qui est non substitué ou porte 1 ou 2 substituants choisis dans l'ensemble constitué par F, Cl, Br, I, CF₃, les groupes méthyle, méthoxy, sulfamoyle et méthylsulfonyle ;
ou
R(1) et R(2)
forment ensemble une chaîne alkylène ayant 2, 3, 4, 5, 6, 7, 8, 9 ou 10 atomes de carbone ;
R(A) représente un groupe hydroxy, alcanoyloxy ayant 1, 2, 3, 4, 5 ou 6 atomes de carbone ou alkylsulfonyloxy ayant 1, 2, 3, 4, 5 ou 6 atomes de carbone ;
R(B) représente un atome d'hydrogène ;
ou
R(A) et R(B)
forment ensemble une liaison ;
R(3) représente R(9)-CₙH₂ₙ[NR(11)]ₘ- ;
R(9) représente un atome d'hydrogène ou un groupe cycloalkyle ayant 3, 4, 5, 6, 7 ou 8 atomes de carbone ;
n est zéro, 1, 2, 3, 4, 5, 6, 7, 8, 9 ou 10 ;
m est zéro ou 1 ;
R(11) représente un atome d'hydrogène ou un groupe alkyle ayant 1, 2, 3, 4, 5 ou 6 atomes de carbone ;
ou
R(11) et R(9)
forment ensemble un groupe alkylène ayant 1, 2, 3, 4, 5, 6, 7 ou 8 atomes de carbone ;
un groupe CH₂ du groupe CₙH₂ₙ pouvant être remplacé par -O-, -SO_{zéro, 1 ou 2}- ou -NR(10);
R(10) représentant un atome d'hydrogène,
le groupe méthyle ou éthyle ;
R(4) représente R(12)-CᵣH₂ᵣ ;
(R12) représente un atome d'hydrogène, un groupe cycloalkyle ayant 3, 4, 5, 6, 7 ou 8 atomes de carbone, pipéridyle, 1-pyrrolidinyle, N-morpholino, N-méthylpipérazino, CF₃, C₂F₅, C₃F₇, pyridyle, thiényle, imidazolyle ou phényle,
les groupes pyridyle, thiényle, imidazolyle et phényle étant non substitués ou portant 1 ou 2 substituants choisis dans l'ensemble constitué par F, Cl, Br, I, CF₃, les groupes méthyle, méthoxy, sulfamoyle, méthylsulfonyle et méthylsulfonylamino ;
r est zéro, 1, 2, 3, 4, 5, 6, 7, 8, 9, 10, 11, 12, 13, 14, 15, 16, 17, 18, 19 ou 20 ;
un groupe CH₂ du groupe CᵣH₂ᵣ pouvant être remplacé par -O-, -CH=CH-, -C≡C-, -CO-, -CO-O-, -SO_{zéro, 1 ou 2}- ou -NR(10)-;
R(5), R(6), R(7) et R(8)
représentent, indépendamment les uns des autres, un atome d'hydrogène, F, Cl, Br, I, un groupe alkyle ayant 1, 2, 3, 4, 5 ou 6 atomes de carbone, un groupe cycloalkyle ayant 3, 4, 5, 6, 7 ou 8 atomes de carbone, -CN, -CF₃, -C₂F₅, -C₃F₇, -N₃, -NO₂, -CONR(13)R(14), -COOR(15), R(16)-CₛH₂ₛ-Y- ou phényle,
qui est non substitué ou porte 1 ou 2 substituants choisis dans l'ensemble constitué par F, Cl, Br, I, CF₃, les groupes méthyle, méthoxy, sulfamoyle et méthylsulfonyle ;
R(13) et R(14)
représentent, indépendamment l'un de l'autre, un atome d'hydrogène ou un groupe alkyle ayant 1, 2 ou 3 atomes de carbone ;
R(15) représente un atome d'hydrogène, un groupe méthyle, éthyle, phényle ou -CᵤH₂ᵤ-NR(13)R(14) ;
u est 2 ou 3 ;
R(16) représente un atome d'hydrogène, un groupe cycloalkyle ayant 3, 4, 5, 6, 7 ou 8 atomes de carbone, -COOR(15), thiényle, imidazolyle, pyridyle, quinolyle, isoquinolyle, pipéridyle, 1-pyrrolidinyle, N-morpholino, N-méthylpipérazino, CF₃, C₂F₅ ou C₃F₇ ou phényle,
qui est non substitué ou porte 1 ou 2 substituants choisis dans l'ensemble constitué par F, Cl, Br, I, CF₃, les groupes méthyle, méthoxy, sulfamoyle et méthylsulfonyle ;
s est zéro, 1, 2, 3, 4, 5 ou 6 ;
Y représente -S-, -SO-, SO₂-, -CO-, -SO₂NR(10)-, -O-, -NR(10)- ou -CO-NR(10)- ;
mais avec la condition que deux des substituants R(5), R(6), R(7) et R(8) ne sont pas des atomes d'hydrogène ;
et leurs sels physiologiquement acceptables ;
ou
5) EP 861 836 (HOE 97/F038)
composés de formule I, 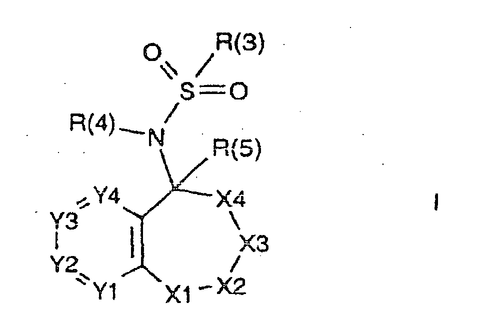 dans laquelle :
X1 représente -O-, -S-, -SO-, -SO₂-, -CR(1)R(2)-, -NR(6)-, -CO- ou -CR(1)R(7)-
ou ;
R(1) et R(2)
représentent, indépendamment l'un de l'autre, un atome d'hydrogène, CF₃, C₂F₅, C₃F₇ un groupe alkyle ayant 1, 2, 3, 4, 5 ou 6 atomes de carbone ou phényle,
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
ou
R(1) et R(2)
forment ensemble une chaîne alkylène ayant 2, 3, 4, 5, 6, 7, 8, 9 ou 10 atomes de carbone ;
R(6) représente un atome d'hydrogène ou -(CₙH₂ₙ)-R(8),
un groupe CH₂ du groupe CₙH₂ₐ pouvant être remplacé par -O-, -CH=CH-, -C≡C-, -CO-, -CO-O-, -O-CO-, -S-, -SO-, -SO₂-, -NR(9)- ou -CONR(9)- ;
R(9) représente un atome d'hydrogène ou un groupe alkyle ayant 1, 2, ou 3 atomes de carbone ;
n est zéro, 1, 2, 3, 4, 5, 6, 7 ou 8 ;
R(8) représente un atome d'hydrogène, CF₃, C₂F₅, C₃F₇, un groupe cycloalkyle ayant 3, 4, 5, 6, 7 ou 8 atomes de carbone, diméthylamino, diéthylamino, 1-pipéridyle, 1-pyrrolidinyle, 4-morpholinyle, 4-méthylpipérazin-1-yle, pyridyle, thiényle, imidazolyle ou phényle,
les groupes pyridyle, thiényle, imidazolyle et phényle étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
X2 représente -CR(1)R(2)- ou -CR(2)R(10)- ;
ou
X2 représente, lorsque X3 et X1 représentent -CR(1)R(2)-, également -O-, -S-, -SO-, -SO₂- ou -NR(6)-,
les radicaux R(1), R(2) et R(6)
étant tels que définis pour X1, mais indépendamment des significations des radicaux dans X1 ;
R(10) forme, conjointement avec R(7), une liaison ;
X3 représente -CR(1)R(2)- ;
ou
X3 représente, lorsque X2 et X4 représentent -CR(1)R(2)-, également -O-, -S-, -SO-, -SO₂- ou -NR(6)-,
les radicaux R(1), R(2) et R(6)
étant tels que définis pour X1, mais indépendamment des significations des radicaux dans X1 ;
X4 représente -CR(1)R(2)-, -NR(6)-, -NR(11), -CH(OR(30))- ou -CR(2)R(11)-,
les radicaux R(1), R(2) et R(6)
étant tels que définis pour X1, mais indépendamment des significations des radicaux dans X1 ;
R(30) représentant un atome d'hydrogène, un groupe alkyle ayant 1, 2 ou 3 atomes de carbone ou acyle ayant 1, 2, 3 ou 4 atomes de carbone;
R(11) forme, conjointement avec R(5), une liaison;
Y1, Y2, Y3 et Y4
représentent, indépendamment les uns des autres, -CR(12)- ou N,
au maximum 2 des groupes Y1, Y2, Y3 et Y4 pouvant représenter simultanément N ;
les radicaux R(12)
représentent, chacun indépendamment, un atome d'hydrogène, F, Cl, Br, I, un groupe alkyle ayant 1, 2, 3, 4 ou 5 atomes de carbone, cycloalkyle ayant 3, 4, 5, 6, 7 ou 8 atomes de carbone, CN, CF₃, C₂F₅, C₃F₇, N₃, NO₂, -Z-CₘH₂ₘ- R(13) ou phényle,
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
Z représente -O-, -CO-, -CO-O-, -O-CO-, -S-, -SO-, -SO₂-, -SO₂NR(14), -NR(14)- ou -CONR(14)-;
R(14) représente un atome d'hydrogène ou un groupe alkyle ayant 1, 2 ou 3 atomes de carbone ;
m est zéro, 1, 2, 3, 4, 5 ou 6 ;
R(13) représente un atome d'hydrogène, CF₃, C₂F₅, C₃F₇, un groupe cycloalkyle ayant 3, 4, 5, 6, 7 ou 8 atomes de carbone, -NR(15)R(16), -CONR(15)R(16), -OR(30a), phényle, thiényle ou un hétérocycle azoté ayant 1, 2, 3, 4, 5, 6, 7, 8 ou 9 atomes de carbone, les groupes phényle, thiényle et l'hétérocycle azoté étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(15) et R(16) représentent, indépendamment l'un de l'autre, un atome d'hydrogène ou un groupe alkyle ayant 1, 2 ou 3 atomes de carbone ;
ou
R(15) et R(16)
forment ensemble une chaîne de 4 ou 5 groupes méthylène, dont un groupe CH₂ peut être remplacé par -O-, -S-, -NH-, -N(CH₃)- ou -N(benzyle)- ;
R(30a)
représente un atome d'hydrogène, un groupe alkyle ayant 1, 2 ou 3 atomes de carbone ou acyle ayant 1, 2, 3 ou 4 atomes de carbone ;
ou
Y1 et Y2
représentent ensemble un atome de soufre et Y3 et Y4 représentent chacun -CR(12)- ;
les radicaux R(12)
sont définis, indépendamment l'un de l'autre, comme dans Y1, Y2, Y3, Y4 ;
R(3) représente R(17)-CₓH₂ₓ-NR(18)- ou R(17)-CₓH₂ₓ-,
un groupe CH₂ dans les groupes CₓH₂ₓ pouvant être remplacé par -O-, -CO-, -S-, SO-, -SO₂- ou -NR(19)- ;
R(19) représente un atome d'hydrogène, le groupe méthyle ou éthyle ;
R(17) représente un atome d'hydrogène, un groupe méthyle, cycloalkyle ayant 3, 4, 5, 6, 7 ou 8 atomes de carbone, CF₃, C₂F₅ ou C₃F₇,
x est zéro, 1, 2, 3, 4, 5, 6, 7, 8, 9 ou 10 ;
R(18) représente un atome d'hydrogène ou un groupe alkyle ayant 1, 2, 3, 4, 5, 6, 7
ou 8 atomes de carbone ;
ou
R(18) et R(17)
forment ensemble une liaison, lorsque x n'est pas inférieur à 3 ;
ou
R(3) représente un groupe phényle
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
ou
R(3) forme conjointement avec R(4)
une chaîne alkylène ayant 3, 4, 5, 6, 7 ou 8 atomes de carbone,
un groupe CH₂ de la chaîne alkylène pouvant être remplacé par -O-, -CO-, -S-, -SO- ou -SO₂- ;
R(4) représente -CᵣH₂ᵣ-R(20)-,
un groupe CH₂ du groupe CᵣH₂ᵣ pouvant être remplacé par -O-, -CH=CH-, -C=C-, -CO-, -CO-O-, -O-CO-, -S-, -SO-, -SO₂-, -NR(21)- ou -CONR(21)- ;
R(21) représente un atome d'hydrogène ou un groupe alkyle ayant 1, 2 ou 3 atomes de carbone ;
r est zéro, 1, 2, 3, 4, 5, 6, 7, 8, 9, 10, 11, 12, 13, 14, 15, 16, 17, 18, 19 ou 20 ;
R(20) représente un atome d'hydrogène, un groupe méthyle, CF₃, C₂F₅, C₃F₇, cycloalkyle ayant 3, 4, 5, 6, 7 ou 8 atomes de carbone, -NR(22)R(23), phényle, thiényle ou un hétérocycle azoté ayant 1, 2, 3, 4, 5, 6, 7, 8 ou 9 atomes de carbone,
les groupes phényle, thiényle et l'hétérocycle azoté étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(22) et R(23) représentent, indépendamment l'un de l'autre, un atome d'hydrogène ou un groupe alkyle ayant 1, 2 ou 3 atomes de carbone;
ou
R(22) et R(23)
forment ensemble une chaîne de 4 ou 5 groupes méthylène,
dont un groupe CH₂ peut être remplacé par -O-, -S-, -NH-, -N(CH₃)- ou -N(benzyle)- ;
R(5) représente un atome d'hydrogène ou forme conjointement avec R(11) une liaison ;
sous toutes leurs formes stéréoisomères et mélanges de celles-ci en rapports quelconques, ainsi que leurs sels physiologiquement acceptables ;
ou
6) HMR 97/L206 - EP 895 994
composés de formule I 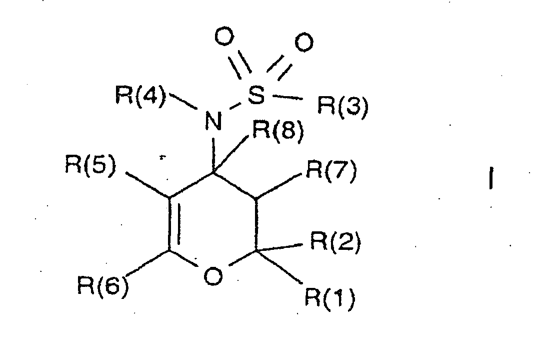 dans laquelle :
R(1) et R(2)
représentent, indépendamment l'un de l'autre, un atome d'hydrogène, CF₃, C₂F₅, C₃F₇ un groupe alkyle ayant 1, 2, 3, 4, 5 ou 6 atomes de carbone ou phényle,
qui est non substitué ou porte 1 ou 2 substituants choisis dans l'ensemble constitué par F, Cl, Br, I, CF₃, les groupes méthyle, méthoxy, sulfamoyle et méthylsulfonyle ;
ou
R(1) et R(2)
forment ensemble une chaîne alkylène ayant 2, 3, 4, 5, 6, 7, 8, 9 ou 10 atomes de carbone ;
un groupe CH₂ de la chaîne alkylène pouvant être remplacé par -O-, -CO-, -S-, SO-, -SO₂- ou -NR(10)- ;
R(10) représente un atome d'hydrogène ou un groupe alkyle ayant 1, 2 ou 3 atomes de carbone ;
R(3) représente R(12)-CₐH₂ₐ[NR(13)]ₘ-,
R(12) représente un atome d'hydrogène ou un groupe cycloalkyle ayant 3, 4, 5, 6, 7 ou 8 atomes de carbone, CF₃, C₂F₅ ou C₃F₇,
a est zéro, 1, 2, 3, 4, 5, 6, 7, 8, 9 ou 10 ;
m est zéro ou 1 ;
R(13) représente un atome d'hydrogène ou un groupe alkyle ayant 1, 2, 3, 4, 5 ou 6 atomes de carbone ;
ou
R(12) et R(13)
forment ensemble un groupe alkylène ayant 4, 5, 6, 7 ou 8 atomes de carbone ;
un groupe CH₂ du groupe alkylène pouvant être remplacé par -O-, -[SO_{zéro, 1 ou 2}]-, -CO- ou -NR(10)- ;
R(10) représentant un atome d'hydrogène ou un groupe alkyle ayant 1, 2 ou 3 atomes de carbone ;
R(4) représente R(14)-CᵣH₂ᵣ ;
r est zéro, 1, 2, 3, 4, 5, 6, 7, 8, 9, 10, 11, 12, 13, 14, 15, 16, 17, 18, 19 ou 20 ;
(R14) représente un atome d'hydrogène, un groupe cycloalkyle ayant 3, 4, 5, 6, 7 ou 8 atomes de carbone, pipéridyle, 1-pyrrolidinyle, N-morpholino, N-méthylpipérazino, CF₃, C₂F₅, C₃F₇, pyridyle, thiényle, imidazolyle ou phényle,
qui est non substitué ou porte 1 ou 2 substituants choisis dans l'ensemble constitué par F, Cl, Br, I, CF₃, les groupes méthyle, méthoxy, sulfamoyle, méthylsulfonyle et méthylsulfonylamino ;
un groupe CH₂ du groupe CᵣH₂ᵣ pouvant être remplacé par -O-, -CH=CH-, -C≡C-, -CO-, -CO-O- , -CO-NR(11)-, - [SO_{zéro}, _{1 ou 2}]- ou -NR(11)- ;
R(11) représente un atome d'hydrogène ou un groupe - (CₐH₂ₐ) -R (10) ;
un groupe CH₂ du groupe CₐH₂ₐ pouvant être remplacé par -O-, -CH=CH-, -C≡C-, -CO-, -CO-O-, -O-CO-, -S-, -SO-, -SO₂-, -NR(10)- ou -CONR(10)- ;
R(10) représente un atome d'hydrogène ou un groupe alkyle ayant 1, 2 ou 3 atomes de carbone ;
ou
R(3) et R(4)
forment ensemble une chaîne alkylène ayant 3, 4, 5, 6, 7 ou 8 atomes de carbone, un groupe CH₂ de la chaîne alkylène pouvant être remplacé par -O-, -[SO_{zéro, 1 ou 2}]-, -CO- ou -NR(11)- ;
R(11) représente un atome d'hydrogène ou un groupe -(CₐH₂ₐ)-R(10) ;
un groupe CH₂ du groupe CₐH₂ₐ pouvant être remplacé par -O-, -CH=CH-, -C≡C-, -CO-, -CO-O-, -O-CO-, -S-, -SO-, -SO₂-, -NR(10)- ou -CONR(10)- ;
R(10) représente un atome d'hydrogène ou un groupe alkyle ayant 1, 2 ou 3 atomes de carbone;
R(5) et R(6)
forment ensemble un groupe
-CR(15)=CR(16)-CR(17)=N-,
-CR(15)=CR(16)-N=CR(17)-,
-CR(15)=N-CR(17)=N-,
-CR(15)=N-N=CR(17)-
-N=CR(16)-CR(17)=N- ou
-S-CR(15)=CR(16)- ;
R(15), R(16) et R(17)
représentent, chacun indépendamment, un atome d'hydrogène, F, Cl, Br, I, un groupe alkyle ayant 1, 2, 3 ou 4 atomes de carbone, cycloalkyle ayant 3, 4, 5, 6, 7 ou 8 atomes de carbone, -CN, -CF₃, -C₂F₅, -C₃F₇, -N₃, -NO₂, -CONR(19)R(21), -COOR(21), R(22)-CₛH₂ₛ-Z- ou phényle,
qui est non substitué ou porte 1 ou 2 substituants choisis dans l'ensemble constitué par F, Cl, Br, I, CF₃, les groupes méthyle, méthoxy, sulfamoyle, et méthylsulfonyle ;
R(19) représente un atome d'hydrogène ou un groupe alkyle ayant 1, 2 ou 3 atomes de carbone ;
R(21) représente un atome d'hydrogène, le groupe méthyle, éthyle, phényle
ou -CᵤH₂ᵤ-NR(19)R(20) ;
le groupe phényle étant non substitué ou portant 1 ou 2 substituants choisis dans l'ensemble constitué par F, Cl, Br, I, CF₃, les groupes méthyle, méthoxy, sulfamoyle, et méthylsulfonyle ;
R(20) représente un atome d'hydrogène ou un groupe alkyle ayant 1, 2 ou 3 atomes de carbone ;
u est 2 ou 3 ;
R(22) représente un atome d'hydrogène, un groupe cycloalkyle ayant 3, 4, 5, 6, 7 ou 8 atomes de carbone, -COOR(21), CONR(19)R(21), thiényle, imidazolyle, pyridyle, quinolyle, isoquinolyle, pipéridyle, 1-pyrrolidinyle, N-morpholino, N-méthylpipérazino, CF₃, C₂F₅, C₃F₇ ou phényle,
qui est non substitué ou porte 1 ou 2 substituants choisis dans l'ensemble constitué par F, Cl, Br, I, CF₃, les groupes méthyle, méthoxy, sulfamoyle et méthylsulfonyle ;
s est zéro, 1, 2, 3, 4, 5 ou 6 ;
Z représente -[S (O) _{zéro, 1 ou 2}]-, -CO-, -SO₍₀, _{1 ou 2})-NR(11)-, -SO₂-O-, -O-, -NR(11)- ou -[CO-NR(11)]- ;
R(7) représente un atome d'hydrogène, un groupe hydroxy, alcoxy ayant 1, 2, 3 ou 4 atomes de carbone, acyloxy ayant 1, 2, 3 ou 4 atomes de carbone, Cl, Br, F, un groupe alkyle ayant 1, 2, 3 ou 4 atomes de carbone ;
R(8) représente un atome d'hydrogène ou un groupe alkyle ayant 1, 2, 3, 4, 5 ou 6 atomes de carbone ;
et leurs sels physiologiquement acceptables ;
ou
7) HMR 97/L223 - EP-A-905 131
composés de formule I 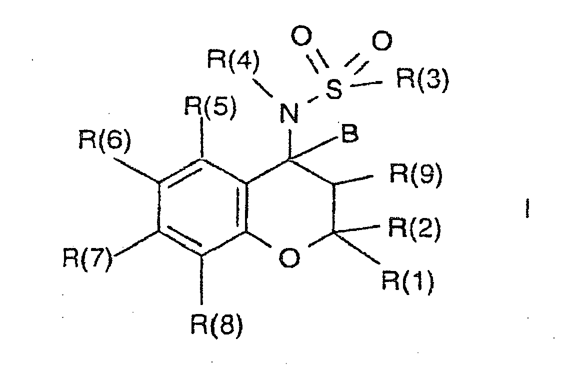 dans laquelle :
R(1) et R(2)
représentent, indépendamment l'un de l'autre, un atome d'hydrogène, CF₃, C₂F₅, C₃F₇ un groupe alkyle ayant 1, 2, 3, 4, 5 ou 6 atomes de carbone ou phényle,
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
ou
R(1) et R(2)
forment ensemble une chaîne alkylène ayant 2, 3, 4, 5, 6, 7, 8, 9 ou 10 atomes de carbone ;
R(3) représente R(10)-CₙH₂ₙ-NR(11)- ou R(10)-CₙH₂ₙ-, un groupe CH₂ dans les groupes CₙH₂ₙ pouvant être remplacé par -O-, -CO-, -S-, SO-, -SO₂- ou -NR(12a)- ;
R(12a) représente un atome d'hydrogène ou un groupe méthyle ou éthyle ;
R(10) représente un atome d'hydrogène ou un groupe méthyle, cycloalkyle ayant 3, 4, 5, 6, 7 ou 8 atomes de carbone, CF₃, C₂F₅ ou C₃F₇,
n est zéro, 1, 2, 3, 4, 5, 6, 7, 8, 9 ou 10 ;
R(11) représente un atome d'hydrogène ou un groupe alkyle ayant 1, 2, 3, 4, 5 ou 6 atomes de carbone ;
ou
R(10) et R(11)
forment ensemble une liaison, lorsque n n'est pas inférieur à 3 ;
R(4) représente R(13)-CᵣH₂ᵣ-Z-C_{q}H_{2q}-;
q est 0, 1, 2, 3, 4, 5, 6, 7 ou 8 ;
r est 0, 1, 2, 3, 4, 5, 6, 7 ou 8 ;
Z représente
-CO-NR(14)-,
-OCO-NR(14)-,
-O-CₓH₂ₓ-O-,
-O-CₓH₂ₓ-NR (14)- ,
-O-CₓH₂ₓ-CO-O-,
-CO-O-CₓH₂ₓ-O- ou
-CO-O-CₓH₂ₓ-NR (14) -
les deux sens de liaison étant chaque fois possibles ;
x est 2, 3 ou 4 ;
R(14)représente un atome d'hydrogène, un groupe alkyle ayant 1, 2 ou 3 atomes de carbone, -C_{y}H_{2y}-OR(12b), -C_{y}H_{2y}-NR(12b)₂ ;
R(12b) représente un atome d'hydrogène, le groupe méthyle ou éthyle ;
y est 2 ou 3 ;
R(13) représente H, CF₃, C₂F₅, C₃F₇, un groupe cycloalkyle ayant 3, 4, 5, 6, 7 ou 8 atomes de carbone, -NR(15)R(16), -CONR(15)R(16), -C(=NR(17))NR(15)R(16), -OR(17), -COOR(17), phényle ou un hétérocycle azoté ayant 1, 2, 3, 4, 5, 6, 7, 8 ou 9 atomes de carbone,
le groupe phényle et l'hétérocycle azoté étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle, aminosulfonyle et méthylsulfonylamino ;
R(15) et R(16)
Représentent, indépendamment l'un de l'autre, un atome d'hydrogène, un groupe alkyle ayant 1, 2, 3 ou 4 atomes de carbone ou -C_{z}H_{2z}-phényle,
où le groupe phényle est non substitué ou porte 1 ou 2 substituants choisis dans l'ensemble constitué par F, Cl, Br, CF₃, NO₂, CN, OH, les groupes méthyle, éthyle, méthoxy, diméthylamino, sulfamoyle, méthylsulfonyle, aminosulfonyle et méthylsulfonylamino ;
ou
R(15) et R(16)
forment ensemble une chaîne de 4 ou 5 groupes méthylène, dont un groupe CH₂ peut être remplacé par -O-, -S-, -NH-, -N(CH₃)- ou -N (benzyle) -;
R(17) représente un atome d'hydrogène ou un groupe alkyle ayant 1, 2 ou 3 atomes de carbone
R(5), R(6), R(7) et R(8)
représentent, indépendamment les uns des autres, un atome d'hydrogène, F, Cl, Br, I, un groupe alkyle ayant 1, 2, 3, 4 ou 5 atomes de carbone, cycloalkyle ayant 3, 4, 5, 6, 7 ou 8 atomes de carbone, -CN, -CF₃, -C₂F₅, -C₃F₇, -N₃, -NO₂, -Y-CₛH₂ₛ-R(18) ou phényle,
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
Y représente -O-, -CO-, -CO-O-, -O-CO-, -S-, -SO-, -SO₂-, -SO₂-O-, -SO₂NR(10c)-, -NR(10c)- ou -CONR(10c)- ;
R(10c) représente un atome d'hydrogène ou un groupe alkyle ayant 1, 2 ou 3 atomes de carbone ;
s est zéro, 1, 2, 3, 4, 5 ou 6 ;
R(18) représente un atome d'hydrogène, CF₃, C₂F₅, C₃F₇, un groupe cycloalkyle ayant 3, 4, 5, 6, 7 ou 8 atomes de carbone, -COOR(21), 1-pipéridyle, 1-pyrrolidinyle, 4-morpholinyle, 4-méthylpipérazin-1-yle, pyridyle, thiényle, imidazolyle, quinolyle, isoquinolyle ou phényle,
les groupes pyridyle, thiényle, imidazolyle, quinolyle, isoquinolyle et phényle étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(21) représente un atome d'hydrogène ou un groupe alkyle ayant 1, 2 ou 3 atomes de carbone ;
R(9) représente un atome d'hydrogène, un groupe OR(10d) ou OCOR(10d) ;
R(10d) représente un atome d'hydrogène ou un groupe alkyle ayant 1, 2 ou 3 atomes de carbone ;
B représente un atome d'hydrogène ;
ou
R(9) et B
forment ensemble une liaison;
ainsi que leurs sels physiologiquement acceptables ;
ou
8) HMR 97/L224 - EP-A-906 911
composés de formule I 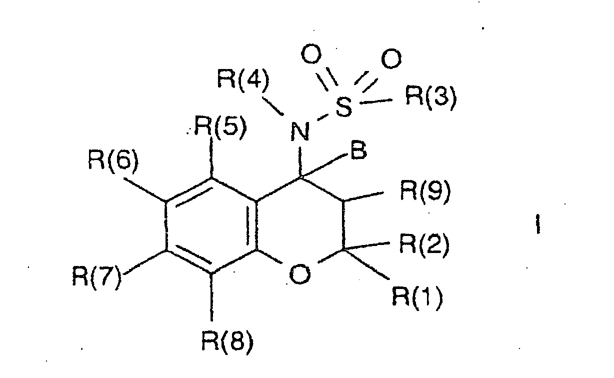 dans laquelle :
R(1) et R(2)
représentent, indépendamment l'un de l'autre, un atome d'hydrogène, CF₃, C₂F₅, C₃F₇ un groupe alkyle ayant 1, 2, 3, 4, 5 ou 6 atomes de carbone ou phényle,
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
ou
R(1) et R(2)
forment ensemble une chaîne alkylène ayant 2, 3, 4, 5, 6, 7, 8, 9 ou 10 atomes de carbone ;
R(3) représente R(10)-CₙH₂ₙ-NR(11)- ou R(10)-CₙH₂n-, un groupe CH₂ dans les groupes CₙH₂ₙ pouvant être remplacé par -O-, -CO-, -S-, SO-, -SO₂- ou -NR(12a)- ;
R(12a) représente un atome d'hydrogène
ou un groupe méthyle ou éthyle ;
R(10) représente un atome d'hydrogène ou un groupe méthyle, cycloalkyle ayant 3, 4, 5, 6, 7 ou 8 atomes de carbone, CF₃, C₂F₅ ou C₃F₇,
n est zéro, 1, 2, 3, 4, 5, 6, 7, 8, 9 ou 10 ;
R(11) représente un atome d'hydrogène ou un groupe alkyle ayant 1, 2, 3, 4, 5 ou 6 atomes de carbone ;
ou
R(10) et R(11)
forment ensemble une liaison, lorsque n n'est pas inférieur à 3 ;
ou
R(3) forme conjointement avec R(4)
une chaîne alkylène ayant 3, 4, 5, 6, 7 ou 8 atomes de carbone,
un groupe CH₂ de la chaîne alkylène pouvant être remplacé par -O-, -CO-, -S-, -SO-, -SO₂- ou -NR(12a)- ;
R(12a) représente un atome d'hydrogène, un groupe méthyle ou éthyle ;
R(4) représente R(13)-CᵣH₂ᵣ,
un groupe CH₂ du groupe CᵣH₂ᵣ pouvant être remplacé par -O-, -CH=CH-, -C≡C-, -CO-, -CO-O-, -O-CO-, -S-, -SO-, -SO₂-, -NR(14)- ou -CONR(14)- ;
R(14)
représente un atome d'hydrogène ou un groupe alkyle ayant 1, 2 ou 3 atomes de carbone, -C_{y}H_{2y}-OR(12b), - C_{y}H_{2y}-NR(12b)₂ ;
R(12b) représente un atome d'hydrogène, le groupe méthyle ou éthyle ;
y est 2 ou 3 ;
R(13) représente H, CF₃, C₂F₅, C₃F₇, un groupe cycloalkyle ayant 3, 4, 5, 6, 7 ou 8 atomes de carbone, -NR(15)R(16), -CONR(15)R(16), -OR(17), -COOR(17), phényle ou un hétérocycle azoté ayant 1, 2, 3, 4, 5, 6, 7, 8 ou 9 atomes de carbone,
le groupe phényle et l'hétérocycle azoté étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(15) et R(16)
représentent, indépendamment l'un de l'autre, un atome d'hydrogène ou un groupe alkyle ayant 1, 2, 3 ou 4 atomes de carbone ;
ou
R(15) et R(16)
forment ensemble une chaîne de 4 ou 5 groupes méthylène, dont un groupe CH₂ peut être remplacé par -O-, -S-, -NH-, -N(CH₃)- ou -N(benzyle)- ;
R(17) représente un atome d'hydrogène ou un groupe alkyle ayant 1, 2 ou 3 atomes de carbone,
-CₓH₂ₓ-OR(12c);
R(12c) représente un atome d'hydrogène, le groupe méthyle ou éthyle;
x est 2 ou 3 ;
r est zéro, 1, 2, 3, 4, 5, 6, 7, 8, 9, 10, 11, 12, 13, 14, 15, 16, 17, 18, 19 ou 20 ;
au moins l'un des substituants R(5), R(6), R(7) et R(8) représente un groupe -Y-CₛH₂s-R(18), thiényle, furyle ou un hétérocycle azoté ayant 1, 2, 3, 4, 5, 6, 7, 8 ou 9 atomes de carbone,
les groupes thiényle, furyle et l'hétérocycle azoté étant non substitués ou portant 1 ou 2 substituants choisis dans l'ensemble constitué par F, Cl, Br, I , CF₃, NO₂, CN, NH₂, OH, les groupes méthyle, éthyle, méthoxy, méthylamino, diméthylamino, éthylamino, diéthylamino, sulfamoyle, méthylsulfonyle et méthylsulfonylamino ;
Y représente -O-, -CO-, -O-CO-, -S-, -SO-, -SO₂-, -SO₂-O-, -SO₂NR(12d)-, -NR(12d)- ou -CONR(12d)- ;
la liaison au noyau benzénique s'effectuant chaque fois par l'atome se trouvant à gauche ;
R(12d) représente un atome d'hydrogène ou un groupe méthyle ou éthyle ;
s est 1, 2, 3, 4, 5 ou 6 ;
R(18) représente un groupe phényle qui porte un ou deux substituants choisis dans l'ensemble constitué par NO₂, CN, NH₂, N(méthyle)₂, OH, éthyle, -COOH, -COO-méthyle, -COO-éthyle, -CONH₂, -CON(méthyle)₂ ;
ou
R(18) représente un hétérocycle azoté ayant 1, 2, 3, 4, 5, 6, 7, 8 ou 9 atomes de carbone, qui porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
ou
R(18) représente -OR(19), -SO₂R(19), -NR(19)R(20), -CONR(19)R(20) ;
R(19) et R(20)
représentent, indépendamment l'un de l'autre, CₜH₂ₜ-R(21) ;
t est zéro, 1, 2, 3, 4, 5 ou 6 ;
R(21) représente un atome d'hydrogène, CF₃, C₂F₅, C₃F₇, un groupe cycloalkyle ayant 3, 4, 5, 6, 7 ou 8 atomes de carbone, NR(22)R(23), -OR(24), un groupe phényle, thiényle ou un hétérocycle azoté ayant 1, 2, 3, 4, 5, 6, 7, 8 ou 9 atomes de carbone,
les groupes phényle, thiényle et l'hétérocycle azoté étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(22) et R(23)
représentent, indépendamment l'un de l'autre, un atome d'hydrogène, un groupe alkyle ayant 1, 2 ou 3 atomes de carbone;
ou
R(22) et R(23)
forment ensemble une chaîne de 4 ou 5 groupes méthylène, dont un groupe CH₂ peut être remplacé par -O-, -S-, -NH-, -N(CH₃)- ou -N(benzyle)- ;
R(24) représente un atome d'hydrogène ou un groupe alkyle ayant 1, 2 ou 3 atomes de carbone
et les autres substituants R(5), R(6), R(7) et R(8) qui n'ont pas encore été donnés par la définition précédente
représentent, indépendamment les uns des autres, un atome d'hydrogène, F, Cl, Br, I, un groupe alkyle ayant 1, 2, 3, 4 ou 5 atomes de carbone, cycloalkyle ayant 3, 4, 5, 6, 7 ou 8 atomes de carbone, CN, CF₃, NO₂, OR(12e) ou NR(12e)R(12f) ;
R(12e) et R(12f)
représentent, indépendamment l'un de l'autre, un atome d'hydrogène ou un groupe alkyle ayant 1, 2, 3 ou 4 atomes de carbone ;
R(9) représente un atome d'hydrogène, OR(12g) ou OCOR(12g) ;
R(12g) représente un atome d'hydrogène ou un groupe alkyle ayant 1, 2 ou 3 atomes de carbone ;
B représente un atome d'hydrogène ;
ou
R(9) et B
forment ensemble une liaison ;
ainsi que leurs sels physiologiquement acceptables ;
ou
9) HMR 97/L232 - EP-913 396
composés de formule I, 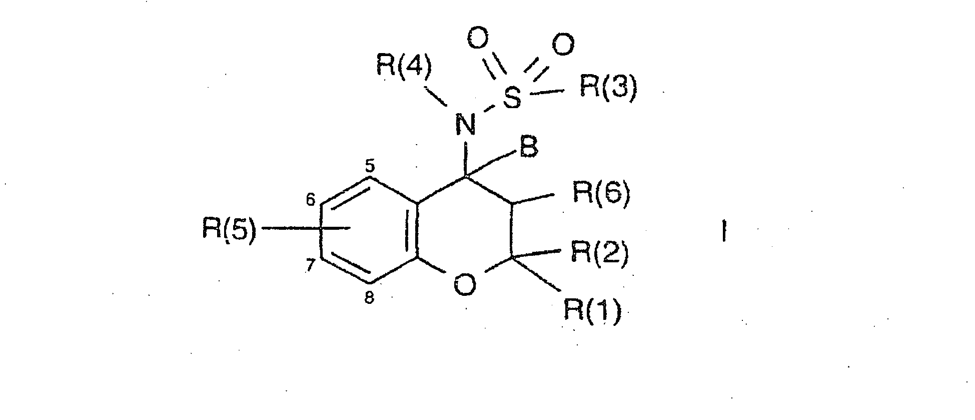 dans laquelle R(5) est lié en l'une des positions **caractérisées par** 5, 6, 7 et 8
et dans laquelle :
R(1) et R(2)
représentent, indépendamment l'un de l'autre, un atome d'hydrogène, CF₃, C₂F₅, C₃F₇ un groupe alkyle ayant 1, 2, 3, 4, 5 ou 6 atomes de carbone ou phényle,
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
ou
R(1) et R(2)
forment ensemble une chaîne alkylène ayant 2, 3, 4, 5, 6, 7, 8, 9 ou 10 atomes de carbone ;
R(3) représente R(10)-CₙH₂ₙ-NR(11)- ou R(10)-CₙH₂ₙ-, un groupe CH₂ dans les groupes CₙH₂ₙ pouvant être remplacé par -O-, -CO-, -S-, SO-, -SO₂- ou -NR(12a)- ;
R(12a) représente un atome d'hydrogène ou un groupe méthyle ou éthyle ;
R(10) représente un atome d'hydrogène ou un groupe méthyle, cycloalkyle ayant 3, 4, 5, 6, 7 ou 8 atomes de carbone, CF₃, C₂F₅ ou C₃F₇,
n est zéro, 1, 2, 3, 4, 5, 6, 7, 8, 9 ou 10 ;
R(11) représente un atome d'hydrogène ou un groupe alkyle ayant 1, 2, 3, 4, 5 ou 6 atomes de carbone ;
ou
R(10) et R(11)
forment ensemble une liaison, lorsque n n'est pas inférieur à 3 ;
ou
R(3) forme conjointement avec R(4)
une chaîne alkylène ayant 3, 4, 5, 6, 7 ou 8 atomes de carbone,
un groupe CH₂ de la chaîne alkylène pouvant être remplacé par -O-, -CO-, -S-, -SO-, -SO₂- ou -NR(12a)- ;
R(12a) représente un atome d'hydrogène, un groupe méthyle ou éthyle ;
R(4) représente R(13)-CᵣH₂ᵣ,
un groupe CH₂ du groupe CᵣH₂ᵣ pouvant être remplacé par -O-, -CH=CH-, -C≡C-, -CO-, -CO-O-, -O-CO-, -S-, -SO-, -SO₂- ou -NR(14)- ;
R(14) représente un atome d'hydrogène ou un groupe alkyle ayant 1, 2 ou 3 atomes de carbone ;
R(13) représente CH₃, CF₃, C₂F₅, C₃F₇, un groupe cycloalkyle ayant 3, 4, 5, 6, 7 ou 8 atomes de carbone, -NR(15)R(16), phényle ou un hétérocycle azoté ayant 1, 2, 3, 4, 5, 6, 7, 8 ou 9 atomes de carbone,
le groupe phényle et l'hétérocycle azoté étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(15) et R(16)
forment ensemble une chaîne de 4 ou 5 groupes méthylène, dont un groupe CH₂ peut être remplacé par -O-, -S-, -NH-, -N(méthyle)- ou -N(benzyle)- ;
r est zéro, 1, 2, 3, 4, 5, 6, 7, 8, 9, 10, 11, 12, 13, 14, 15, 16, 17, 18, 19 ou 20 ;
R(5) représente un groupe -Y-CₛH₂ₛ-R(18) ou phényle,
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
Y représente -O-, -S- ou -NR(10c)- ;
R(10c) représente un atome d'hydrogène ou un groupe alkyle ayant 1, 2 ou 3 atomes de carbone ;
s est zéro, 1, 2, 3, 4, 5, 6, 7 ou 8 ;
R(18) représente un atome d'hydrogène, CF₃, C₂F₅, C₃F₇, un groupe cycloalkyle ayant 3, 4, 5, 6, 7 ou 8 atomes de carbone, -COOR(21), NR(15a)R(16a), un hétérocycle azoté ayant 1, 2, 3, 4, 5, 6, 7, 8 ou 9 atomes de carbone, phényle ou thiényle,
les groupes phényle et thiényle étant non substitués ou portant 1 ou 2 substituants choisis dans l'ensemble constitué par F, Cl, Br, I, CF₃, les groupes méthyle, méthoxy, sulfamoyle, méthylsulfonyle et méthylsulfonylamino ;
R(15a) et R(16a)
forment ensemble une chaîne de 4 ou 5 groupes méthylène, dont un groupe CH₂ peut être remplacé par -O-, -S-, -NH-, -N(méthyle)- ou -N(benzyle)- ;
R(21) représente un atome d'hydrogène ou un groupe alkyle ayant 1, 2 ou 3 atomes de carbone ;
R(6) représente OR(10d) ou OCOR(10d) ;
R(10d) représente un atome d'hydrogène ou un groupe alkyle ayant 1, 2 ou 3 atomes de carbone ;
B représente un atome d'hydrogène ;
ou
R(6) et B
forment ensemble une liaison ;
ainsi que leurs sels physiologiquement acceptables.
ou
10) HMR 97/L235 - EP-915 087
composés de formule I, 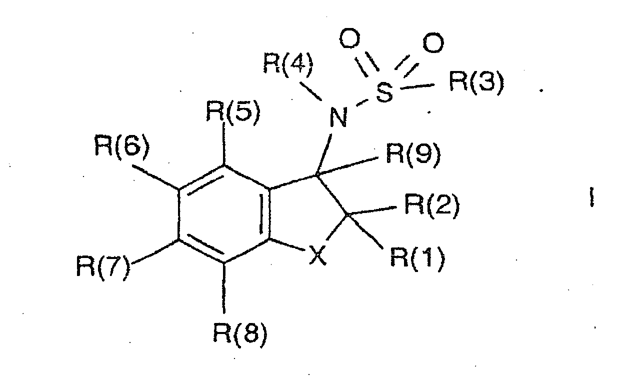 dans laquelle :
R(1) et R(2) représentent, indépendamment l'un de l'autre, un atome d'hydrogène, CF₃, un groupe alkyle ayant 1, 2, 3, 4, 5 ou 6 atomes de carbone ;
ou
R(2) et R(9) forment ensemble une liaison ;
ou R(2)
représente -OR(10a), lorsque X représente -CR(22)R(23)- ;
R(10a) représente un atome d'hydrogène ou un groupe acétyle ou alkyle ayant 1, 2 ou 3 atomes de carbone ;
R(3) représente R(10b)-CₙH₂ₙ-NR(11)- ou
R(10b) -CₙH₂ₙ-,
un groupe CH₂ dans les groupes CₙH₂ₙ pouvant être remplacé par -O-, -CO-, -S-, SO-, -SO₂- ou -NR(12a)- ;
R(12a) représente un atome d'hydrogène ou un groupe méthyle ou éthyle ;
R(10b) représente un groupe méthyle, cycloalkyle ayant 3, 4, 5, 6, 7 ou 8 atomes de carbone, CF₃, C₂F₅ ou C₃F₇,
n est zéro, 1, 2, 3, 4, 5, 6, 7, 8, 9 ou 10 ;
R(11) représente un atome d'hydrogène ou un groupe alkyle ayant 1, 2, 3, 4, 5 ou 6 atomes de carbone ;
ou
R(10b) et R(11)
forment ensemble une liaison, lorsque n est supérieur à 2 ;
ou
R(3) forme conjointement avec R(4)
une chaîne alkylène ayant 3, 4, 5, 6, 7 ou 8 atomes de carbone,
un groupe CH₂ de la chaîne alkylène pouvant être remplacé par -O-, -CO-, -S-, -SO-, -SO₂- ou -NR(12a)- ;
R(12a) représente un atome d'hydrogène, un groupe méthyle ou éthyle ;
R(4) représente R(13)-CᵣH₂ᵣ,
un groupe CH₂ du groupe CᵣH₂ᵣ pouvant être remplacé par -O-, -CH=CH-, -C≡C-, -CO-, -CO-O-, -O-CO-, -S-, -SO-, -SO₂-, -NR(14)- ou -CONR(14)- ;
R(14)
représente un atome d'hydrogène ou un groupe alkyle ayant 1, 2 ou 3 atomes de carbone, -C_{y}H_{2y}-OR(12b), -CyH₂y-NR(12b)₂ ;
R(12b) représente un atome d'hydrogène, le groupe méthyle ou éthyle ;
y est 2 ou 3 ;
R(13) représente CH₃, CF₃, C₂F₅, C₃F₇, un groupe cycloalkyle ayant 3, 4, 5, 6, 7 ou 8 atomes de carbone, -NR(15)R(16), -CONR(15)R(16), -OR(17), -COOR(17), phényle ou un hétérocycle azoté ayant 1, 2, 3, 4, 5, 6, 7, 8 ou 9 atomes de carbone,
le groupe phényle et l'hétérocycle azoté étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(15) et R(16)
représentent, indépendamment l'un de l'autre, un atome d'hydrogène ou un groupe alkyle ayant 1, 2, 3 ou 4 atomes de carbone ;
ou
R(15) et R(16)
forment ensemble une chaîne de 4 ou 5 groupes méthylène, dont un groupe CH₂ peut être remplacé par -O-, -S-, -NH-, -N(CH₃)- ou -N(benzyle)- ;
R(17) représente un atome d'hydrogène ou un groupe alkyle ayant 1, 2 ou 3 atomes de carbone,
-C_{z}H_{2z}-OR(12c) ;
R(12c) représente un atome d'hydrogène, le groupe méthyle ou éthyle ;
z est 2 ou 3 ;
r est zéro, 1, 2, 3, 4, 5, 6, 7, 8, 9, 10, 11, 12, 13, 14, 15, 16, 17, 18, 19 ou 20 ;
R(5), R(6), R(7) et R(8)
représentent, indépendamment les uns des autres, un atome d'hydrogène, F, Cl, Br, I, un groupe alkyle ayant 1, 2, 3, 4 ou 5 atomes de carbone, cycloalkyle ayant 3, 4, 5, 6, 7 ou 8 atomes de carbone, -CN, -CF₃, -C₂F₅, -C₃F₇, -N₃, -NO₂, -Y-CₛH₂ₛ-R(18), phényle, thiényle, furyle ou un hétérocycle ayant 1, 2, 3, 4, 5, 6, 7, 8 ou 9 atomes de carbone,
les groupes phényle, thiényle, furyle et l'hétérocycle azoté étant non substitués ou portant 1 ou 2 substituants choisis dans l'ensemble constitué par F, Cl, Br, I , CF₃, NO₂, CN, NH₂, OH, les groupes méthyle, éthyle, méthoxy, diméthylamino, sulfamoyle, méthylsulfonyle et méthylsulfonylamino ;
Y représente -O-, -CO-, -CO-O-, -O-CO-, -S-, -SO-, -SO₂-, -O-SO₂-, -SO₂NR(10c)- ou -CONR(10c)-, la liaison à la structure de base s'effectuant chaque fois par l'atome se trouvant à gauche ; R(10c) représente un atome d'hydrogène
ou un groupe alkyle ayant 1, 2 ou 3 atomes de carbone ;
s est zéro, 1, 2, 3, 4, 5 ou 6 ;
R(18) représente un atome d'hydrogène, un groupe méthyle, CF₃, C₂F₅, C₃F₇, cycloalkyle ayant 3, 4, 5, 6, 7 ou 8 atomes de carbone, -OR(21), -COOR(21), -NR(15a)R(16a), -CONR(15a)R(16a), phényle ou un hétérocycle azoté ayant 1, 2, 3, 4, 5, 6, 7, 8 ou 9 atomes de carbone,
le groupe phényle et l'hétérocycle azoté étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(15a) et R(16a)
Représentent, indépendamment l'un de l'autre, un atome d'hydrogène ou un groupe alkyle ayant 1, 2 ou 3 atomes de carbone ;
ou
R(15a) et R(16a)
forment ensemble une chaîne de 4 ou 5 groupes méthylène, dont un groupe CH₂ peut être remplacé par -O-, -S-, -NH-, -N(CH₃)- ou -N(benzyle)- ;
R(21) représente un atome d'hydrogène ou un groupe alkyle ayant 1, 2 ou 3 atomes de carbone ;
R(9) représente un atome d'hydrogène ou forme, conjointement avec R(2), une liaison ;
X représente -CR(22)R(23)-, -O-, -NR(24)-, -S-, -SO-, -SO₂- ;
R(22) et R(23)
représentent, indépendamment l'un de l'autre, un atome d'hydrogène, CF₃ ou un groupe alkyle ayant 1, 2, 3, 4, 5 ou 6 atomes de carbone ;
R(24) représente un atome d'hydrogène, un groupe alkyle ayant 1, 2, 3, 4, 5 ou 6 atomes de carbone ou phényle,
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
ainsi que leurs sels physiologiquement acceptables.

2. Utilisation selon la revendication 1 d'inhibiteurs du canal Iₖₛ à action simultanée en tant qu'inhibiteurs du canal KQT1, pour la fabrication d'un médicament destiné au traitement de maladies qui sont provoquées par des helminthes.
